(19) Europäisches Patentamt
European Patent Office
Office européen des brevets

(11) EP 3 777 832 A1

(12) EUROPEAN PATENT APPLICATION

(43) Date of publication:
**17.02.2021 Bulletin 2021/07**

(51) Int Cl.:
***A61K 9/00*** *(2006.01)*

(21) Application number: **20196756.9**

(22) Date of filing: **20.10.2008**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **20.10.2007 US 999943 P**

(62) Document number(s) of the earlier application(s) in accordance with Art. 76 EPC:
**08839636.1 / 2 217 229**

(71) Applicant: **Athenex, Inc.**
**Buffalo, NY 14203 (US)**

(72) Inventor: **HANGAUER, David G.**
**Lancaster, NY New York 10486 (US)**

(74) Representative: **Cooley (UK) LLP**
**Dashwood**
**69 Old Broad Street**
**London EC2M 1QS (GB)**

Remarks:
This application was filed on 17-09-2020 as a divisional application to the application mentioned under INID code 62.

# (54) PHARMACEUTICAL COMPOSITIONS FOR MODULATING A KINASE CASCADE AND METHODS OF USE THEREOF

(57) The invention relates to a pharmaceutical composition comprising 2-(5-(4-(2-mopholinoethoxy)phenyl)pyridin-2-yl)-N-benzylacetamide or a pharmaceutically acceptable salt thereof and a pharmaceutically acceptable carrier.

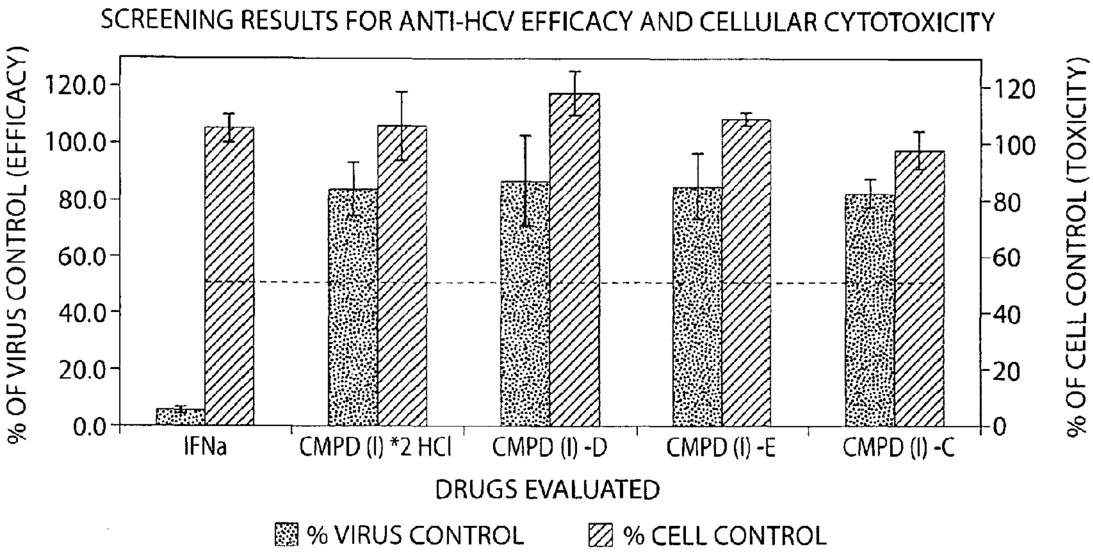


Fig. 23

EP 3 777 832 A1

## Description

### RELATED APPLICATION

This application claims priority to U.S. provisional patent application serial number 60/999,943, filed October 20, 2007. The entire contents of which are incorporated by reference herein.

### FIELD OF THE INVENTION

The present invention is directed to pharmaceutical compositions comprising 2-(5-(4-(2-morpholinoethoxy)phenyl)pyridin-2-yl)-N-benzylacetamide (compound (I)), and its pharmaceuticaly acceptable salts e.g., a mesylate salt. The invention also relates to methods of using such compositions.

### BACKGROUND OF THE INVENTION

This invention relates to a biaryl compound and pharmaceutically acceptable salts thereof, including a mesylate salt useful in the treatment of or protecting against certain conditions or disorders. More particularly, the invention relates to the compound 2-(5-(4-(2-morpholinoethoxy)phenyl)pyridin-2-yl)-N-benzylacetamide, compound (I) having the formula:

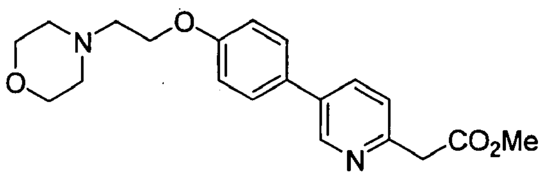

(I)

or a pharmaceutically acceptable salt thereof, including a mesylate salt.

Compound (I) is specifically disclosed and claimed in U.S. Patent No. 7,300,931. This patent also discloses the use of compound (I) in treating cell proliferation disorders.

Compound (I) and pharmaceutically acceptable salts thereof, are potent Src tyrosine kinase inhibitors which can be used in the treatment of or protection against conditions or disorders including cancer, cell proliferative disorder, microbial infection, hyperproliferative disorder, macular edema, osteoporosis, cardiovascular disorder, eye disease, immune system disfunction, type II diabetes, obesity, transplant rejection, hearing loss, stroke, athrosclerosis, chronic neuropathic pain, hepatitis B, and autoimmune disease. The use of compound (I) for treatment of and protection against these conditions and disorders is described in US 2007/0015752, PCT/US2008/004847, and WO2008/002676.

The present invention discloses certain pharmaceutical compositions comprising compound (I) or a pharmaceutically acceptable salt thereof.

### SUMMARY OF THE INVENTION

The invention provides a pharmaceutical composition for oral, intravenous, intramuscular, or subcutaneous administration comprising an amount of compound (I) or a pharmaceutically acceptable salt thereof, ranging from 2 mg to 400 mg per dose administered two or three times daily and a pharmaceutically acceptable carrier. In one aspect, the amount is from 10 mg to 300 mg. In another aspect, the amount is from 20 mg to 250 mg. In another aspect, the amount is from 40 mg to 200 mg. In another aspect, the amount is from 60 mg to 160 mg. In one aspect, the dose is administered two times daily. In another aspect, the dose is administered three times daily.

The invention provides a pharmaceutical composition for oral, intravenous, intramuscular, or subcutaneous administration comprising an amount of compound (I) or a pharmaceutically acceptable salt thereof ranging from 4 mg to 800 mg per dose administered once daily and a pharmaceutically acceptable carrier. In one aspect, the the amount is from 20 mg to 600 mg. In another aspect, the amount is from 40 mg to 500 mg. In another aspect, the amount is from 80 mg to 400 mg. In another aspect, the amount is from 120 mg to 320 mg.

The invention provides a pharmaceutical composition, wherein the composition comprises the mesylate salt of compound (I).

The invention provides a pharmaceutical composition, wherein the administration is oral. In another aspect, the administration is intravenous. In another aspect, the administration is intramuscular. In another aspect, the administration is subcutaneous.

The invention provides a pharmaceutical composition, wherein the composition is administered in combination with one or more anti-cancer treatments or anti-cancer agents. In one aspect, the pharmaceutical composition is administered in combination with the anti-cancer agent gemcitabine. In another aspect, the pharmaceutical composition is administered in combination with the anti-cancer agent oxaliplatin.

The invention provides a method of treating or preventing a condition or disorder selected from cancer, cell proliferative disorder, microbial infection, hyperproliferative disorder, macular edema, osteoporosis, cardiovascular disorder, eye disease, immune system disfunction, type II diabetes, obesity, transplant rejection, hearing loss, stroke, athrosclerosis, chronic neuropathic pain, hepatitis B, and autoimmune disease comprising administering the pharmaceutical composition described herein. In one aspect, the condition or disorder is cancer. In one aspect, the cancer is selected from renal, prostate, liver, lung, pancreatic, brain, breast, colon, leukemia, ovarian, epithelial, and esophageal. In another aspect, the cancer is selected from an advanced malignancy, a solid tumor, and lymphoma. In one aspect, the condition or disorder is a cell proliferative disorder. In one aspect, the cell proliferative disorder is selected from psoriasis, diabetic retinopathy, and macular degeneration. In one aspect, the condition or disorder is a microbial infection. In one aspect, the microbial infection is selected from bacterial, fungal, parasitic, and viral. In one aspect, the disorder or condition is selected from hyperproliferative disorder, macular edema, osteoporosis, cardiovascular disorder, eye disease, immune system disfunction, type II diabetes, obesity, transplant rejection, hearing loss, stroke, athrosclerosis, chronic neuropathic pain, hepatitis B, and autoimmune disease.

The invention provides a method of regulating immune system activity comprising administering the pharmaceutical composition described herein.

The invention provides use of the pharmaceutical composition of the invention in the manufacture of a medicament for treating or preventing a condition or disorder selected from cancer, cell proliferative disorder, microbial infection, hyperproliferative disorder, macular edema, osteoporosis, cardiovascular disorder, eye disease, immune system disfunction, type II diabetes, obesity, transplant rejection, hearing loss, stroke, athrosclerosis, chronic neuropathic pain, hepatitis B, and autoimmune disease. In one aspect, the condition or disorder is cancer. In one aspect, the cancer is selected from renal, prostate, liver, lung, pancreatic, brain, breast, colon, leukemia, ovarian, epithelial, and esophageal. In another aspect, the cancer is selected from an advanced malignancy, a solid tumor, and lymphoma. In one aspect, the condition or disorder is a cell proliferative disorder. In one aspect, the cell proliferative disorder is selected from psoriasis, diabetic retinopathy, and macular degeneration. In one aspect, the condition or disorder is a microbial infection. In one aspect, the microbial infection is selected from bacterial, fungal, parasitic, and viral. In one aspect, the disorder or condition is selected from hyperproliferative disorder, macular edema, osteoporosis, cardiovascular disorder, eye disease, immune system disfunction, type II diabetes, obesity, transplant rejection, hearing loss, stroke, athrosclerosis, chronic neuropathic pain, hepatitis B, and autoimmune disease.

The invention provides use of the pharmaceutical composition of the invention in the manufacture of a medicament for regulating immune system activity.

## BRIEF DESCRIPTION OF THE DRAWINGS

Figure 1A is a graph indicating the effect of AZ28 and compound (I) on Src autophosphorylation in c-Src/NIH-3T3 cells; Figure 1B is a graph indicating the effect of AZ28 and compound (I) on Src autophosphorylation in HT-29 cells.

Figure 2A is a graph indicating the effect of AZ28 and compound (I) on FAK phosphorylation in c-Src/NIH-3T3 cells; Figure 2B is a graph indicating the effect of AZ28 and compound (I) on FAK phosphorylation in HT-29 cells.

Figure 3A is a graph indicating the effect of AZ28 and compound (I) on Shc phosphorylation in c-Src/NIH-3T3 cells; Figure 3B is a graph indicating the effect of AZ28 and compound (I) on Shc phosphorylation in HT-29 cells.

Figure 4 is a graph indicating the effect of AZ28 and compound (I) on paxillin phosphorylation in c-Src/NIH-3T3 cells.

Figure 5A is a graph indicating the effect of AZ28 and compound (I) on caspase-3 cleavage in c-Src/NIH-3T3 cells; Figure 5B is a graph indicating the effect of AZ28 and compound (I) on caspase-3 cleavage in HT-29 cells.

Figure 6A is a graph indicating the effect of AZ28 and compound (I) on total phosphotyrosine levels in c-Src/NIH-3T3 cells; Figure 6B is a graph indicating the effect of AZ28 and compound (I) on total phosphotyrosine levels in HT-29 cells.

Figure 7 is a graph indicating the effect of AZ28 and compound (I) on autophosphorylation of PDGFR in c-Src/NIH-3T3 cells.

Figure 8A is a graph indicating the effect of AZ28 and compound (I) on autophosphorylation of FAK in c-Src/NIH-3T3 cells; Figure 8B is a graph indicating the effect of AZ28 and compound (I) on autophosphorylation of FAK in HT-29 cells.

Figure 9A is a graph indicating the effect of AZ28 and compound (I) on autophosphorylation of EGFR in c-Src/NIH-3T3 cells; Figure 9B is a graph indicating the effect of AZ28 and compound (I) on autophosphorylation of EGFR in HT-29 cells.

Figures 10A, 10B, 10C, and 10D are a series of graphs depicting the inhibition of Src kinase activity in whole cells.

Figure 10A is a graph depicting the effect of compound (I) on Src autophosphorylation in c-Src/NIH-3T3 cells; Figure 10B is a graph indicating the effect of compound (I) on Src autophosphorylation in HT-29 cells; Figure 10C is a graph depicting the effect of compound (I) on Src transphosphorylation in c-Src/NIH-3T3 cells; and Figure 10D is a graph indicating the effect of compound (I) on Src autophosphorylation in HT-29 cells.

Figure 11 is an illustration depicting the selectivity of compound (I) for protein tyrosine kinases (PTKs) in whole cells as compared to Dasatinib, an ATP-competitive Src inhibitor currently in clinical trials.

Figure 12 is a graph indicating the effect of Dasatinib on Dasatinib and Imatinib resistant leukemia cells.

Figure 13 is a graph indicating the effect of compound (I) on Dasatinib and Imatinib resistant leukemia cells.

Figure 14 shows the growth inhibition curves and $GI_{50}$ of compound (I) as compared to Dasatinib (BMS354825) in HT-29 cells.

Figure 15 shows the growth inhibition curves and $GI_{50}$ of compound (I) as compared to Dasatinib (BMS354825) in SKOV-3 cells.

Figure 16 shows the growth inhibition curves and $GI_{50}$ of compound (I) as compared to Dasatinib (BMS354825) in A549 cells.

Figure 17 shows the growth inhibition curves and $GI_{50}$ of compound (I) as compared to Dasatinib (BMS354825) in K562 cells.

Figure 18 shows the growth inhibition curves and $GI_{50}$ of compound (I) as compared to Dasatinib (BMS354825) in MDA-MB-231 cells.

Figure 19 shows the growth inhibition curves and $GI_{50}$ of the combination of Gemzar® and compound (I) in the L3.6pl cell line using the BrdU assay.

Figure 20 shows the growth inhibition curves and $GI_{50}$ of Gemzar® and compound (I) in the L3.6pl cell line using the BrdU assay.

Figure 21 shows the tumor weight from the orthotopic prostate model for measuring *in vivo* metastases at various concentration of compound (I).

Figure 22 shows a second week IVIS follow up study after the treatment of compound (I) at 2.5 mg/dose bid, 5.0 mg/dose bid, and Dasatinib 7.5 mg/dose bid.

Figure 23 is a bar graph of the screening results for anti-HBV efficacy and cellular cytotoxicity.

Figure 24 is a graph depicting the oral potency of compound (I) in mouse xenografts. Compound (I) demonstrated higher oral potency in staged HT29 (a human colon cancer cell line) mouse than Dasatinib.

Figures 25A-D are a series of graphs showing the weight gain in each of the C57BL/6 mice in the different treatment groups of the intracranial GL261 glioma survival study.

Figure 26 is a graph showing the average weights over a 40-day period for each of the treatment groups in the intracranial GL261 glioma survival study.

Figure 27 is a graph showing synergistic growth inhibitory effects of tamoxifen and compound (I) on MCF-7 cells.

## DETAILED DESCRIPTION OF THE INVENTION

The details of one or more embodiments of the invention are set forth in the accompanying description below. Although any methods and materials similar or equivalent to those described herein can be used in the practice or testing of the present invention, the preferred methods and materials are now described. Other features, objects, and advantages of the invention will be apparent from the description. In the specification, the singular forms also include the plural unless the context clearly dictates otherwise. Unless defined otherwise, all technical and scientific terms used herein have the same meaning as commonly understood by one of ordinary skill in the art to which this invention belongs. In the case of conflict, the present specification will control. All publications, patent applications, patents, and other references mentioned herein are incorporated by reference in their entirety.

Compound (I) is a synthetic, orally bioavailable, and highly selective small molecule Src tyrosine kinase inhibitor. It is first in its class because compound (I) targets the peptide substrate-binding site and not the ATP-binding site like all other known Src kinase inhibitors. In defining its tumor cell biological activity, compound (I) has been shown to potently inhibit the Src-catalyzed transphosphorylation of focal adhesion kinase (FAK), Shc, paxillin, and Src kinase autophosphorylation with $IC_{50}$'s around 20 nM. It has also been demonstrated to induce p53 expression and stimulate Caspase-3 and PARP cleavage, all of which lead to tumor cell apoptosis.

Compound (I) is potent against a broad range of solid tumor cell types as well as many leukemia types including those resistant to imatinib and/or dasatinib. Unlike Src kinase inhibitors that are commercially available and currently in development, compound (I) does not compete for the ATP binding site. It is highly selective in that it does not inhibit PDGFR, EGFR, JAK1, JAK2, Lck and ZAP70. It has a 10-100 fold lower potency than dasatinib in inhibiting Bcr/Abl. As the inhibition of Bcr/Abl by dasatinib and imatinib mesylate has been shown to be associated with cardiotoxicity, compound (I) is less likely to be cardiotoxic.

In terms of in vivo efficacy, compound (I) is about five times more potent than dasatinib against tumor cell

proliferation in an HT29 (human colon cancer) xenograft mouse model. In a PC3-MM2 (human prostatic cancer) orthotopic mouse model, compound (I) demonstrated strong inhibition of both primary tumor growth as well as lymph node metastasis.

Because kinases are involved in the regulation of a wide variety of normal cellular signal transduction pathways (e.g., cell growth, differentiation, survival, adhesion, migration, etc.), kinases are thought to play a role in a variety of diseases and disorders. Thus, modulation of kinase signaling cascades may be an important way to treat or prevent or protect against such diseases and disorders. Such diseases and disorders include, for example, cancers, osteoporosis, cardiovascular disorders, immune system dysfunction, type II diabetes, obesity, and transplant rejection.

Compound (I) or a pharmaceutically acceptable salt thereof, is useful in modulation a component of the kinase signaling cascade. A number of protein kinases and phosphatases are known, and are targets for the development of therapeutics. See, e.g., Hidaka and Kobayashi, Annu. Rev. Pharmacol. Toxicol, 1992, 32:377-397; Davies et al., Biochem. J., 2000, 351:95-105, each of which is incorporated by reference herein.

One family of kinases, the protein tyrosine kinases are divided into two large families: receptor tyrosine kinases, or RTKs (e.g., insulin receptor kinase (IRK), epidermal growth factor receptor (EGFR), basic fibroblast growth factor receptor (FGFR), platelet-derived growth factor receptor (PDGFR), vascular endothelial growth factor receptor (VEGFR-2 or Flk1/KDR), and nerve growth factor receptor (NGFR)) and nonreceptor tyrosine kinases, or NRTKs (e.g., the Src family (Src, Fyn, Yes, Blk, Yrk, Fgr, Hck, Lck, and Lyn), Fak, Jak, Abl and Zap70). See, for example, Parang and Sun, Expert Opin. Ther. Patents, 2005, 15:1183-1207, incorporated by reference herein.

Because of the role of Src kinases in a variety of cancers, these kinases are the subject of a number of studies relating to the development of Src inhibitors as cancer therapeutics, including highly metastatic cancer cell growth. Src inhibitors are sought as therapeutics for a variety of cancers, including, for example, colon cancer, precancerous colon lesions, ovarian cancer, breast cancer, epithelial cancers, esophageal cancer, non-small cell lung cancer, pancreatic cancer, and others. See, e.g., Frame, Biochim. Biophys. Acta, 2002, 1602:114-130 and Parang and Sun, Expert Opin. Ther. Patents, 2005, 15:1183-1207.

Inhibition of other kinases may be useful in the treatment and modulation of other types of diseases and disorders. For example, various eye diseases may be inhibited or prevented by administration of VEGF receptor tyrosine kinase inhibitors. Inhibitors of the tyrosine phosphatase PTP-1B and/or glycogen phosphorylase may provide treatments for Type II diabetes or obesity. Inhibitors of p561ck may be useful in treating immune system disorders. Other targets include HIV reverse transcriptase, thromboxane synthase, EGFRTK, p55 fyn, etc.

Compound (I) is a Src signaling inhibitor that binds in the Src peptide substrate site. The activity of compound (I) has been studied in c-Src (527F, constitutively active and transforming) transformed NIH3T3 cells and in human colon cancer cells (HT29). For example, in these cell lines, compound (I) was shown to reduce the phosphorylation level of known Src protein substrates in a dose-dependent fashion and in good correlation with growth inhibitory effects.

Without wishing to be bound by theory, it is believed that the conformation of some kinases (e.g., Src) outside cells relative to the conformation inside cells is markedly different, because inside cells, many kinases are is embedded in multiprotein signaling complexes. Thus, because the peptide substrate binding site is not well formed in an isolated kinase (as shown by Src x-ray structures), it is believed that the activity against isolated kinase for a peptide substrate binding inhibitor would be weak. Binding to this site in an isolated kinase assay requires the inhibitor to capture the very small percentage of total protein in an isolated enzyme assay that is in the same conformation that exists inside cells. This requires a large excess of the inhibitor to drain significant amounts of the enzyme from the catalytic cycle in the assay in order to be detectable.

However, for cell-based assays, a large inhibitor excess is not needed because the peptide binding site is expected to be formed. In cell-based Src assays, SH2 & SH3 domain binding proteins have already shifted the Src conformation so that the peptide substrate binding site is fully formed. Thus, low concentrations of the inhibitor can remove the enzyme from the catalytic cycle since all of the enzyme is in the tight binding conformation.

The vast majority of known kinase inhibitors are ATP competitive and show poor selectivity in a panel of isolated kinase assays. However, compound (I) is thought to be peptide substrate binding inhibitor. Thus, traditional high throughput screening of compound (I) against isolated enzymes, such as Src, would not result in the discovery of compound (I).

There is considerable recent literature support for targeting pp60c-src (Src) as a broadly useful approach to cancer therapy without resulting in serious toxicity. For example, tumors that display enhanced EGF receptor PTK signaling, or overexpress the related Her-2/neu receptor, have constitutively activated Src and enhanced tumor invasiveness. Inhibition of Src in these cells induces growth arrest, triggers apoptosis, and reverses the transformed phenotype (Kami et al. (1999) Oncogene 18(33): 4654-4662). It is known that abnormally elevated Src activity allows transformed cells to grow in an anchorage-independent fashion. This is apparently caused by the fact that extracellular matrix signaling elevates Src activity in the FAK/Src pathway, in a coordinated fashion with mitogenic signaling, and thereby blocks an apoptotic mechanism which would normally have been activated. Consequently FAK/Src inhibition in tumor cells may induce apoptosis because the apoptotic mechanism which would have normally become activated upon breaking free from the extracellular matrix would be induced (Hisano, et al., Proc. Annu. Meet. Am. Assoc. Cancer Res. 38:A1925

(1997)). Additionally, reduced VEGF mRNA expression was noted upon Src inhibition and tumors derived from these Src-inhibited cell lines showed reduced angiogenic development (Ellis et al., Journal of Biological Chemistry 273 (2):1052-1057 (1998)).

For example, a knock-out of the Src gene in mice led to only one defect, namely osteoclasts that fail to form ruffled borders and consequently do not resorb bone. However, the osteoclast bone resorb function was rescued in these mice by inserting a kinase defective Src gene (Schwartzberg et al., (1997) Genes & Development 11: 2835-2844). This suggested that Src kinase activity can be inhibited *in vivo* without triggering the only known toxicity because the presence of the Src protein is apparently sufficient to recruit and activate other PTKs (which are essential for maintaining osteoclast function) in an osteoclast essential signaling complex.

Src has been proposed to be a "universal" target for cancer therapy since it has been found to be overactivated in a growing number of human tumors (Levitzki, Current Opinion in Cell Biology, 8, 239-244 (1996); Levitzki, Anti-Cancer Drug Design, 11, 175-182 (1996)). The potential benefits of Src inhibition for cancer therapy appear to be four-fold inhibition of uncontrolled cell growth caused by autocrine growth factor loop effects, inhibition of metastasis due to triggering apoptosis upon breaking free from the cell matrix, inhibition of tumor angiogenesis via reduced VEGF levels, low toxicity.

Prostate cancer cells have been reported to have both an over expression of paxillin and p130cas and are hyperphosphorylated (Tremblay et al., Int. J. Cancer, 68, 164-171, 1996) and may thus be a prime target for Src inhibitors.

In certain embodiments, the type of cancer includes solid tumors and non-solid tumors. In specific embodiments the solid tumors are selected from tumors in the CNS (central nervous system), liver cancer, colorectal carcinoma, breast cancer, gastric cancer, pancreatic cancer, bladder carcinoma, cervical carcinoma, head and neck tumors, vulvar cancer and dermatological neoplasms including melanoma, squamous cell carcinoma and basal cell carcinomas. In other embodiment, non-solid tumors include lymphoproliferative disorders including leukemias and lymphomas. In other embodiments a disorder is metastatic disease.

Compound (I) displays broad solid tumor activity, as is reported in the table below.

| Human Tumor Cell Line | Compound (I) GI50 (nM) | Dasatinib GI50 (nM) |
|---|---|---|
| HT29 (Colon) | 25 | 20 |
| SKOV-3 (Ovarian) | 9.8 | 3.2 |
| PC3-MM2 (Prostate) | 8.9 | 8.9 |
| L3.6pl (Pancreas) | 25 (n=3) | 3.9 |
| MDA231 (Breast) | 20 | 6.9 |
| A549 (Lung) | 9.4 | 13 |

Compound (I) also may be used in the treatment of a cancer or cell proliferation disorder in combination therapy with one or more of anti-cancer treatments such as radiation therapy, and/or one or more anti-cancer agents selected from the group consisting of anti-proliferative agents, cytotoxic agents, cytostatic agents, and chemotherapeutic agents and salts and derivatives thereof. According to certain embodiments, compound (I) may be used in the treatment of a cancer or cell proliferation disorder in combination therapy with any one of the drugs selected from a group consisting of an alkaloid, an alkylating agent, an antitumor antibiotic, an antimetabolite, an Bcr-Abl tyrosine kinase inhibitor, a nucleoside analogue, a multidrug resistance reversing agent, a DNA binding agent, microtubule binding drug, a toxin and a DNA antagonist. Those of skill in the art will recognize the chemotherapeutic agents classified into one or more particular classes of chemotherapeutic agents described above.

According to preferred embodiments, compound (I) may be used in the treatment of a cancer or cell proliferation disorder in combination therapy with one or more agents selected from the group consisting of antimetabolites (*e.g.*, gemcitabine), inhibitors of topoisomerase I and II, alkylating agents and microtubule inhibitors (*e.g.*, taxol), as well as tyrosine kinase inhibitors (*e.g.,* surafenib), EGF kinase inhibitors (*e.g.*, tarceva or erlotinib), platinum complexes (*e.g.,* oxaliplatin); and ABL kinase inhibitors (*e.g.,* Gleevec or Imatinib).

Alkaloids include, but are not limited to, docetaxel, etoposide, irinotecan, paclitaxel (Taxol), teniposide, topotecan, vinblastine, vincristine, vindesine.

Alkylating agents include, but are not limited to, busulfan, improsulfan, piposulfan, benzodepa, carboquone, meturedepa, uredepa, altretamine, triethylenemelamine, triethylenephosphoramide, triethylenethiophosphoramide, chlorambucil, chloranaphazine, cyclophosphamide, estramustine, ifosfamide, mechlorethamine, mechlorethamine oxide HCl, melphalan novemebichin, perfosfamide phenesterine, prednimustine, trofosfamide, uracil mustard, carmustine, chlorozotocin, fotemustine, lomustine, nimustine, semustine ranimustine, dacarbazine, mannomustine, mitobronitol,

mitolactol, pipobroman, temozolomide.

Antibiotics and analogs thereof include, but are not limited to, aclacinomycins, actinomycins, anthramycin, azaserine, bleomycins, cactinomycin, carubicin, carzinophilin, cromomycins, dactinomycins, daunorubicin, 6-diazo-5-oxo-1-norleucine, doxorubicin, epirubicin, idarubicin, menogaril, mitomycins, mycophenolic acid, nogalamycine, olivomycins, peplomycin, pirarubicin, plicamycin, porfiromycin, puromycine, streptonigrin, streptozocin, tubercidin, zinostatin, zorubicin.

Antimetabolites include, but are not limited to, denopterin, edatrexate, mercaptopurine (6-MP), methotrexate, piritrexim, pteropterin, pentostatin (2'-DCF), tomudex, trimetrexate, cladridine, fludarabine, thiamiprine, ancitabine, azacitidine, 6-azauridine, carmofur, cytarabine, doxifluridine, emitefur, floxuridine, fluorouracil, gemcitabine, tegafur, hydroxyurea and urethan.

Platinum complexes include, but are not limited to, caroplatin, cisplatin, miboplatin, oxaliplatin.

Anti-mitotic agents or microtubule binding agents include, but are not limited to, vincristine, and vinblastine, and taxol.

When use in combination with additional anti-proliferation agents, compound (I) or a pharmaceutically acceptable salt thereof, may enhance (*e.g.*, synergize) the activity of these agents. Further, such synergism would permit compound (I), additional anti-proliferation agents, or both to be administered at lower dosages, and/or may significantly enhance the anti-proliferation properties of compounds at any given dose. The table below provides the results of combination treatments using compound (I) and additional anti-proliferation agents.

| Cell Line | Drug 1-$GI_{50}$ (nM) | Compound (I) $GI_{50}$ (nM) | Drug1:compound (I) $GI_{50}$ ratio | Drug1+compound (I) Combo $GI_{50}$ (nM) | Result |
|---|---|---|---|---|---|
| HT29 (Colon) | 1,480 (n=2) oxaliplatin | 25 (n=5) | 59 | 180 + 1.8 (used 100X) | Synergy, ca. 10X |
| SKOV-3 (Ovarian) | 3.9 (n=2) taxol | 9.8 (n=1) | 0.40 | 3.9 + 11 | No interference |
| A549 (Lung) | 1,735 (n=2) Tarceva | 13 (n=3) | 134 | 2,500 + 11 (used 233X) | No interference |
| L3.6pl (Pancreas) | 2.0 (n=2) Gemcitabine | 32 (n=4) | 1/13 | 0.09 + 1.15 | Synergy, ca. 25X |

In one embodiment, compound (I) or a pharmaceutically acceptable salt thereof, is used to treat or prevent or protect against brain cancer in a subject. Another aspect of the invention includes use of compound (I) or a pharmaceutically acceptable salt thereof, in the manufacture of a medicament to treat or prevent or protect against brain cancer. In order to prevent or protect against brain cancer, compound (I) or a pharmaceutically acceptable salt thereof, is administered prior to the development of brain cancer in a subject. Alternatively, the compound may be used to treat brain cancer in a subject. Compound (I) or a pharmaceutically acceptable salt thereof, used to treat or prevent or protect against brain cancer may be involved in modulating a kinase signaling cascade e.g., a kinase inhibitor, a non-ATP competitive inhibitor, a tyrosine kinase inhibitor, a protein kinase phosphatase inhibitor or a protein-tyrosine phosphates 1B inhibitor.

The term "brain cancer" encompasses a variety of cancers. There can be actual brain tumors which arise from the brain itself, known as primary brain cancers of which there are several. The term "brain cancer" refers to malignant tumors i.e., tumors that grow and spread aggressively, overpowering healthy cells by taking up their space, blood, and nutrients. Tumors that do not spread aggressively are called benign tumors. Benign tumors are generally less serious than a malignant tumor, but a benign tumor can still cause problems in the brain. There can also be brain metastases, which represent the spread of other cancers, such as lung or breast to the brain.

Brain tumors are classified by both the cell of the brain that makes them up and how the tumor looks under the microscope. Primary brain tumors arise from any of the cells in the brain, or from specific structures in the brain. Glia cells support the neurons of the brain and tumors which arise from these cells are known as glial tumors. The membrane that surrounds the brain can also develop tumors and these are known as meningiomas. There are other types of tumors, which involve other structures of the brain including ependymoma. The most common primary brain tumors are gliomas, meningiomas, pituitary adenomas, vestibular schwannomas, and primitive neuroectodermal tumors (medullablastomas).

The present invention provides a method of treating or preventing or protecting against glioblastoma, a malignant rapidly growing astrocytoma of the central nervous system and usually of a cerebral hemisphere. Synonyms for glioblastoma include glioblastoma multiforme (GBM), giant cell glioblastoma, and multiforme spongioblastoma multiforme. Gioblastoma is the most common malignant primary brain tumor and has proven very difficult to treat. These tumors

are often aggressive and infiltrate surrounding brain tissue. Glioblastomas arise from glial cells, which are cells that form the tissue that surrounds and protects other nerve cells found within the brain and spinal cord. Gioblastomas are mainly composed of star-shaped glial cells known as astrocytes. The term "glioma" includes any type of brain tumor such as astrocytomas, oligodendrogliomas, ependymomas, and choroid plexus papillomas. Astrocytomas come in four grades based on how fast the cells are reproducing and the likelihood that they will infiltrate nearby tissue. Grades I or II astrocytomas are nonmalignant and may be referred to as low-grade. Grades III and IV astrocytomas are malignant and may be referred to as high-grade astrocytomas. Grade II astrocytomas are known as anaplastic astrocytomas. Grade IV astrocytomas are known as glioblastoma multiforme.

The invention provides a method of treating or preventing or protecting against medulloblastoma. Medulloblastoma is a highly malignant primary brain tumor that originates in the cerebellum or posterior fossa. Originally considered to be a glioma, medulloblastoma is now known to be of the family of cranial primitive neuroectodermal tumors (PNET).

Tumors that originate in the cerebellum are referred to as infratentorial because they occur below the tentorium, a thick membrane that separates the cerebral hemispheres of the brain from the cerebellum. Another term for medulloblastoma is infratentorial PNET. Medulloblastoma is the most common PNET originating in the brain. All PNET tumors of the brain are invasive and rapidly growing tumors that, unlike most brain tumors, spread through the cerebrospinal fluid (CSF) and frequently metastasize to different locations in the brain and spine. The peak of occurrence of medullablastoma is seven years of age. Seventy percent of medulloblastomas occur in individuals younger than 16. Desmoplastic medulloblastoma is encountered especially in adulthood. This type of tumor rarely occurs beyond the fifth decade of life.

The present invention provides a method for treating or preventing or protecting against neuroblastoma, a cancer that forms in nerve tissue. The cells of neuroblastoma usually resemble very primitive developing nerve cells found in an embryo or fetus. The term neuro indicates "nerves," while blastoma refers to a cancer that affects immature or developing cells. Neurons (nerve cells) are the main component of the brain and spinal cord and of the nerves that connect them to the rest of the body. Neuroblastoma usually begins in the adrenal glands, but it may also begin in the spinal cord. Neuroblastoma is the most common extracranial solid cancer in childhood. In 2007, neuroblasoma was the most common cancer in infancy, with an annual incidence of about 650 new cases per year in the US. Close to 50 percent of neuroblastoma cases occur in children younger than two years old. It is a neuroendocrine tumor, arising from any neural crest element of the sympathetic nervous system or SNS. A branch of the autonomic nervous system, the SNS is a nerve network that carries messages from the brain throughout the body and is responsible for the fight-or-flight response and production of adrenaline or epinephrine.

The invention provides a method of treating or preventing or protecting against neuroepithelioma, malignant tumors of the neuroepithelium. Neuroepithelioma is found most commonly in children and young adults. It arises most often in the chest wall, pelvis, or extremity, either in bone or soft tissue. Procedures used in the diagnosis may include blood and urine tests, X rays of the affected bone and the whole body and lungs, bone marrow aspirations, CT scans, and fluoroscopy. Treatments include surgery, radiation therapy and chemotherapy. Ewing's tumors are an example of a type of peripheral neuroepithelioma.

Kinases have been shown to play a role in brain cancers. Gene expression profiles of glioblastoma multiforme have identified tyrosine kinases as playing a role in glioma migration/invasion. For example, PYK2 is a member of the focal adhesion family of nonrecptor tyrosine kinases; it is closely involved with src-induced increased actin polymerization at the fibroblastic cell periphery. Its role in glioma migration/invasion has become more clear, as overexpression of PYK2 induced glioblastoma cell migration in culture. Levels of activated PYK2 positively correlated with the migration phenotype in four glioblastoma cell lines (SF767, G112, T98G and U118). Analysis of activated PYK2 in GBM invastion in situ revealed strong staining in infiltrating GBM cells. (*See,* Hoelzinger et al, Neoplasia, vol. 7(1)7-16. Thus, modulation of a kinase receptor using compound (I) or a pharmaceutically acceptable salt thereof, may be useful in the protecting against, prevention or treatment of brain cancers such as glioblastoma multiforme.

Alternatively, compound (I) or a pharmaceutically acceptable salt thereof, may be used to treat or prevent or protect against renal cancer in a subject. Another aspect of the invention includes compound (I) or a pharmaceutically acceptable salt thereof, in the manufacture of a medicament to treat or prevent or protect against renal cancer. In order to prevent or protect against renal cancer, compound (I) or a pharmaceutically acceptable salt thereof, is administered prior to the development of renal cancer in a subject. Alternatively, compound (I) or a pharmaceutically acceptable salt thereof, may be used to treat renal cancer in a subject.

Several types of cancer can develop in the kidneys. Renal cell carcinoma (RCC), the most common form, accounts for approximately 85% of all cases. The present invention provides a method of treating or preventing renal cell carcinoma. The invention also provides a method for the treatment of other types of kidney cancer including, for example, renal pelvis carcinoma (cancer that forms in the center of the kidney where urine collects), Wilms tumors, which are a type of kidney cancer that usually develops in children under the age of 5, clear cell carcinoma also called clear cell adenocarcinoma and mesonephroma (a tumor type, usually of the female genital tract, in which the inside of the cells look clear when viewed under a microscope), renal adenocarcinoma (a type of kidney tumor characterized by

the development of finger-like projections in at least some of the tumor), and renal rhabdomyosarcoma, a rare and highly aggressive tumor in the adult population.

In RCC, cancerous (malignant) cells develop in the lining of the kidney's tubules and grow into a tumor mass. In most cases, a single tumor develops, although more than one tumor can develop within one or both kidneys. RCC is characterized by a lack of early warning signs, diverse clinical manifestations, resistance to radiation and chemotherapy, and infrequent but reproducible responses to immunotherapy agents such as interferon alpha and interleukin (IL)-2. In the past, RCC tumors were believed to derive from the adrenal gland; therefore, the term hypernephroma was used often.

The tissue of origin for renal cell carcinoma is the proximal renal tubular epithelium. Renal cancer occurs in both a sporadic (nonhereditary) and a hereditary form, and both forms are associated with structural alterations of the short arm of chromosome 3 (3p). Genetic studies of the families at high risk for developing renal cancer led to the cloning of genes whose alteration results in tumor formation. These genes are either tumor suppressors (*VHL, TSC*) or oncogenes (*MET*). At least 4 hereditary syndromes associated with renal cell carcinoma are recognized: (1) von Hippel-Lindau (VHL) syndrome, (2) hereditary papillary renal carcinoma (HPRC), (3) familial renal oncocytoma (FRO) associated with Birt-Hogg-Dube syndrome (BHDS), and (4) hereditary renal carcinoma (HRC).

RCC has a very poor prognosis, mainly because, in nearly 30% of all patients with localized disease, 40% of them develop distant metastases following removal of the primary tumor. The age-adjusted incidence of renal cell carcinoma has been rising by 3% per year. According to the American Cancer Society, in 2007 there were approximately 51,500 cases of malignant tumors of the kidney diagnosed in the United States with approximately 12,500 deaths; renal cell cancer accounted for 80% of this incidence and mortality. Radical nephrectomy is the main treatment for localized RCC. However radiotherapy and available chemotherapeutic agents are ineffective against advanced and metastic RCC.

Immunotherapy using interferon-$\alpha$ and interluckin-2 is effective in only a small percentage of patients with metastatic RCC and is extremely toxic. Recently, kinase inhibitors have been developed for the treatment of renal cancer *e.g.,* Gleevec® and other new agents, such as sorafenib and sunitinib, having anti-angiogenic effects through targeting multiple receptor kinases, have shown activity in patients failing immunotherapy. However, these treatments are also not without limitations. For example, it's been found that the effect of Gleevec® is limited to a certain type of tumor and resistance can develop. Also, it is recommended that patients taking sunitinib should be monitored for cardiovascular side effects such as hypertension. As such, a need exists for the development of methods for the treatment and prevention of renal cancer.

Alternatively, compound (I) or a pharmaceutically acceptable salt thereof, may be used to treat or prevent or protect against liver cancer in a subject. Another aspect of the invention includes use of compound (I) or a pharmaceutically acceptable salt thereof, in the manufacture of a medicament to treat or prevent or protect against liver cancer. In order to prevent or protect against liver cancer, compound (I) or a pharmaceutically acceptable salt thereof, is administered prior to the development of liver cancer in a subject. Alternatively, compound (I) or a pharmaceutically acceptable salt thereof, may be used to treat liver cancer in a subject.

Several types of cancer can develop in the liver. Hepatocellular carcinoma (HCC) accounts for 80-90% of all liver cancers. The present invention provides a method of treating or preventing hepatocellular carcinoma. HCC begins in the hepatocytes, the main type of liver cell. About 3 out of 4 primary liver cancers are this type. HCC can have different growth patterns. Some begin as a single tumor that grows larger. Only late in the disease does it spread to other parts of the liver. HCC may also begin in many spots throughout the liver and not as a single tumor.

The invention also provides a method for the treatment of other types of liver cancer including, for example, cholangiocarcinomas, which starts in the bile ducts of the gallbladder; angiosarcomas and hemangiosarcomas are two other forms of cancer that begin in the blood vessels of the liver. These tumors grow quickly. Often by the time they are found they are too widespread to be removed and treatment may not help very much; hepatoblastoma is a cancer that develops in children, usually found in children younger than 4 years old.

Kinases have been shown to play a role in liver cancer. For example, changes known to occur in human HCC are overexpression, amplification or mutation of the protooncogene MET, which encodes the receptor protein tyrosine kinase Met (*See,* Tward et al., PNAS, vol. 104(37)14771-14776). It has also been demonstrated that FAK is involved in early events of integrin-mediated adhesion of circulating carcinoma cells under fluid flow in vitro and in vivo. It is thought that this kinase may take part in the establishment of definite adhesion interactions that enable adherent tumor cells to resist shear forces (*See,* Sengbusch et al., American Journal of Pathology, vol 166(2)585-595). In 2007, liver cancer was the third leading cause of cancer-related deaths worldwide, and the sixth most widespread cancer globally. 600,000 people are annually are diagnosed with liver cancer worldwide and the incidence is rising. Accordingly, a need exists for the development of methods for the treatment, prevention, and protection against liver cancer.

According to another embodiment, there is provided a method for treating, preventing or protecting against leukemia in a host comprising administering to a patient compound (I). In another embodiment, there is provided a method for treating leukemia in a host comprising administering to a patient an effective amount of compound (I) and at least one further therapeutic agent selected from the group consisting of anti-proliferative agents, cytotoxic agents, cytostatic agents, and chemotherapeutic agents and salts and derivatives thereof. According to certain embodiments,

compound (I) may be used in the treatment of a leukemia in combination therapy with one or more of the drugs selected from a group consisting of an alkaloid, an alkylating agent, an antitumor antibiotic, an antimetabolite, an Bcr-Abl tyrosine kinase inhibitor, a nucleoside analogue, a multidrug resistance reversing agent, a DNA binding agent, microtubule binding drug, a toxin and a DNA antagonist. Those of skill in the art will recognize the chemotherapeutic agents classified into one or more particular classes of drugs described above.

Leukemia is a malignant cancer of the bone marrow and blood and is characterized by the uncontrolled growth of blood cells. The common types of leukemia are divided into four categories: acute or chronic myelogenous, involving the myeloid elements of the bone marrow (white cells, red cells, megakaryocytes) and acute or chronic lymphocytic, involving the cells of the lymphoid lineage. Treatment of leukemia generally depends upon the type of leukemia. Standard treatment for leukemia usually involves chemotherapy and/or bone marrow transplantation and/or radiation therapy. See *e.g.,* U.S. Patent No. 6,645,972, hereby incorporated herein by reference in its entirety.

Chemotherapy in leukemia may involve a combination of two or more anti-cancer drugs. Approximately 40 different drugs are now being used in the treatment of leukemia, either alone or in combination. Other treatments for leukemia also include the reversal of multidrug resistance, involving the use of agents which decrease the mechanisms allowing the malignant cells to escape the damaging effects of the chemotherapeutic agent (and leads to refractoriness or relapses); and biological therapy, involving the use of monoclonal antibodies, in which toxins are attached to antibodies that react with the complementary antigen carried by the malignant cells; and cytokines (*e.g.*, interferons, interleukins, colony-stimulating factors CSFs) which are naturally occurring chemicals that stimulate blood cell production and help restore blood cell counts more rapidly after treatment. Examples of these drugs include multidrug resistance reversing agent PSC 833, the monoclonal antibody Rituxan and the following cytokines: Erythropoetin and Epoetin, which stimulate the production of red cells; G-CSF, GM-CSF, filgrastim, and Sargramostim which stimulate the production of white cells; and thrombopoietin, which stimulate the production of platelets.

Many nucleoside analogues have been found to possess anticancer activity. Cytarabine, Fludarabine, Gemcitabine and Cladribine are some examples of nucleoside analogues which are currently important drugs in the treatment of leukemia. β-L-OddC ((-)-β-L-Dioxolane-Cytidine, Troxatyl®, from Shire BioChem Inc.) is also a nucleoside analogue which was first described as an antiviral agent by Belleau *et al.* (EP 337713, herein incorporated by reference in its entirety) and was shown to have potent antitumor activity (K. L. Grove et al., Cancer Res., 55(14), 3008-11, 1995; K. L. Grove et al., Cancer Res., 56(18), 4187-4191, 1996, K. L. Grove et al., Nucleosides Nucleotides, 16:1229-33, 1997; S. A Kadhim et al., Can. Cancer Res., 57(21), 4803-10, 1997). In clinical studies, β-L-OddC has been reported to have significant activity in patients with advanced leukemia (Giles et al., J. Clin. Oncology, Vol 19, No 3, 2001).

Bcr-Abl tyrosine kinase inhibitors, such as STI-571 (Gleevec®, Imatinib mesylate, from Novartis Pharmaceuticals Corp.), have shown significant antileukemic activity and specifically in chronic myeologenous leukemia. STI-571, for example, has become a promising therapy in the group of patients targeting Bcr-Abl tyrosine kinase inhibition. However, despite significant hematologic and cytogenic responses, resistance to Bcr-Abl tyrosine kinase inhibitors occurs, particularly in the advanced phases of chronic myelogenous leukemia. Such resistance has been demonstrated for the Bcr-Abl tyrosine kinase inhibitors Imatinib, Dasatinib, AZD0530.

Accordingly, there is a great need for the further development of agents for the treatment of leukemia patients who have been previously treated with a Bcr-Abl tyrosine kinase inhibitor and have become resistant to the Bcr-Abl tyrosine kinase inhibitor. Thus, in another embodiment, there is provide a method for treating leukemia in a host comprising administering to a patient that has been previously treated with a Bcr-Abl tyrosine kinase inhibitor and has become resistant to the Bcr-Abl tyrosine kinase inhibitor treatment, an amount of compound (I). Further, there is provide a method for combination therapy of leukemia in a host comprising administering to a patient a Bcr-Abl tyrosine kinase inhibitor in combination with an amount of compound (I). In one embodiment, the combination is administered to a patient that has become resistant to the Bcr-Abl tyrosine kinase inhibitor treatment.

Compound (I) displays anti-leukemia activity as compared to existing therapeutic agents, as is shown in the table below.

| Human Leukemia Cell Line | Compound (I) GI50 (nM) | Dasatinib GI50 (nM) |
|---|---|---|
| K562 (CML) | 13 (n=2) | 0.37 (n=1-2) |
| K562R (Gleevec resistant CML) | 0.64 (n=1-2) | 0.81 (n=2) |
| MOLT-4 (Adult lymphoblastic leukemia) | 13 (n=1) | 644 (n=1) |
| CCRF-HSB-2 (Adult lymphoblastic leukemia) | 12 (n=1) | Inactive (n=1) |
| Jurkat (Adult T cell leukemia) | 10 (n=1) | 8 (n=1) |
| Ba/F3 (IL-3 induced) | 3.5 | Inactive |

(continued)

| Human Leukemia Cell Line | Compound (I) GI50 (nM) | Dasatinib GI50 (nM) |
|---|---|---|
| Ba/F3 + WT BCR-Abl | 85 | 1 |
| Ba/F3 + BCR-Abl E225K mutant | 80 | 1 |
| Ba/F3 + BCR-Abl T315I mutant | 35 | >10,000 |

Compound (I) or a pharmaceutically acceptable salt thereof, may be used for other cell proliferation-related disorders such as psoriases.

As described herein, compound (I) or a pharmaceutically acceptable salt thereof, may be used to treat or protect against or prevent hearing loss in a subject. Another aspect of the invention includes use of compound (I) or a pharmaceutically acceptable salt thereof, in the manufacture of a medicament to prevent or protect against or treat hearing loss. In order to protect or prevent against hearing loss, the compound may be administered prior to noise exposure or exposure to a drug which induces hearing loss. Such drugs may include chemotherapeutic drugs (e.g., platinum-based drugs which target hair cells) and aminoglycoside antibiotics. Compound (I) or a pharmaceutically acceptable salt thereof, may provide a synergistic effect with certain cancer drugs. For example, promising inhibitors can be screened in primary human tumor tissue assays, particularly to look for synergy with other known anti-cancer drugs. In addition, the protein kinase inhibitors may reduce toxicity of certain cancer drugs (e.g., platinum-based drugs which are toxic to the cochlea and kidney), thereby allowing increased dosage.

Alternatively, compound (I) or a pharmaceutically acceptable salt thereof, may be used to treat hearing loss in a subject. In this embodiment, the compound is administered to the subject subsequent to the initiation of hearing loss to reduce the level of hearing loss. Compound (I) or a pharmaceutically acceptable salt thereof, may be involved in modulating a kinase cascade, e.g. a kinase inhibitor, a non-ATP competitive inhibitor, a tyrosine kinase inhibitor, a Src inhibitor or a focal adhesion kinase (FAK) modulator. Although not wishing to be bound by theory, it is believed that the administration of kinase inhibitors prevents apoptosis of cochlear hair cells, thereby preventing hearing loss. In one embodiment, compound (I) or a pharmaceutically acceptable salt thereof, is administered to a subject suffering from hearing loss in order to prevent further hearing loss. In another embodiment, compound (I) or a pharmaceutically acceptable salt thereof, is administered to a subject suffering from hearing loss in order to restore lost hearing. In particular, following noise exposure, the tight cell junctures between the cochlear hair cells, as well as the cell-extracellular matrix interaction, are torn and stressed. The stressing of these tight cell junctures initiates apoptosis in the cells through a complex signaling pathway in which tyrosine kinases act as molecular switches, interacting with focal adhesion kinase to transduce signals of cell-matrix disruptions to the nucleus. It is believed that the administration of kinase inhibitors prevents the initiation of apoptosis in this cascade.

The identification of apoptosis in the noise-exposed cochlea has generated a number of new possibilities for the prevention of noise-induced hearing loss (NIHL) (Hu, et al.; 2000, Acta. Otolaryngol., 120, 19-24). For example, the ear can be protected from NIHL by administration of antioxidant drugs to the round window of the ear (Hight, et al.; 2003, Hear. Res., 179, 21-32; Hu, et al.; Hear. Res. 113, 198-206). Specifically, NIHL has been reduced by the administration of FDA-approved antioxidant compounds (N-L-acetylcysteine (L-NAC) and salicylate) in the chinchilla (Kopke, et al.; 2000, Hear. Res., 149, 138-146). Moreover, Harris et al. have recently described prevention of NIHL with Src-PTK inhibitors (Harris, et al.; 2005, Hear. Res., 208, 14-25). Thus, it is hypothesized that the administration of a compound of the instant invention which modulates the activity of kinases, is useful for treating hearing loss.

Changes in cell attachment or cell stress can activate a variety of signals through the activation of integrins and through the phosphorylation of PTKs, including the Src family of tyrosine kinases. Src interactions have been linked to signaling pathways that modify the cytoskeleton and activate a variety of protein kinase cascades that regulate cell survival and gene transcription (reviewed in Giancotti and Ruoslahti; 1999, Science, 285, 1028-1032). In fact, recent results have indicated that outer hair cells (OHC), which had detached at the cell base following an intense noise exposure, underwent apoptotic cell death. Specifically, the Src PTK signaling cascade is thought to be involved in both metabolic- and mechanically-induced initiation of apoptosis in sensory cells of the cochlea. In a recent study, Src inhibitors provided protection from a 4 hour, 4 kHz octave band noise at 106 dB, indicating that Src-PTKs might be activated in outer hair cells following noise exposure (Harris, et al.; 2005, Hear. Res., 208, 14-25). Thus, compound (I) or a pharmaceutically acceptable salt thereof, that modulate the activity of Src, is useful in treating hearing loss.

The present invention relates to a method for preventing or protecting against or treating osteoporosis in a subject. Another aspect of the invention includes use of compound (I) or a pharmaceutically acceptable salt thereof, in the manufacture of a medicament to prevent or protect against or treat osteoporosis. This method involves administering an effective amount of compound (I) or a pharmaceutically acceptable salt thereof, to the subject to prevent or protect against or to treat osteoporosis. In order to prevent or protect against osteoporosis, compound (I) or a pharmaceutically

acceptable salt thereof, is administered prior to the development of osteoporosis. Alternatively, compound (I) or a pharmaceutically acceptable salt thereof, may be used to treat osteoporosis in a subject. In this embodiment, compound (I) or a pharmaceutically acceptable salt thereof, is administered to the subject subsequent to the initiation of osteoporosis to reduce the level of osteoporosis.

It has been shown that Src deficiency is associated with osteoporosis in mice, because of loss of osteoclast function (Soriano, et al.; 1991, Cell, 64, 693-702). It is also know that mice that lack IRAK-M develop severe osteoporosis, which is associated with the accelerated differentiation of osteoclasts, an increase in the half-life of osteoclasts, and their activation (Hongmei, et al.; 2005, J. Exp. Med., 201, 1169-1177).

Multinucleated osteoclasts originate from the fusion of mononuclear phagocytes and play a major role in bone development and remodeling via the resorption of bone. Osteoclasts are multinucleated, terminally differentiated cells that degrade mineralized matrix. In normal bone tissue, there is a balance between bone formation by osteoblasts and bone resorption by osteoclasts. When the balance of this dynamic and highly regulated process is disrupted, bone resorption can exceed bone formation resulting in quantitative bone loss. Because osteoclasts are essential for the development and remodeling of bone, increases in their number and /or activity lead to diseases that are associated with generalized bone loss (e.g., osteoporosis) and others with localized bone loss (e.g., rheumatoid arthritis, periodontal disease).

Osteoclasts and osteoblasts both command a multitude of cellular signaling pathways involving protein kinases. Osteoclast activation is initiated by adhesion to bone, cytoskeletal rearrangement, formation of the sealing zone, and formation of the polarized ruffled membrane. It is believed that protein-tyrosine kinase 2 (PYK2) participates in the transfer of signals from the cell surface to the cytoskeleton, as it is tyrosine phosphorylated and activated by adhesion-initiated signaling in osteoclasts (Duong, et al.; 1998, J. Clin. Invest., 102, 881-892). Recent evidence has indicated that the reduction of PYK2 protein levels results in the inhibition of osteoclast formation and bone resorption *in vitro* (Duong, et al.; 2001, J. Bio. Chem., 276, 7484-7492). Therefore, the inhibition of PYK2 or other protein tyrosine kinases might reduce the level of osteoporosis by decreasing osteoclast formation and bone resorption. Thus, without wishing to be bound by theory, it is hypothesized that the administration of compound (I) or a pharmaceutically acceptable salt thereof, will modulate kinase (e.g. PTK) activity and therefore result in the inhibition of osteoclast formation and/or bone resorption, thereby treating osteoporosis.

Src tyrosine kinase stands out as a promising therapeutic target for bone disease as validated by Src knockout mouse studies and in vitro cellular experiments, suggesting a regulatory role for Src in both osteoclasts (positive) and osteoblasts (negative). In osteoclasts, Src plays key roles in motility, polarization, survival, activation (ruffled border formation) and adhesion, by mediating various signal transduction pathways, especially in cytokine and integrin signaling (Parang and Sun; 2005, Expert Opin. Ther. Patents, 15, 1183-1207). Moreover, targeted disruption of the *src* gene in mice induces osteopetrosis, a disorder characterized by decreased bone resorption, without showing any obvious morphological or functional abnormalities in other tissues or cells (Soriano, et al.; 1991, Cell, 64, 693-702). The osteopetrotic phenotype of *src*$^{-/-}$ mice is cell-autonomous and results from defects in mature osteoclasts, which normally express high levels of Src protein (Home, et al.; 1991, Cell, 119, 1003-1013). By limiting the effectiveness of Src tyrosine kinase, which triggers osteoclast activity and inhibits osteoblasts, Src inhibitors are thought to lessen bone break down and encourage bone formation. Because osteoclasts normally express high levels of Src, inhibition of Src kinase activity might be useful in the treatment of osteoporosis (Missbach, et al.; 1999, Bone, 24, 437-449).

As described herein, compound (I) or a pharmaceutically acceptable salt thereof, may be used to treat, protect against or prevent obesity in a subject. Another aspect of the invention includes use of compound (I) or a pharmaceutically acceptable salt thereof, in the manufacture of a medicament to prevent, protect against or to treat obesity. In order to prevent or protect against obesity, the compound is administered prior to the development of obesity in a subject. Alternatively, compound (I) or a pharmaceutically acceptable salt thereof, may be used to treat obesity in a subject.

Obesity is associated with diabetes and increased insulin resistance in insulin responsive tissues, such as skeletal muscle, liver, and white adipose tissue (Klaman, et al.; 2000, Mol. Cell. Biol., 20, 5479-5489). Insulin plays a critical role in the regulation of glucose homeostasis, lipid metabolism, and energy balance. Insulin signaling is initiated by binding of insulin to the insulin receptor (IR), a receptor tyrosine kinase. Insulin binding evokes a cascade of phosphorylation events, beginning with the autophosphorylation of the IR on multiple tyrosyl residues. Autophosphorylation enhances IR kinase activity and triggers downstream signaling events. The stimulatory effects of protein tyrosine kinases and the inhibitory effects of protein tyrosine phosphatases largely define the action of insulin. Appropriate insulin signaling minimizes large fluctuations in blood glucose concentrations and ensures adequate delivery of glucose to cells. Since insulin stimulation leads to multiple tyrosyl phosphorylation events, enhanced activity of one or more protein-tyrosine phosphatases (PTPs) could lead to insulin resistance, which may lead to obesity. Indeed, increased PTP activity has been reported in several insulin-resistant states, including obesity (Ahmad, et al.; 1997, Metabolism, 46, 1140-1145). Thus, without wishing to be bound by theory, the administration compound (I) or a pharmaceutically acceptable salt thereof, modulates kinase (e.g., PTP) activity, thereby treating obesity in a subject.

Insulin signaling begins with the activation of the IR via tyrosine phosphorylation and culminates in the uptake

of glucose into cells by the glucose transporter, GLUT4 (Saltiel and Kahn; 2001, Nature, 414, 799-806). The activated IR must then be deactivated and returned to a basal state, a process that is believed to involve protein-tyrosine phosphatase-1B (PTP-1B) (Ahmad, et al; 1997, J. Biol. Chem., 270, 20503-20508). Disruption of the gene that codes for PTP-1B in mice results in sensitivity to insulin and increased resistance to diet-induced obesity (Elchebly, et al.; 1999, Science, 283, 1544-1548; Klaman, et al.; 2000, Mol. Cell. Biol., 20, 5479-5489). The decreased adiposity in PTP-1B deficient mice was due to a marked reduction in fat cell mass without a decrease in adipocyte number (Klaman, et al.; 2000, Mol. Cell. Biol., 20, 5479-5489). Moreover, leanness in PTP-1B-deficient mice was accompanied by increased basal metabolic rate and total energy expenditure, without marked alteration of uncoupling protein mRNA expression. The disruption of the PTP-1B gene demonstrated that altering the activity of PTP-1B can modulate insulin signaling and dietary-induced obesity *in vivo.* Thus, without wishing to be bound by theory, the administration compound (I) or a pharmaceutically acceptable salt thereof, that modulates insulin signaling (e.g., PTP-1B activity), is useful in treating obesity in a subject.

Compound (I) or a pharmaceutically acceptable salt thereof, may be used to prevent or protect against or to treat diabetes in a subject. Another aspect of the invention includes use of compound (I) or a pharmaceutically acceptable salt thereof, in the manufacture of a medicament to prevent, protect against, or treat diabetes. In order to prevent or protect against diabetes, compound (I) or a pharmaceutically acceptable salt thereof, is administered prior to the development of diabetes in a subject. Alternatively, compound (I) or a pharmaceutically acceptable salt thereof, may be used to treat diabetes in a subject.

Type 2 diabetes mellitus (T2DM) is a disorder of dysregulated energy metabolism. Energy metabolism is largely controlled by the hormone insulin, a potent anabolic agent that promotes the synthesis and storage of proteins, carbohydrates and lipids, and inhibits their breakdown and release back into the circulation. Insulin action is initiated by binding to its tyrosine kinase receptor, which results in autophosphorylation and increased catalytic activity of the kinase (Patti, et al.; 1998, J. Basic Clin. Physiol. Pharmacol. 9, 89-109). Tyrosine phosphorylation causes insulin receptor substrate (IRS) proteins to interact with the p85 regulatory subunit of phosphatidylinositol 3-kinase (PI3K), leading to the activation of the enzyme and its targeting to a specific subcellular location, depending on the cell type. The enzyme generates the lipid product phosphatidylinositol-3,4,5-trisphosphate (PtdIns(3,4,5)$P_3$), which regulates the localization and activity of numerous proteins (Kido, et al.; 2001, J. Clin. Endocrinol. Metab., 86, 972-979). PI3K has an essential role in insulin-stimulated glucose uptake and storage, inhibition of lipolysis and regulation of hepatic gene expression (Saltiel, et al.; 2001, Nature, 414, 799-806). Overexpression of dominant-interfering forms of PI3K can block glucose uptake and translocation of glutamate transporter four, GLUT4, to the plasma membrane (Quon, et al.; 1995, Mol. Cell. Biol., 15, 5403-5411). Thus, the administration of a compound of the instant invention that modulates kinase (e.g. PI3K) activity, and therefore results in increased glucose uptake, is useful in treating diabetes.

PTEN is a major regulator of PI3K signaling in may cell types, and functions as a tumor suppressor due to antagonism of the anti-apoptotic, proliferative and hypertrophic activities of the PI3K pathway (Goberdhan, et al.; 2003, Hum. Mol. Genet., 12, R239-R248; Leslie, et al.; 2004, J. Biochem., 382, 1-11). Although not wishing to be bound by theory, it is believed that PTEN attenuates the PI3K pathway by dephosphorylation of the PtdIns(3,4,5)$P_3$ molecule, degrading this important lipid second messenger to PtdIns(4,5)$P_2$. In a recent study, reduction of endogenous PTEN protein by 50% using small interfering RNA (siRNA) enhanced insulin-dependent increases in PtdIns(3,4,5)$P_3$ levels, and glucose uptake (Tang, et al.; 2005, J. Biol. Chem., 280, 22523-22529). Thus, without wishing to be bound by theory, it is hypothesized that the administration of compound (I) or a pharmaceutically acceptable salt thereof, that modulates PTEN activity, and therefore results in increased glucose uptake, is useful for treating diabetes.

PtdIns(3,4,5)$P_3$ levels are also controlled by the family of SRC homology 2 (SH2)- containing inositol 5'-phosphatase (SHIP) proteins, SHIP1 and SHIP2 (Lazar and Saltiel; 2006, Nature Reviews, 5, 333-342). SHIP2, expressed in skeletal muscle, among other insulin-sensitive tissues, catalyzes the conversion of PtdIns(3,4,5)$P_3$ into PtdIns(3,4)$P_2$ (Pesesse, et al.; 1997; Biochem Biophys. Res. Commun., 239, 697-700; Backers, et al.; 2003, Adv. Enzyme Regul., 43, 15-28; Chi, et al.; 2004, J. Biol. Chem., 279, 44987-44995; Sleeman, et al.; 2005, Nature Med., 11, 199-205). Overexpression of SHIP2 markedly reduced insulin-stimulated PtdIns(3,4,5)$P_3$ levels, consistent with the proposed capacity of SHIP2 to attenuate the activation of downstream effectors of PI3K (Ishihara, et al.; 1999, Biochem. Biophys. Res. Commun., 260, 265-272).

As described herein, compound (I) or a pharmaceutically acceptable salt thereof, may be used to treat, protect against or prevent eye disease in a subject. Another aspect of the invention includes use compound (I) or a pharmaceutically acceptable salt thereof, in the manufacture of a medicament to treat, protect against or prevent eye disease. In order to protect against or prevent eye disease, the compound is administered prior to the development of eye disease in a subject. Alternatively, compound (I) or a pharmaceutically acceptable salt thereof, may be used to treat eye disease in a subject, e.g. macular degeneration, retinopathy, and macular edema.

Vision-threatening neovascularization of the physiologically avascular cornea can occur. The proliferative retinopathies, principally diabetic retinopathy and age-related macular degeneration, are characterized by increased vascular permeability, leading to retinal edema and subretinal fluid accumulation, and the proliferation of new vessels that are

prone to hemorrhage. Angiogenesis, the formation of new blood vessels from preexisting capillaries, is an integral part of both normal development and numerous pathological processes. VEGF, a central mediator of the complex cascade of angiogenesis and a potent permeability factor, is an attractive target for novel therapeutics. VEGF is the ligand for two membrane-bound tyrosine kinase receptors, VEGFR-1 and VEGFR-2. Ligand binding triggers VEGFR dimerization and transphosphorylation with subsequent activation of an intracellular tyrosine kinase domain. The ensuing intracellular signaling axis results in vascular endothelial cell proliferation, migration, and survival. Thus, without wishing to be bound by theory, it is hypothesized that the administration of a compound of the instant invention which modulates kinase activity, e.g. tyrosine kinase activity, and results in the inhibition of angiogenesis and/or neovascularization, is useful for treating an eye disease, e.g. macular degeneration, retinopathy and/or macular edema.

Macular degeneration is characterized by VEGF-mediated retinal leakage (an increase in vascular permeability) and by the abnormal growth of small blood vessels in the back of the eye (angiogenesis). VEGF has been identified in neovascular membranes in both diabetic retinopathy and age-related macular degeneration, and intraocular levels of the factor correlate with the severity of neovascularization in diabetic retinopathy (Kvanta, et al.; 1996, Invest. Ophthal. Vis. Sci., 37, 1929-1934.; Aiello, et al.; 1994, N. Engl. J. Med., 331, 1480-1487). Therapeutic antagonism of VEGF in these models results in significant inhibition of both retinal and choroidal neovascularization, as well as a reduction in vascular permeability (Aiello, et al.; 1995, Proc. Natl. Acad. Sci. USA., 92, 10457-10461; Krzystolik, et al.; 2002, Arch. Ophthal., 120, 338-346; Qaum, et al.; 2001, Invest. Ophthal. Vis. Sci., 42, 2408-2413). Thus, without wishing to be bound by theory, it is hypothesized that the administration of compound (I) or a pharmaceutically acceptable salt thereof, which modulates VEGF activity, and results in the inhibition of angiogenesis and/or neovascularization, is useful for treating an eye disease, e.g. macular degeneration, retinopathy and/or macular edema.

Compound (I) or a pharmaceutically acceptable salt thereof, is used in methods to prevent or protect against or treat a stroke in a subject who is at risk of suffering a stroke, is suffering from a stroke or has suffered a stroke. Compound (I) or a pharmaceutically acceptable salt is useful in methods of treating patients who are undergoing post-stroke rehabilitation. Another aspect of the invention includes use of compound (I) or a pharmaceutically acceptable salt thereof, in the manufacture of a medicament to treat, prevent, or protect against stroke.

A stroke, also known as a cerebrovascular accident (CVA), is an acute neurological injury whereby the blood supply to a part of the brain is interrupted due to either blockage of an artery or rupture of a blood vessel. The part of the brain in which blood supply is interrupted no longer receives oxygen and/or nutrients carried by the blood. The brain cells become damaged or necrotic, thereby impairing function in or from that part of the brain. Brain tissue ceases to function if deprived of oxygen for more than 60 to 90 seconds and after a few minutes will suffer irreversible injury possibly leading to a death of the tissue, *i.e.,* infarction.

Strokes are classified into two major types: ischemic, *i.e.*, blockage of a blood vessel supplying the brain, and hemorrhagic, *i.e.,* bleeding into or around the brain. The majority of all strokes are ischemic strokes. Ischemic stroke is commonly divided into thrombotic stroke, embolic stroke, systemic hypoperfusion (Watershed stroke), or venous thrombosis. In thrombotic stroke, a thrombus-forming process develops in the affected artery, the thrombus, *i.e.,* blood clot, gradually narrows the lumen of the artery, thereby impeding blood flow to distal tissue. These clots usually form around atherosclerotic plaques. There are two types of thrombotic strokes, which are categorized based on the type of vessel on which the thrombus is formed. Large vessel thrombotic stroke involves the common and internal carotids, vertebral, and the Circle of Willis. Small vessel thrombotic stroke involves the intracerebral arteries, branches of the Circle of Willis, middle cerebral artery stem, and arteries arising from the distal vertebral and basilar artery.

A thrombus, even if non-occluding, can lead to an embolic stroke if the thrombus breaks off, at which point it becomes an embolus. An embolus refers to a traveling particle or debris in the arterial bloodstream originating from elsewhere. Embolic stroke refers to the blockage of arterial access to a part of the brain by an embolus. An embolus is frequently a blood clot, but it can also be a plaque that has broken off from an atherosclerotic blood vessel or a number of other substances including fat, air, and even cancerous cells. Because an embolus arises from elsewhere, local therapy only solves the problem temporarily. Thus, the source of the embolus must be identified. There are four categories of embolic stroke: those with a known cardiac source; those with a potential cardiac or aortic source (from trans-thoracic or trans-esophageal echocardiogram); those with an arterial source; and those with unknown source.

Systemic hypoperfusion is the reduction of blood flow to all parts of the body. It is most commonly due to cardiac pump failure from cardiac arrest or arrhythmias, or from reduced cardiac output as a result of myocardial infarction, pulmonary embolism, pericardial effusion, or bleeding. Hypoxemia (*i.e.,* low blood oxygen content) may precipitate the hypoperfusion. Because the reduction in blood flow is global, all parts of the brain may be affected, especially the "watershed" areas which are border zone regions supplied by the major cerebral arteries. Blood flow to these area has not necessary stopped, but instead may have lessened to the point where brain damage occurs.

Veins in the brain function to drain the blood back to the body. When veins are occluded due to thrombosis, the draining of blood is blocked and the blood backs up, causing cerebral edema. This cerebral edema can result in both ischemic and hemorrhagic strokes. This commonly occurs in the rare disease sinus vein thrombosis.

Stroke is diagnosed in a subject or patient using one or more of a variety of techniques known in the art, such

as, for example, neurological examination, blood tests, CT scans (without contrast enhancements), MRI scans, Doppler ultrasound, and arteriography (*i.e.,* roentgenography of arteries after injection of radiopacque material into the blood stream). If a stroke is confirmed on imaging, various other studies are performed to determine whether there is a peripheral source of emboli. These studies include, *e.g.,* an ultrasound/doppler study of the carotid arteries (to detect carotid stenosis); an electrocardiogram (ECG) and echocardiogram (to identify arrhythmias and resultant clots in the heart which may spread to the brain vessels through the bloodstream); a Holter monitor study to identify intermittent arrhythmias and an angiogram of the cerebral vasculature (if a bleed is thought to have originated from an aneurysm or arteriovenous malformation).

Compound (I) or a pharmaceutically acceptable salt thereof, useful in these methods to treat, prevent or protect against a stroke or a symptom associated with stroke is a compound that modulates one or more kinase signaling cascades preceding, during or after a stroke. In one embodiment, compound (I) or a pharmaceutically acceptable salt thereof, used in the methods to treat, prevent or protect against a stroke or a symptom associated with stroke described herein is an allosteric inhibitor of kinase signaling cascade preceding, during or after a stroke. In one aspect, compound (I) or a pharmaceutically acceptable salt thereof, used in the methods to treat, prevent, or protect against a stroke or a symptom associated with stroke described herein is a non-ATP competitive inhibitor of kinase signaling cascade preceding, during or after a stroke.

Inhibition of Src activity has been shown to provide cerebral protection during stroke. (*See* Paul et al., Nature Medicine, vol. 7(2):222-227 (2001), which is hereby incorporated by reference in its entirety). Vascular endothelia growth factor (VEGF), which is produced in response to the ischemic injury, has been shown to promote vascular permeability. Studies have shown that the Src kinase regulates VEGF-mediated VP in the brain following stroke, and administration of an Src inhibitor before and after stroke reduced edema, improved cerebral perfusion and decreased infarct volume after injury occurred. (Paul *et al.,* 2001). Thus, Src inhibition may be useful in the prevention, treatment or amelioration of secondary damage following a stroke.

Compound (I) or a pharmaceutically acceptable salt thereof, prevents, treats or protects against a stroke or a symptom associated with stroke. Another aspect of the invention includes use of compound (I) or a pharmaceutically acceptable salt thereof, in the manufacture of a medicament to prevent, treat, or to protect against stroke or a symptom associated with stroke. Symptoms of a stroke include sudden numbness or weakness, especially on one side of the body; sudden confusion or trouble speaking or understanding speech; sudden trouble seeing in one or both eyes; sudden trouble with walking, dizziness, or loss of balance or coordination; or sudden severe headache with no known cause.

Generally there are three treatment stages for stroke: prevention, therapy immediately after the stroke, and post-stroke rehabilitation. Therapies to prevent a first or recurrent stroke are based on treating the underlying risk factors for stroke, such as, *.e.g.,* hypertension, high cholesterol, atrial fibrillation, and diabetes. Acute stroke therapies try to stop a stroke while it is happening by quickly dissolving the blood clot causing an ischemic stroke or by stopping the bleeding of a hemorrhagic stroke. Post-stroke rehabilitation helps individuals overcome disabilities that result from stroke damage. Medication or drug therapy is the most common treatment for stroke. The most popular classes of drugs used to prevent or treat stroke are anti-thrombotics (*e.g.*, anti-platelet agents and anticoagulants) and thrombolytics. Compound (I) or a pharmaceutically acceptable salt thereof, is administered to a patient who is at risk of suffering a stroke, is suffering from a stroke or has suffered a stroke at a time before, during, after, or any combination thereof, the occurrence of a stroke. Compound (I) or a pharmaceutically acceptable salt thereof, is in a pharmaceutical compositions, or in combination with any of a variety of known treatments, such as, for example, an anti-platelet medication (*e.g*., aspirin, clopidogrel, dipyridamole), an anticoagulant (*e.g.,* warfarin), or a thrombolytic medication (*e.g.,* tissue plasminogen activator (t-PA), reteplase, Urokinase, streptokinase, tenectaplase, lanoteplase, or anistreplase.

Compound (I) or a pharmaceutically acceptable salt thereof, is used in methods to treat, prevent, or protect against atherosclerosis or a symptom thereof in a subject who is at risk for or suffering from atherosclerosis. Another aspect of the invention includes use of compound (I) or a pharmaceutically acceptable salt thereof, in the manufacture of a medicament to to treat, prevent, or protect against atherosclerosis.

Atherosclerosis is a disease affecting the arterial blood vessel and is commonly referred to as a "hardening" of the arteries. It is caused by the formation of multiple plaques within the arteries. Atherosclerotic plaques, though compensated for by artery enlargement, eventually lead to plaque ruptures and stenosis (*i.e.,* narrowing) of the artery, which, in turn, leads to an insufficient blood supply to the organ it feeds. Alternatively, if the compensating artery enlargement process is excessive, a net aneurysm results. These complications are chronic, slowly progressing and cumulative. Most commonly, soft plaque suddenly ruptures, causing the formation of a blood clot (*i.e.,* thrombus) that rapidly slows or stops blood flow, which, in turn, leads to death of the tissues fed by the artery. This catastrophic event is called an infarction. For example, coronary thrombosis of a coronary artery causes a myocardial infarction, commonly known as a heart attack. A myocardial infarction occurs when an atherosclerotic plaque slowly builds up in the inner lining of a coronary artery and then suddenly ruptures, totally occluding the artery and preventing blood flow downstream.

Atherosclerosis and acute myocardial infarction are diagnosed in a patient using any of a variety of clinical and/or laboratory tests such as, physical examination, radiologic or ultrasound examination and blood analysis. For

example, a doctor or clinical can listen to a subject's arteries to detect an abnormal whooshing sound, called a bruit. A bruit can be heard with a stethoscope when placed over the affected artery. Alternatively, or in addition, the clinician or physician can check pulses, *e.g.,* in the leg or foot, for abnormalities such as weakness or absence. The physician or clinical may perform blood work to check for cholesterol levels or to check the levels of cardiac enzymes, such as creatine kinase, troponin and lactate dehydrogenase, to detect abnormalities. For example, troponin sub-units I or T, which are very specific for the myocardium, rise before permanent injury develops. A positive troponin in the setting of chest pain may accurately predict a high likelihood of a myocardial infarction in the near future. Other tests to diagnose atherosclerosis and/or myocardial infarction include, for example, EKG (electrocardiogram) to measure the rate and regularity of a subject's heartbeat; chest X-ray, measuring ankle/brachial index, which compares the blood pressure in the ankle with the blood pressure in the arm; ultrasound analysis of arteries; CT scan of areas of interest; angiography; an exercise stress test, nuclear heart scanning; and magnetic resonance imaging (MRI) and positron emission tomography (PET) scanning of the heart.

Cellular signal transduction by Src is believed to play a key role in increased permeability of vessels, known as vascular permeability (VP). Vascular endothelia growth factor (VEGF), which is produced in response to the ischemic injury, including, *e.g.,* myocardial infarction, has been shown to promote vascular permeability. Studies have shown that the inhibition of Src kinase decreases VEGF-mediated VP. (*See* Parang and Sun, Expert Opin. Ther. Patents, vol. 15(9): 1183-1206 (2005), which is hereby incorporated by reference in its entirety). Mice treated with an Src inhibitor demonstrated reduced tissue damage associated with trauma or injury to blood vessels after myocardial infarction, as compared to untreated mice. (*See e.g.,* U.S. Patent Publication Nos. 20040214836 and 20030130209 by Cheresh *et al.,* the contents of which are hereby incorporated by reference in their entirety). Thus, Src inhibition may be useful in the prevention of, treatment of or protection against secondary damage following injury due to atherosclerosis, such as, for example, myocardial infarction.

Compound (I) or a pharmaceutically acceptable salt thereof, is used to prevent, treat or protect against atherosclerosis or a symptom associated with atherosclerosis. Another aspect of the invention includes use of compound (I) or a pharmaceutically acceptable salt thereof, in the manufacture of a medicament to prevent, treat, or protect against atherosclerosis or a symptom associated with atherosclerosis. Atherosclerosis generally does not produce symptoms until it severely narrows the artery and restricts blood flow, or until it causes a sudden obstruction. Symptoms depend on where the plaques and narrowing develop, *e.g.,* in the heart, brain, other vital organs and legs or almost anywhere in the body. The initial symptoms of atherosclerosis may be pain or cramps when the body requires more oxygen, for example during exercise, when a person may feel chest pain (angina) because of lack of oxygen to the heart or leg cramps because of lack of oxygen to the legs. Narrowing of the arteries supplying blood to the brain may cause dizziness or transient ischemic attacks (TIA's) where the symptoms and signs of a stroke last less than 24 hours. Typically, these symptoms develop gradually.

Symptoms of myocardial infarction are characterized by varying degrees of chest pain, discomfort, sweating, weakness, nausea, vomiting, and arrhythmias, sometimes causing loss of consciousness. Chest pain is the most common symptom of acute myocardial infarction and is often described as a tightness, pressure, or squeezing sensation. Pain may radiate to the jaw, neck, arms, back, and epigastrium, most often to the left arm or neck. Chest pain is more likely caused by myocardial infarction when it lasts for more than 30 minutes. Patients suffering from a myocardial infarction may exhibit shortness of breath (dyspnea) especially if the decrease in myocardial contractility due to the infarct is sufficient to cause left ventricular failure with pulmonary congestion or even pulmonary edema.

Compound (I) or a pharmaceutically acceptable salt thereof, is administered in a pharmaceutical composition, or in combination with any of a variety of known treatments for atherosclerosis, such as, for example, cholesterol-lowering drugs (*e.g*., statins), anti-platelet medications, or anti-coagulants.

Compound (I) or a pharmaceutically acceptable salt thereof, is used in methods to treat, prevent, or protect against neuropathic pain, such as chronic neuropathic pain, or a symptom thereof in a subject who is at risk of suffering from, is suffering from, or has suffered neuropathic pain. Another aspect of the invention includes use of compound (I) or a pharmaceutically acceptable salt thereof, in the manufacture of a medicament to treat, prevent or protect against neuropathic pain.

Neuropathic pain, also known as neuralgia, is qualitatively different from ordinary nociceptive pain. Neuropathic pain usually presents as a steady burning and/or "pins and needles" and/or "electric shock" sensations. The difference between nociceptive pain and neuropathic pain is due to the fact that "ordinary", nociceptive pain stimulates only pain nerves, while a neuropathy often results in the stimulation of both pain and non-pain sensory nerves (*e.g*., nerves that respond to touch, warmth, cool) in the same area, thereby producing signals that the spinal cord and brain do not normally expect to receive.

Neuropathic pain is a complex, chronic pain state that usually is accompanied by tissue injury. With neuropathic pain, the nerve fibers themselves may be damaged, dysfunctional or injured. These damaged nerve fibers send incorrect signals to other pain centers. The impact of nerve fiber injury includes a change in nerve function both at the site of injury and areas around the injury.

Neuropathic pain is diagnosed in a subject or patient using one or more of a variety of laboratory and/or clinical techniques known in the art, such as, for example, physical examination.

c-Src has been shown to regulate the activity of N-methyl-D-aspartate (NMDA) receptors. (*See* Yu et al., Proc. Natl. Acad. Sci. USA, vol. 96:7697-7704 (1999), which is hereby incorporated by reference in its entirety). Studies have shown that PP2, a low molecular weight Src kinase inhibitor, decreases phosphorylation of the NMDA receptor NM2 subunit. (*See* Guo et al., J. Neuro., vol. 22:6208-6217 (2002), which is hereby incorporated by reference in its entirety). Thus, Src inhibition, which in turn, inhibits the activity NMDA receptors, may be useful in the prevention, treatment or amelioration of neuropathic pain, such as chronic neuropathic pain.

Compound (I) or a pharmaceutically acceptable salt thereof is used to prevent, treat or protect against neuropathic pain, such as chronic neuropathic pain, or a symptom associated with neuropathic pain. Symptoms of neuropathic pain include shooting and burning pain, tingling and numbness.

Compound (I) or a pharmaceutically acceptable salt thereof is administered alone, in pharmaceutical compositions, or in combination with any of a variety of known treatments, such as, for example, analgesics, opioids, tricyclic antidepressants, anticonvulsants or serotonin norepinephrine reuptake inhibitors.

Compound (I) or a pharmaceutically acceptable salt thereof, is used in a method to treat, prevent, or protect against hepatitis B or a symptom thereof in a subject who is at risk for or suffering from hepatitis B. Another aspect of the invention includes use of compound (I) or a pharmaceutically acceptable salt thereof, in the manufacture of a medicament to treat, prevent, or protect against hepatitis B.

The hepatitis B virus, a member of the Hepadnavirus family, consists of a proteinaceous core particle containing the viral genome in the form of double stranded DNA with single-stranded regions and an outer lipid-based envelope with embedded proteins. The envelope proteins are involved in viral binding and release into susceptible cells. The inner capsid relocates the DNA genome to the cell's nucleus where viral mRNAs are transcribed. Three subgenomic transcripts encoding the envelope proteins are made, along with a transcript encoding the X protein. A fourth pre-genomic RNA is transcribed, which is exported to the cytosol and translates the viral polymerase and core proteins. Polymerase and pre-genomic RNA are encapsidated in assembling core particles, where reverse transcription of the pre-genomic RNA to genomic DNA occurs by the polymerase protein. The mature core particle then exits the cell via normal secretory pathways, acquiring an envelope along the way.

Hepatitis B is one of a few known non-retroviral viruses that employ reverse transcription as part of the replication process. Other viruses which use reverse transcription include, *e.g.,* HTLV or HIV.

During HBV infection, the host immune response is responsible for both hepatocellular damage and viral clearance. While the innate immune response does not play a significant role in these processes, the adaptive immune response, particularly virus-specific cytotoxic T lymphocytes (CTLs), contributes to nearly all of the liver injury associated with HBV infection. By killing infected cells and by producing antiviral cytokines capable of purging HBV from viable hepatocytes, CTLs also eliminate the virus. Although liver damage is initiated and mediated by the CTLs, antigen-nonspecific inflammatory cells can worsen CTL-induced immunopathology and platelets may facilitate the accumulation of CTLs into the liver.

Hepatitis B is diagnosed in a patient using any of a variety of clinical and/or laboratory tests such as, physical examination, and blood or serum analysis. For example, blood or serum is assayed for the presence of viral antigens and/or antibodies produced by the host. In a common test for Hepatitis B, detection of hepatitis B surface antigen (HBsAg) is used to screen for the presence of infection. It is the first detectable viral antigen to appear during infection with this virus; however, early in an infection, this antigen may not be present and it may be undetectable later in the infection as it is being cleared by the host. During this 'window' in which the host remains infected but is successfully clearing the virus, IgM antibodies to the hepatitis B core antigen (anti-HBc IGM) may be the only serologic evidence of disease.

Shortly after the appearance of the HBsAg, another antigen named as the hepatitis B e antigen (HBeAg) will appear. Traditionally, the presence of HBeAg in a host's serum is associated with much higher rates of viral replication; however, some variants of the hepatitis B virus do not produce the "e" antigen at all. During the natural course of an infection, the HBeAg may be cleared, and antibodies to the "e" antigen (anti-HBe) will arise immediately afterward. This conversion is usually associated with a dramatic decline in viral replication. If the host is able to clear the infection, eventually the HBsAg will become undetectable and will be followed by antibodies to the hepatitis B surface antigen (anti-HBs). A person negative for HBsAg but positive for anti-HBs has either cleared an infection or has been vaccinated previously. A number of people who are positive for HBsAg may have very little viral multiplication, and hence may be at little risk of long-term complications or of transmitting infection to others.

Src plays a role in the replication of the hepatitis B virus. The virally encoded transcription factor HBx activates Src in a step that is required from propagation of the HBV virus. (*See, e.g.,* Klein et al., EMBO J., vol. 18:5019-5027 (1999); Klein et al., Mol. Cell. Biol., vol. 17:6427-6436 (1997), each of which is hereby incorporated by reference in its entirety). Thus, Src inhibition, which in turn, inhibits Src-mediated propagation of the HBV virus, may be useful in the prevention, treatment or protecting against hepatitis B or a symptom thereof.

Compound (I) or a pharmaceutically acceptable salt thereof, prevents, treats or protects against hepatitis B or

a symptom associated with hepatitis B. Symptoms of hepatitis B typically develop within 30-180 days of exposure to the virus. However, up to half of all people infected with the hepatitis B virus have no symptoms. The symptoms of hepatitis B are often compared to flu, and include, *e.g.,* appetite loss; fatigue; nausea and vomiting, itching all over the body; pain over the liver (*e.g*., on the right side of the abdomen, under the lower rib cage), jaundice, and changes in excretory functions.

Compound (I) or a pharmaceutically acceptable salt thereof is administered in pharmaceutical compositions, or in combination with any of a variety of known treatments for hepatitis B, such as, for example, interferon alpha, lamivudine (Epivir-HBV) or baraclude (entecavir).

As described herein, compound (I) or a pharmaceutically acceptable salt thereof, may be used to regulate immune system activity in a subject, thereby protecting against or preventing autoimmune disease, e.g., rheumatoid arthritis, multiple sclerosis, sepsis and lupus as well as transplant rejection and allergic diseases. Another aspect of the invention includes use of compound (I) or a pharmaceutically acceptable salt thereof, in the manufacture of a medicament to regulate the immune system. Alternatively, compound (I) or a pharmaceutically acceptable salt thereof, may be used to treat autoimmune disease in a subject. For example, compound (I) or a pharmaceutically acceptable salt thereof, may result in reduction in the severity of symptoms or halt impending progression of the autoimmune disease in a subject.

Autoimmune diseases are diseases caused by a breakdown of self-tolerance such that the adaptive immune system responds to self antigens and mediates cell and tissue damage. Autoimmune diseases can be organ specific (e.g., thyroiditis or diabetes) or systemic (e.g., systemic lupus erythematosus). T cells modulate the cell-mediated immune response in the adaptive immune system. Under normal conditions, T cells express antigen receptors (T cell receptors) that recognize peptide fragments of foreign proteins bound to self major histocompatibility complex molecules. Among the earliest recognizable events after T cell receptor (TCR) stimulation are the activation of Lck and Fyn, resulting in TCR phosphorylation on tyrosine residues within immunoreceptor tyrosine-based activation motifs (Zamoyska, et al.; 2003, Immunol. Rev., 191, 107-118). Tyrosine kinases, such as Lck (which is a member of the Src family of protein tyrosine kinases) play an essential role in the regulation of cell signaling and cell proliferation by phosphorylating tyrosine residues of peptides and proteins (Levitzki; 2001, Top. Curr. Chem., 211, 1-15; Longati, et al.; 2001, Curr. Drug Targets, 2, 41-55; Qian, and Weiss; 1997, Curr. Opin. Cell Biol., 9, 205-211). Thus, although not wishing to be bound by theory, it is hypothesized that the administration of compound (I) or a pharmaceutically acceptable salt thereof, which modulates tyrosine kinase (e.g., Src) activity is useful in the treatment of autoimmune disease.

The tyrosine kinases lck and fyn are both activated in the TCR pathway; thus, inhibitors of lck and/or fyn have potential utility as autoimmune agents (Palacios and Weiss; 2004, Oncogene, 23, 7990-8000). Lck and Fyn are predominantly expressed by T cells through most of their lifespan. The roles of Lck and Fyn in T cell development, homeostasis and activation have been demonstrated by animal and cell line studies (Parang and Sun; 2005, Expert Opin. The. Patents, 15, 1183-1207). Lck activation is involved in autoimmune diseases and transplant rejection (Kamens, et al.; 2001, Curr. Opin. Investig. Drugs, 2, 1213-1219). Results have shown that the lck (-) Jurkat cell lines are unable to proliferate, produce cytokines, and generate increases in intracellular calcium, inositol phosphate, and tyrosine phosphorylation in response to T cell receptor stimulation (Straus and Weiss; 1992, Cell., 70, 585-593; Yamasaki, et al.; 1996, Mol. Cell. Biol., 16, 7151-7160). Therefore, an agent inhibiting lck would effectively block T cell function, act as an immunosuppressive agent, and have potential utility in autoimmune diseases, such as rheumatoid arthritis, multiple sclerosis, and lupus, as well as in the area of transplant rejection and allergic diseases (Hanke and Pollok; 1995, Inflammation Res., 44, 357-371). Thus, although not wishing to be bound by theory, it is hypothesized that the administration of compound (I) or a pharmaceutically acceptable salt thereof which modulates one or more members of the Src family of protein tyrosine kinases (e.g., lck and/or fyn) is useful in the treatment of autoimmune disease.

Pharmacokinetic characterization of compound (I) in mice, rats and dogs showed that compound (I) is orally bioavailable and has dose-related increases in drug exposure and maximum plasma concentration.

Both compound (I)·2HCl (dihydrochloride) and compound (I)·MSA (mesylate) have been developed. Two bridging pharmacokinetic studies have demonstrated that these two salt forms of compound (I) share similar pharmacokinetic profiles in both rats and dogs. Thus, the findings of compound (I)·2HCl are applicable to the development of compound (I)·MSA.

Compound (I) is a specific and selective Src kinase inhibitor that is very potent against cancer cells, and may spare patients of serious side effects, such as cardiotoxicity, when compared to approved kinase inhibitors and those under development.

Compound (I) is in pure, isolated form (i.e. synthetically produced).

Compound (I) or its pharmaceutically acceptable salt thereof can be prepared conventionally, e.g., by the techniques described in US 2008/0221102 and PCT/US2008/006419.

Pharmaceutical compositions containing compound (I) or a pharmaceutically acceptable salt thereof, can be formulated in a conventional manner using one or more pharmaceutically acceptable carriers. Compound (I) or a pharmaceutically acceptable salt thereof, is administered orally, nasally, transdermally, pulmonary, inhalationally, buccally, sublingually, intraperintoneally, subcutaneously, intramuscularly, intravenously, rectally, intrapleurally, intrathecally or

parenterally. In one embodiment, compound (I) or a pharmaceutically acceptable salt thereof, is administered orally. One skilled in the art will recognize the advantages of certain routes of administration.

The dosage regimen utilizing compound (I) is selected in accordance with a variety of factors including type, species, age, weight, sex and medical condition of the patient; the severity of the condition to be treated; the route of administration; the renal and hepatic function of the patient; and the particular compound or salt thereof employed.

In one embodiment, the invention includes a pharmaceutical composition for oral, intravenous, intramuscular, or subcutaneous administration comprising an amount of compound (I) or a pharmaceutically acceptable salt thereof, ranging from 2 mg to 400 mg per dose administered two or three times daily and a pharmaceutically acceptable carrier. In another embodiment, the amount is from 10 mg to 300 mg. In another embodiment, the amount is from 20 mg to 250 mg. In another embodiment, the amount is from 40 mg to 200 mg. In another embodiment, the amount is from 60 mg to 160 mg. In one embodiment, the dose is administered two times daily. In one embodiment, the dose is administered three times daily.

In one embodiment, the invention includes a pharmaceutical composition for oral, intravenous, intramuscular, or subcutaneous administration comprising an amount of compound (I) or a pharmaceutically acceptable salt thereof ranging from 4 mg to 800 mg per dose administered once daily and a pharmaceutically acceptable carrier. In another embodiment, the amount is from 20 mg to 600 mg. In another embodiment, the amount is from 40 mg to 500 mg. In another embodiment, the amount is from 80 mg to 400 mg. In another embodiment, the amount is from 120 mg to 320 mg.

In one embodiment, the invention includes a pharmaceutical composition administered as described above, wherein the composition comprises the mesylate salt of compound (I). In one embodiment, the administration is oral. In another embodiment, the administration is intravenous. In another embodiment, the administration is intramuscular. In another embodiment, the administration is subcutaneous.

Techniques for formulation and administration of compound (I) or a pharmaceutically acceptable salt thereof, can be found in Remington: the Science and Practice of Pharmacy, 19th edition, Mack Publishing Co., Easton, PA (1995). In an embodiment, compound (I) or a pharmaceutically acceptable salt thereof, is used in pharmaceutical preparations in combination with a pharmaceutically acceptable carrier or diluent. Suitable pharmaceutically acceptable carriers include inert solid fillers or diluents and sterile aqueous or organic solutions. Compound (I) or a pharmaceutically acceptable salt thereof, is present in such pharmaceutical compositions in amounts sufficient to provide the desired dosage amount in the range described herein.

In one embodiment, compound (I) or a pharmaceutically acceptable salt thereof, is prepared for oral administration, wherein compound (I) or a pharmaceutically acceptable salt thereof is combined with a suitable solid or liquid carrier or diluent to form capsules, tablets, pills, powders, syrups, solutions, suspensions and the like.

The tablets, pills, capsules, and the like contain from about 1 to about 99 weight percent of the active ingredient and a binder such as gum tragacanth, acacias, corn starch or gelatin; excipients such as dicalcium phosphate; a disintegrating agent such as corn starch, potato starch or alginic acid; a lubricant such as magnesium stearate; and/or a sweetening agent such as sucrose, lactose, saccharin, xylitol, and the like. When a dosage unit form is a capsule, it often contains, in addition to materials of the above type, a liquid carrier such as a fatty oil.

In some embodiments, various other materials are present as coatings or to modify the physical form of the dosage unit. For instance, in some embodiments, tablets are coated with shellac, sugar or both. In some embodiments, a syrup or elixir contains, in addition to the active ingredient, sucrose as a sweetening agent, methyl and propylparabens as preservatives, a dye and a flavoring such as cherry or orange flavor, and the like.

For some embodiments relating to parental administration, compound (I) or a pharmaceutically acceptable salt thereof, is combined with sterile aqueous or organic media to form injectable solutions or suspensions. In one embodiment, injectable compositions are aqueous isotonic solutions or suspensions. The compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. The compositions are prepared according to conventional mixing, granulating or coating methods, respectively, and contain about 0.1 to 75%, in another embodiment, the compositions contain about 1 to 50%, of the active ingredient.

For example, injectable solutions are produced using solvents such as sesame or peanut oil or aqueous propylene glycol, as well as aqueous solutions of water-soluble pharmaceutically acceptable salts of compound (I). In some embodiments, dispersions are prepared in glycerol, liquid polyethylene glycols and mixtures thereof in oils. Under ordinary conditions of storage and use, these preparations contain a preservative to prevent the growth of microorganisms. The terms "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

For rectal administration, suitable pharmaceutical compositions are, for example, topical preparations, suppositories or enemas. Suppositories are advantageously prepared from fatty emulsions or suspensions. The compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. The compositions are prepared according to conventional mixing, granulating or coating methods, respectively, and contain about 0.1 to 75%, in another embodiment, compositions contain about 1 to 50%, of the active ingredient.

In some embodiments, compound (I) or a pharmaceutically acceptable salt thereof, is formulated to deliver the active agent by pulmonary administration, e.g., administration of an aerosol formulation containing the active agent from, for example, a manual pump spray, nebulizer or pressurized metered-dose inhaler. In some embodiments, suitable formulations of this type also include other agents, such as antistatic agents, to maintain the disclosed compounds as effective aerosols.

A drug delivery device for delivering aerosols comprises a suitable aerosol canister with a metering valve containing a pharmaceutical aerosol formulation as described and an actuator housing adapted to hold the canister and allow for drug delivery. The canister in the drug delivery device has a headspace representing greater than about 15% of the total volume of the canister. Often, the polymer intended for pulmonary administration is dissolved, suspended or emulsified in a mixture of a solvent, surfactant and propellant. The mixture is maintained under pressure in a canister that has been sealed with a metering valve.

For nasal administration, either a solid or a liquid carrier can be used. The solid carrier includes a coarse powder having particle size in the range of, for example, from about 20 to about 500 microns and such formulation is administered by rapid inhalation through the nasal passages. In some embodiments where the liquid carrier is used, the formulation is administered as a nasal spray or drops and includes oil or aqueous solutions of the active ingredients.

Also contemplated are formulations that are rapidly dispersing dosage forms, also known as "flash dose" forms. In particular, some embodiments of the present invention are formulated as compositions that release their active ingredients within a short period of time, *e.g.,* typically less than about five minutes, in another embodiment, less than about ninety seconds, in another embodiment, less than about thirty seconds and in another embodiment, in less than about ten or fifteen seconds. Such formulations are suitable for administration to a subject via a variety of routes, for example by insertion into a body cavity or application to a moist body surface or open wound.

Typically, a "flash dosage" is a solid dosage form that is administered orally, which rapidly disperses in the mouth, and hence does not require great effort in swallowing and allows the compound to be rapidly ingested or absorbed through the oral mucosal membranes. In some embodiments, suitable rapidly dispersing dosage forms are also used in other applications, including the treatment of wounds and other bodily insults and diseased states in which release of the medicament by externally supplied moisture is not possible.

"Flash dose" forms are known in the art; see for example, effervescent dosage forms and quick release coatings of insoluble microparticles in U.S. Pat. Nos. 5,578,322 and 5,607,697; freeze dried foams and liquids in U.S. Pat. Nos. 4,642,903 and 5,631,023; melt spinning of dosage forms in U.S. Pat. Nos. 4,855,326, 5,380,473 and 5,518,730; solid, free-form fabrication in U.S. Pat. No. 6,471,992; saccharide-based carrier matrix and a liquid binder in U.S. Pat. Nos. 5,587,172, 5,616,344, 6,277,406, and 5,622,719; and other forms known to the art.

Compound (I) or a pharmaceutically acceptable salt thereof, is also formulated as "pulsed release" formulations, in which the compound or salt is released from the pharmaceutical compositions in a series of releases (*i.e.,* pulses). Compound (I) or a pharmaceutically acceptable salt thereof, is also formulated as "sustained release" formulations in which the compound or salt is continuously released from the pharmaceutical composition over a prolonged period.

Also contemplated are formulations, *e.g.,* liquid formulations, including cyclic or acyclic encapsulating or solvating agents, *e.g.,* cyclodextrins, polyethers, or polysaccharides (*e.g*., methylcellulose), or in another embodiment, polyanionic β-cyclodextrin derivatives with a sodium sulfonate salt group separate from the lipophilic cavity by an alkyl ether spacer group or polysaccharides. In one embodiment, the agent is methylcellulose. In another embodiment, the agent is a polyanionic β-cyclodextrin derivative with a sodium sulfonate salt separated from the lipophilic cavity by a butyl ether spacer group, *e.g*., CAPTISOL® (CyDex, Overland, KS). One skilled in the art can evaluate suitable agent/disclosed compound formulation ratios by preparing a solution of the agent in water, *e.g.,* a 40% by weight solution; preparing serial dilutions, *e.g.* to make solutions of 20%, 10, 5%, 2.5%, 0% (control), and the like; adding an excess (compared to the amount that can be solubilized by the agent) of the disclosed compound; mixing under appropriate conditions, *e.g.,* heating, agitation, sonication, and the like; centrifuging or filtering the resulting mixtures to obtain clear solutions; and analyzing the solutions for concentration of the disclosed compound.

All publications and patent documents cited herein are incorporated herein by reference as if each such publication or document was specifically and individually indicated to be incorporated herein by reference. Citation of publications and patent documents is not intended as an admission that any is pertinent prior art, nor does it constitute any admission as to the contents or date of the same. The invention having now been described by way of written description, those of skill in the art will recognize that the invention can be practiced in a variety of embodiments and that the foregoing description and examples below are for purposes of illustration and not limitation of the claims that follow.

## Definitions

For convenience, certain terms used in the specification, examples and appended claims are collected here.

Protein kinases are a large class of enzymes which catalyze the transfer of the γ-phosphate from ATP to the hydroxyl group on the side chain of Ser/Thr or Tyr in proteins and peptides and are intimately involved in the control of various important cell functions, perhaps most notably: signal transduction, differentiation, and proliferation. There are estimated to be about 2,000 distinct protein kinases in the human body, and although each of these phosphorylate particular protein/peptide substrates, they all bind the same second substrate ATP in a highly conserved pocket. About 50% of the known oncogene products are protein tyrosine kinases (PTKs), and their kinase activity has been shown to lead to cell transformation.

The PTKs can be classified into two categories, the membrane receptor PTKs (e.g. growth factor receptor PTKs) and the non-receptor PTKs (*e.g.* the Src family of proto-oncogene products and focal adhesion kinase (FAK)). The hyperactivation of Src has been reported in a number of human cancers, including those of the colon, breast, lung, bladder, and skin, as well as in gastric cancer, hairy cell leukemia, and neuroblastoma.

The phrase "inhibits one or more components of a protein kinase signaling cascade" means that one or more components of the kinase signaling cascade are effected such that the functioning of the cell changes. Components of a protein kinase signaling cascade include any proteins involved directly or indirectly in the kinase signaling pathway including second messengers and upstream and downstream targets.

"Treating", includes any effect, *e.g.,* lessening, reducing, modulating, or eliminating, that results in the improvement of the condition, disease, disorder, etc. "Treating" or "treatment" of a disease state includes: (a) inhibiting an existing disease-state i.e., arresting its development or clinical symptoms; and/or (b) relieving the disease-state i.e., causing regression of the disease.

"Preventing" means causing the clinical symptoms of the disease state not to develop i.e., inhibiting the onset of disease, in a subject that may be exposed to or predisposed to the disease state, but does not yet experience or display symptoms of the disease state.

"Protecting against" means reducing the severity of the clinical symptoms of the disease state (lessening) in a subject that may be exposed to or predisposed to the disease state by administereing the compound to a subject prior to the subject experiencing or displaying symptoms of the disease state.

"Disease state" means any disease, disorder, condition, symptom, or indication.

As used herein, the term "cell proliferative disorder" refers to conditions in which the unregulated and/or abnormal growth of cells can lead to the development of an unwanted condition or disease, which can be cancerous or non-cancerous, for example a psoriatic condition. As used herein, the terms "psoriatic condition" or "psoriasis" refers to disorders involving keratinocyte hyperproliferation, inflammatory cell infiltration, and cytokine alteration.

In one embodiment, the cell proliferation disorder is cancer. As used herein, the term "cancer" includes solid tumors, such as lung, breast, colon, ovarian, brain, liver, pancreas, prostate, malignant melanoma, non-melanoma skin cancers, as well as hematologic tumors and/or malignancies, such as childhood leukemia and lymphomas, multiple myeloma, Hodgkin's disease, lymphomas of lymphocytic and cutaneous origin, acute and chronic leukemia such as acute lymphoblastic, acute myelocytic or chronic myelocytic leukemia, plasma cell neoplasm, lymphoid neoplasm and cancers associated with AIDS.

In addition to psoriatic conditions, the types of proliferative diseases which may be treated using the compositions of the present invention are epidermic and dermoid cysts, lipomas, adenomas, capillary and cutaneous hemangiomas, lymphangiomas, nevi lesions, teratomas, nephromas, myofibromatosis, osteoplastic tumors, and other dysplastic masses and the like. The proliferative diseases can include dysplasias and disorders of the like.

An "effective amount" of compound (I) or a pharmaceutically acceptable salt thereof, is the quantity which, when administered to a subject having a disease or disorder, results in regression of the disease or disorder in the subject. For example, an effective amount of compound (I) or a pharmaceutically acceptable salt thereof, is the quantity which, when administered to a subject having a cell proliferation disorder, results in regression of cell growth in the subject. The amount of the compound or pharmaceutically acceptable salt thereof, to be administered to a subject will depend on the particular disorder, the mode of administration, co-administered compounds, if any, and the characteristics of the subject, such as general health, other diseases, age, sex, genotype, body weight and tolerance to drugs.

As used herein, the term "effective amount" refers to an amount of compound (I) or a pharmaceutically acceptable salt thereof, or a combination of compounds, effective when administered alone or in combination as an anti-proliferative agent. For example, an effective amount refers to an amount of compound (I) present in a formulation or on a medical device given to a recipient patient or subject sufficient to elicit biological activity, for example, anti-proliferative activity, such as *e.g.,* anti-cancer activity or antineoplastic activity. The combination of compounds optionally is a synergistic combination. Synergy, as described, for example, by Chou and Talalay, Adv. Enzyme Regul. vol. 22, pp. 27-55 (1984), occurs when the effect of the compounds when administered in combination is greater than the additive effect of the compounds when administered alone as a single agent. In general, a synergistic effect is most clearly demonstrated at

sub-optimal concentrations of the compounds. Synergy can be in terms of lower cytotoxicity, or increased anti-proliferative effect, or some other beneficial effect of the combination compared with the individual components.

An effective amount of one or more of the compounds can be formulated with a pharmaceutically acceptable carrier for administration to a human or an animal. Accordingly, the compounds or the formulations can be administered, for example, via oral, parenteral, or topical routes, to provide an effective amount of the compound. In alternative embodiments, compound (I) or a salt thereof, prepared in accordance with the present invention can be used to coat or impregnate a medical device, *e.g.,* a stent.

The term "prophylactically effective amount" means an effective amount of compound (I) or a salt thereof, that is administered to prevent or reduce the risk of unwanted cellular proliferation.

"Pharmacological effect" as used herein encompasses effects produced in the subject that achieve the intended purpose of a therapy. In one embodiment, a pharmacological effect means that primary indications of the subject being treated are prevented, alleviated, or reduced. For example, a pharmacological effect would be one that results in the prevention, alleviation or reduction of primary indications in a treated subject. In another embodiment, a pharmacological effect means that disorders or symptoms of the primary indications of the subject being treated are prevented, alleviated, or reduced. For example, a pharmacological effect would be one that results in the prevention or reduction of primary indications in a treated subject.

A "pharmaceutical composition" is a formulation containing compound (I) or a salt thereof, in a form suitable for administration to a subject. In one embodiment, the pharmaceutical composition is in bulk or in unit dosage form. The unit dosage form is any of a variety of forms, including, for example, a capsule, an IV bag, a tablet, a single pump on an aerosol inhaler, or a vial. The quantity of active ingredient (*e.g.*, a formulation of the disclosed compound or salt, hydrate, solvate, or isomer thereof) in a unit dose of composition is an effective amount and is varied according to the particular treatment involved. One skilled in the art will appreciate that it is sometimes necessary to make routine variations to the dosage depending on the age and condition of the patient. The dosage will also depend on the route of administration. A variety of routes are contemplated, including oral, pulmonary, rectal, parenteral, transdermal, subcutaneous, intravenous, intramuscular, intraperitoneal, inhalational, buccal, sublingual, intrapleural, intrathecal, intranasal, and the like. Dosage forms for the topical or transdermal administration of a compound of this invention include powders, sprays, ointments, pastes, creams, lotions, gels, solutions, patches and inhalants. In one embodiment, the active compound is mixed under sterile conditions with a pharmaceutically acceptable carrier, and with any preservatives, buffers, or propellants that are required.

The term "flash dose" refers to compound formulations that are rapidly dispersing dosage forms.

The term "immediate release" is defined as a release of compound from a dosage form in a relatively brief period of time, generally up to about 60 minutes. The term "modified release" is defined to include delayed release, extended release, and pulsed release. The term "pulsed release" is defined as a series of releases of drug from a dosage form. The term "sustained release" or "extended release" is defined as continuous release of a compound from a dosage form over a prolonged period.

A "subject" includes mammals, *e.g.,* humans, companion animals (*e.g.,* dogs, cats, birds, and the like), farm animals (*e.g.*, cows, sheep, pigs, horses, fowl, and the like) and laboratory animals (*e.g.*, rats, mice, guinea pigs, birds, and the like). In one embodiment, the subject is human.

As used herein, the phrase "pharmaceutically acceptable" refers to those compounds, materials, compositions, carriers, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" is art-recognized, and includes, for example, pharmaceutically acceptable materials, compositions or vehicles, such as a liquid or solid filler, diluent, excipient, solvent or encapsulating material, involved in carrying or transporting any subject composition from one organ, or portion of the body, to another organ, or portion of the body. Each carrier must be "acceptable" in the sense of being compatible with the other ingredients of a subject composition and not injurious to the patient. In certain embodiments, a pharmaceutically acceptable carrier is non-pyrogenic. Some examples of materials which may serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, sunflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

"Pharmaceutically acceptable excipient" means an excipient that is useful in preparing a pharmaceutical composition that is generally safe, non-toxic and neither biologically nor otherwise undesirable, and includes excipient that

is acceptable for veterinary use as well as human pharmaceutical use. A "pharmaceutically acceptable excipient" as used in the specification and claims includes both one and more than one such excipient.

Compound (I) of the invention is capable of further forming salts. All of these forms are also contemplated within the scope of the claimed invention.

Compound (I) of the invention may contain isotopes of the atoms present. The present invention is intended to include all isotopes of atoms occurring in the present compounds. Isotopes include those atoms having the same atomic number but different mass numbers. By way of general example and without limitation, isotopes of hydrogen include tritium and deuterium, and isotopes of carbon include C-13 and C-14.

"Pharmaceutically acceptable salt" of a compound means a salt that is pharmaceutically acceptable and that possesses the desired pharmacological activity of the parent compound.

As used herein, "pharmaceutically acceptable salts" refer to derivatives of compound (I) wherein compound (I) is modified by making acid or base salts thereof. Examples of pharmaceutically acceptable salts include, but are not limited to, mineral or organic acid salts of basic residues such as amines, alkali or organic salts of acidic residues such as carboxylic acids, and the like. The pharmaceutically acceptable salts include the conventional non-toxic salts or the quaternary ammonium salts of the parent compound formed, for example, from non-toxic inorganic or organic acids. For example, such conventional non-toxic salts include, but are not limited to, those derived from inorganic and organic acids selected from 2-acetoxybenzoic, 2-hydroxyethane sulfonic, acetic, ascorbic, benzene sulfonic, benzoic, bicarbonic, carbonic, citric, edetic, ethane disulfonic, 1,2-ethane sulfonic, fumaric, glucoheptonic, gluconic, glutamic, glycolic, glycollyarsanilic, hexylresorcinic, hydrabamic, hydrobromic, hydrochloric, hydroiodic, hydroxymaleic, hydroxynaphthoic, isethionic, lactic, lactobionic, lauryl sulfonic, maleic, malic, mandelic, methane sulfonic, napsylic, nitric, oxalic, pamoic, pantothenic, phenylacetic, phosphoric, polygalacturonic, propionic, salicyclic, stearic, subacetic, succinic, sulfamic, sulfanilic, sulfuric, tannic, tartaric, toluene sulfonic, and the commonly occurring amine acids, *e.g.*, glycine, alanine, phenylalanine, arginine, etc.

Other examples include hexanoic acid, cyclopentane propionic acid, pyruvic acid, malonic acid, 3-(4-hydroxybenzoyl)benzoic acid, cinnamic acid, 4-chlorobenzenesulfonic acid, 2-naphthalenesulfonic acid, 4-toluenesulfonic acid, camphorsulfonic acid, 4-methylbicyclo-[2.2.2]-oct-2-ene-1-carboxylic acid, 3-phenylpropionic acid, trimethylacetic acid, tertiary butylacetic acid, muconic acid, and the like. The invention also encompasses salts formed when an acidic proton present in the parent compound either is replaced by a metal ion, *e.g.,* an alkali metal ion, an alkaline earth ion, or an aluminum ion; or coordinates with an organic base such as ethanolamine, diethanolamine, triethanolamine, tromethamine, N-methylglucamine, and the like.

It should be understood that all references to pharmaceutically acceptable salts include solvent addition forms (solvates) or crystal forms (polymorphs) as defined herein, of the same salt.

The terms "crystal polymorphs" or "polymorphs" or "crystal forms" means crystal structures in which compound (I) (or salt or solvate thereof) can crystallize in different crystal packing arrangements, all of which have the same elemental composition. Different crystal forms usually have different X-ray diffraction patterns, infrared spectral, melting points, density hardness, crystal shape, optical and electrical properties, stability and solubility. Recrystallization solvent, rate of crystallization, storage temperature, and other factors may cause one crystal form to dominate. Crystal polymorphs of compound (I) can be prepared by crystallization under different conditions.

Additionally, compound (I), for example, the salts of compound (I), can exist in either hydrated or unhydrated (the anhydrous) form or as solvates with other solvent molecules. Nonlimiting examples of hydrates include monohydrates, dihydrates, etc. Nonlimiting examples of solvates include ethanol solvates, acetone solvates, etc.

"Solvates" means solvent addition forms that contain either stoichiometric or non stoichiometric amounts of solvent. Some compounds have a tendency to trap a fixed molar ratio of solvent molecules in the crystalline solid state, thus forming a solvate. If the solvent is water the solvate formed is a hydrate, when the solvent is alcohol, the solvate formed is an alcoholate. Hydrates are formed by the combination of one or more molecules of water with one of the substances in which the water retains its molecular state as $H_2O$, such combination being able to form one or more hydrate.

The pharmaceutically acceptable salts of the present invention can be synthesized from compound (I) by conventional chemical methods. Generally, such salts can be prepared by reacting compound (I) with a stoichiometric amount of the appropriate base or acid in water or in an organic solvent, or in a mixture of the two; non-aqueous media like ether, ethyl acetate, ethanol, isopropanol, or acetonitrile can be used. Lists of suitable salts are found in Remington's Pharmaceutical Sciences, 18th ed. (Mack Publishing Company, 1990).

Compound (I) can also be prepared as a prodrug, for example pharmaceutically acceptable prodrug. The terms "pro-drug" and "prodrug" are used interchangeably herein and refer to any compound which releases an active parent drug *in vivo.* Since prodrugs are known to enhance numerous desirable qualities of pharmaceuticals (*e.g.*, solubility, bioavailability, manufacturing, etc.) compound (I) can be delivered in prodrug form. Thus, the present invention is intended to cover prodrugs of compound (I), methods of delivering the same and compositions containing the same. "Prodrugs" are intended to include any covalently bonded carriers that release an active compound (I) *in vivo* when such prodrug is administered to a subject. Prodrugs are prepared by modifying functional groups present in compound (I) such a way

that the modifications are cleaved, either in routine manipulation or *in vivo,* to compound (I).

"Combination therapy" (or "co-therapy") includes the administration of compound (I) or a salt thereof, and at least a second agent as part of a specific treatment regimen intended to provide the beneficial effect from the co-action of these therapeutic agents. The beneficial effect of the combination includes, but is not limited to, pharmacokinetic or pharmacodynamic co-action resulting from the combination of therapeutic agents. Administration of these therapeutic agents in combination typically is carried out over a defined time period (usually minutes, hours, days or weeks depending upon the combination selected). "Combination therapy" may, but generally is not, intended to encompass the administration of two or more of these therapeutic agents as part of separate monotherapy regimens that incidentally and arbitrarily result in the combinations of the present invention.

"Combination therapy" is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single capsule having a fixed ratio of each therapeutic agent or in multiple, single capsules for each of the therapeutic agents. Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination selected may be administered by intravenous injection while the other therapeutic agents of the combination may be administered orally. Alternatively, for example, all therapeutic agents may be administered orally or all therapeutic agents may be administered by intravenous injection. The sequence in which the therapeutic agents are administered is not narrowly critical.

"Combination therapy" also embraces the administration of the therapeutic agents as described above in further combination with other biologically active ingredients and non-drug therapies (*e.g*., surgery or radiation treatment) . Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks.

Throughout the description, where compositions are described as having, including, or comprising specific components, it is contemplated that compositions also consist essentially of, or consist of, the recited components. Similarly, where processes are described as having, including, or comprising specific process steps, the processes also consist essentially of, or consist of, the recited processing steps. Further, it should be understood that the order of steps or order for performing certain actions are immaterial so long as the invention remains operable. Moreover, two or more steps or actions may be conducted simultaneously.

## EXAMPLES

### Example 1: Small Scale Synthesis of Compound (I)

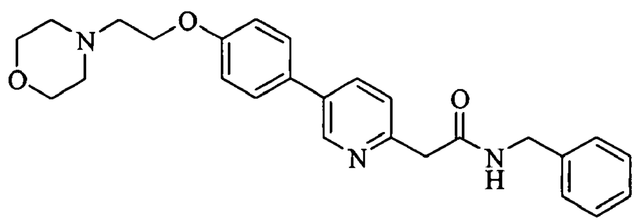

The preliminary synthesis described below was illustrated in US20060160800A1. This procedure is useful for small scale reactions, for example, reactions that produce up to 50 g of product.

For the following synthesis, unless otherwise noted, reagents and solvents were used as received from commercial suppliers. Proton and carbon nuclear magnetic resonance spectra were obtained on a Bruker AC 300 or a Bruker AV 300 spectrometer at 300 MHz for proton and 75 MHz for carbon. Spectra are given in ppm (δ) and coupling constants, *J*, are reported in Hertz. Tetramethylsilane was used as an internal standard for. proton spectra and the solvent peak was used as the reference peak for carbon spectra. Mass spectra and LC-MS mass data were obtained on a Perkin Elmer Sciex 100 atmospheric pressure ionization (APCI) mass spectrometer. LC-MS analyses were obtained using a Luna C8(2) Column (100 × 4.6 mm, Phenomenex) with UV detection at 254 nm using a standard solvent gradient program (Method B). Thin-layer chromatography (TLC) was performed using Analtech silica gel plates and visualized

by ultraviolet (UV) light, iodine, or 20 wt % phosphomolybdic acid in ethanol. HPLC analyses were obtained using a Prevail C18 column (53 x 7 mm, Alltech) with UV detection at 254 nm using a standard solvent gradient program (Method A or B).

Method A:

| Time (min) | Flow (mL/min) | %A | %B |
|---|---|---|---|
| 0.0 | 3.0 | 95.0 | 5.0 |
| 10.0 | 3.0 | 0.0 | 100.0 |
| 11.0 | 3.0 | 0.0 | 100.0 |
| A = Water with 0.1 v/v Trifluoroacetic Acid<br>B = Acetonitrile with 0.1 v/v Trifluoroacetic Acid | | | |

Method B:

| Time (min) | Flow (mL/min) | %A | %B |
|---|---|---|---|
| 0.0 | 2.0 | 95.0 | 5.0 |
| 4.0 | 2.0 | 5.0 | 95.0 |
| A = Water with 0.02 v/v Trifluoroacetic Acid<br>B = Acetonitrile with 0.02 v/v Trifluoroacetic Acid | | | |

Synthesis of N*-benzyl-2-(5-bromopyridin-2-yl)acetamide:*

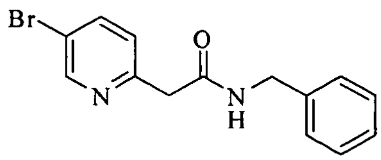

A flask was charged with 5-(5-bromopyridin-2(1H)-ylidene)-2,2-dimethyl-1,3-dioxane-4,6-dione (1.039 g, 3.46 mmol), benzylamine (0.50 mL, 4.58 mmol), and toluene (20 mL). The reaction was brought to reflux under nitrogen for 18 hours, then cooled and placed in a freezer until cold. The product was collected by filtration and washed with hexanes to yield a mass of bright white crystals (1.018 g, 96%).

Synthesis of *4-(2-(4-(4,4,5,5-tetramethyl[1,3,2]dioxaborolan-2-yl)-phenoxy)ethyl)morpholine:*

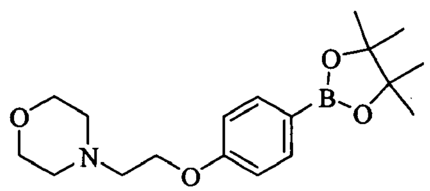

To a stirring solution of 4-(4,4,5,5-tetramethyl[1,3,2]dioxaborolan-2-yl)-phenol (2.55 g, 11.58 mmol), 2-morpholin-4-ylethanol (1.60 mL, 1.73 g, 13.2 mmol) and triphenyl phosphine (3.64 g, 13.9 mmol) in methylene chloride (60 mL) at 0 °C was added dropwise DIAD (2.82 g, 13.9 mmol). The reaction was allowed to warm to room temperature and stir overnight. After 18 hours, additional portions of triphenyl phosphine (1.51 g, 5.8 mmol), 2-morpholin-4-ylethanol (0.70 mL, 5.8 mmol), and DIAD (1.17 g, 5.8 mmol) were added. After stirring an additional 2 hours at room temperature the reaction was concentrated and the residue purified by flash chromatography (5% to 25% EtOAc in $CHCl_3$) to provide the product as a white solid (2.855 g, 74%).

Synthesis of *2-(5-(4-(2-morpholinoethoxy)phenyl)pyridin-2-yl)-N-benzylacetamide* Compound (I)

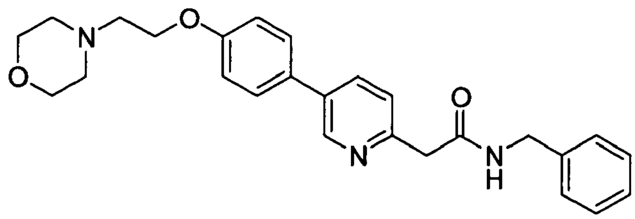

A 10 mL reaction tube with a septum closure and stir bar was charged with *N*-benzyl-2-(5-bromopyridin-2-yl)acetamide (123 mg, 0.403 mmol), 4-(2-(4-(4,4,5,5-tetramethyl[1,3,2]dioxaborolan-2-yl)-phenoxy)ethyl)morpholine (171 mg, 0.513 mmol), and FibreCat 1007[1] (30 mg, 0.015 mmol). Ethanol (3 mL) was added, followed by aqueous potassium carbonate solution (0.60 mL, 1.0 M, 0.60 mmol). The tube was sealed and heated under microwave conditions at 150 °C for 10 minutes. The reaction was cooled and concentrated to remove the majority of the ethanol, and then taken up in 10 mL of ethyl acetate and washed successively with water and saturated sodium chloride solution. The organic layer was dried with $MgSO_4$, filtered and concentrated to a white solid. This white solid was triturated with ethyl ether to give compound (I) as a white solid (137 mg, 79%): mp 135-137 °C.; $^1H$ NMR (300 MHz, $CDCl_3$) $\delta$ 8.70 (d, 1H, *J*=2.0 Hz), 7.81 (dd, 1H, *J*=2.4 Hz, *J*=8.0Hz), 7.65 (br s, 1H), 7.49 (d, 2H, *J*=8.8 Hz), 7.37-7.20 (m, 6H), 7.01 (d, 2H, *J*=8.8 Hz), 4.49 (d, 2H, *J*=5.8 Hz), 4.16 (t, 2H, *J*=5.7 Hz, 3.82 (s, 2H), 3.78-3.72 (m, 4H), 2.84 (t, 2H, *J*=5.7 Hz), 2.62-2.58 (m, 4H); HPLC (Method B) 98.0% (AUC), $t_R$ = 1.834 min.; APCI MS *m/z* 432 $[M+H]^+$.

[1] Polymer bound di(acetato)dicyclohexylphenylphosphinepalladium(II), manufactured by Johnson Matthey, Inc. and available from Aldrich (catalog # 590231).

**Example 2: Intermediate Scale Synthesis of Compound (I) di-hydrochloride**

The synthesis outlined in this example can be used on intermediate-scale reactions. The preparation of batches of at least 50 g of the dihydrochloride salt of compound (I) is shown in Scheme 1. The linear synthesis consisted of 6 steps, a seventh step being the preparation of one of the reagents, 6-fluoropyridin-3-ylboronic acid (which is also available commercially). The overall yield of the sequence was 35% with an average yield of 83%, with the lowest yielding step being that of 68%. Of the seven steps only one required chromatography. The procedure listed below was performed on a 70 g scale.

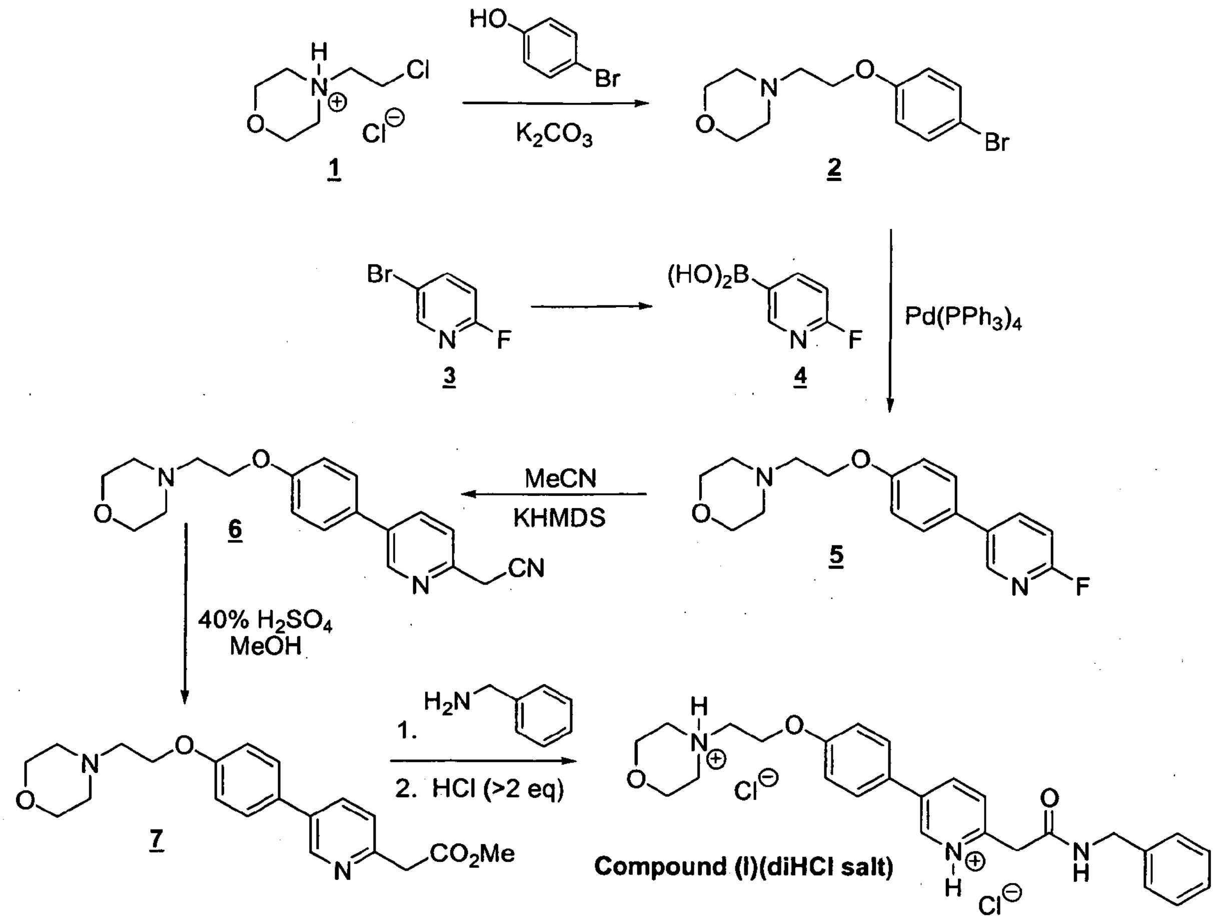


Compound (I)(diHCl salt)

The first step is a Williamson ether synthesis between 4-bromophenol (131 g) and N-chloroethylmorpholine (**1** as the HCl salt; 141 g) using $K_2CO_3$ powder (3 to 3.5 equivalents) as the base and having acetonitrile as the solvent. The ingredients were mixed and stirred at reflux overnight with high conversion (96.3-99.1%). After dilution with dichloromethane and heptane, the reaction mixture was filtered and evaporated to give the desired product **2** in essentially a quantitative yield (216 g). Note that with similar substrates (e.g., 4-bromo-3-fluorophenol), conversions (even with extensive heating) were not always so high (e.g., 59.9-98.3%). Both the alkyl chloride and the $K_2CO_3$ are preferably purchased from Aldrich. If continued heating does not drive reaction to completion, unreacted bromophenol can readily be removed by dissolving the crude reaction mixture in 4 parts toluene and washing out the phenol with 4 parts 15% aqueous NaOH.

One of the reagents required for the second step (Suzuki coupling) was 6-fluoropyridin-3-ylboronic acid **(4).** Although available commercially, this reagent was readily prepared by lithium-bromide exchange of 5-bromo-2-fluoropyridine (**3**, 102 g) with n-butyllithium (1.2 eq) at low temperatures (<-60 °C) in TBME followed by the addition of triisopropylborate (1.65 eq). Both stages of the reaction are brief, with an overall reaction time (including addition times) of ~3 h. Quenching is achieved with aqueous 24% NaOH, which also extracts the product leaving impurities in the organic layer. Once the aqueous layer is removed, it is then neutralized with HCl and extracted with EtOAc. After drying the organics and diluting with some heptane, concentration leads to precipitation/ crystallization of the product. Filtration gave the boronic acid **4** in relatively high purity (96.4% AUC) and good yield (69 g, 79-90%; see note on estimation of yield in the experimental section), which can be used without further purification.

The second reaction step in the linear sequence (a Suzuki coupling) is a simple reaction to set up; all the reagents [**2** (111 g), aqueous $Na_2CO_3$, DME, and $Pd(PPh_3)_4$ (0.04 eq)] were charged to the reaction flask and the mixture heated at reflux; note that the reaction mixture was degassed to remove oxygen. Once the reaction is complete (within 7 h), the work-up involved decanting (or siphoning off) of reaction solution from the organic salts on the side of the flask (there was no visible aqueous layer), the flask was rinsed, and dried, and the solvent was removed from the combined organics. Crystallization of crude **5** from isopropanol/heptane provided material of improved purity compared to the crude, but still required chromatography (ratio of silica gel to crude was ~8.5:1) to obtain material of adequate purity (>98%); the yield was 68% (79.5 g). Use of clean **5** prevented the need for chromatography in the next step, acetonitrile

displacement of the fluorine atom.

The replacement of fluoride with acetonitrile was also a simple reaction, and a simple room temperature crystallization of the crude product provided clean **6** in high yield and purity. The reaction involved initial formation of the "enolate" from acetonitrile (6.5 eq) using potassium hexamethyldisilane KHMDS (8 eq)/THF at -10 °C followed immediately by the addition of fluoride **5** (79 g). The reaction was quick and after one hour quenching was achieved with saturated brine. After drying and evaporation of solvent of the organics, the resulting crude mixture consisted of only two components, the desired product and a much less polar product from apparent self-condensation of acetonitrile. The crude mixture was swirled in isopropanol/heptane and allowed to sit overnight, which resulted in complete crystallization of the product, which was filtered off and washed to provide high purity **6** (99.3% AUC) in good yield (64 g, 76%).

Methanolysis of **6** (64 g) was accomplished by heating in 40% $H_2SO_4$ (in MeOH) until the reaction was complete (25 h). The reaction was then cooled, stirred with $MgSO_4$ to convert traces of hydrolyzed product ($ArCH_2$-$CO_2Me$) back to product, and then added to cooled, aqueous $K_2CO_3$, with simultaneous extraction into dichloromethane. Drying and evaporation of most of the DCM followed by addition of 5% EtOAc (in heptane) and further concentration resulted in the crystallization of the product. Filtration of the solid and washing gave high purity (98.9% AUC) **7** in good yield (82%), additional high purity product (4 g) being obtained from the mother liquors for a total yield of 61.7 g (87%).

The amidation step also involved charging of the reaction vessel with the ingredients (**7** (61 g), benzyl amine (3 eq), and high boiling anisole) and then heating at reflux until the reaction was complete. Cooling of the reaction mixture resulted in complete crystallization of the target compound with high purity (98.9%) and good yield (81%).

The final step was the formation of the dihydrochloric salt of the target compound. In order to ensure complete protonation at both basic sites, the reaction was conducted in absolute ethanol, which freely dissolved the dihydrochloride salt. After evaporation to near dryness, the reaction mixture was "chased" with ethanol twice to remove excess hydrogen chloride. The resulting viscous oil was dissolved in ethanol (2 parts) and then added, with rapid stirring, to a large volume (20 parts) EtOAc (ethyl acetate). Filtration, washing with ethyl acetate (no heptane) and vacuum drying provided the dihydrochloride salt of compound (I) as a creamy-white powder. A total of 68 g (yield of 97%) was obtained of the final salt in high purity (99.6% AUC), which contained traces of EtOAc (4.8% w/w), EtOH (0.3% w/w), and heptane (0.6% w/w; from a final wash with heptane prior to vacuum drying). This salt was also crystallized (instead of the precipitation method described above) from hot EtOH/EtOAc to afford crystalline beads that had much lower entrapped solvent levels (only 0.26% w/w of EtOAc and 0.45% w/w of EtOH) and was free-flowing.

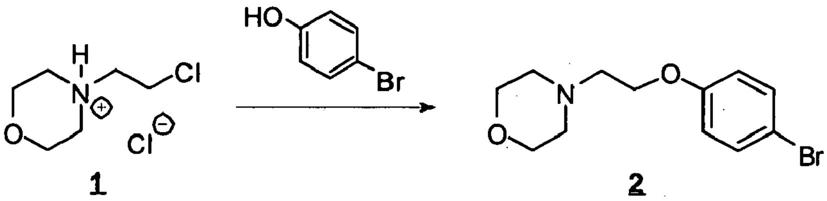

1 2

**Preparation of 4-(2-(4-bromophenoxy)ethyl)morpholine (2):**

A 5 L three-necked round-bottomed flask, equipped with mechanical stirrer, thermometer with adapter, condenser, and nitrogen inlet (on top of condenser), was charged with 1 (140.7 g, 0.756 mol), 4-bromophenol (130.6 g, 0.755 mol), anhydrous $K_2CO_3$ powder (367.6 g, 2.66 mol, 3.5 eq), and acetonitrile (1.3 L). The mixture was vigorously stirred (blade touching bottom of flask) at 80 °C (overnight), followed by dilution with DCM (500 mL) and heptane (200 mL) and filtration through Celite. Evaporation to dryness (rotovap, then high vac) gave **2** as a light yellow oil (216.00 g, yield of 100%, 96.3% AUC, contains 3.7% unreacted bromophenol). This material was used successfully without further purification.

$^1$H NMR ($CDCl_3$) δ 2.57 (t, 4 H), 2.79 (t, 2 H), 3.73 (t, 4 H), 4.08 (t, 2 H), 6.78 (d, 2 H), 7.37 (d, 2 H). MS (from LC/MS): *m/z* 287.1 [M + 1].

That the bromophenol can be readily removed was demonstrated on a 2 g sample by first dissolving the sample in toluene (8 g) and washing with 8 g of 15% aqueous NaOH; liquid chromatography showed no trace of unreacted bromophenol in the recovered product (1.97 g; 98.5% recovery).

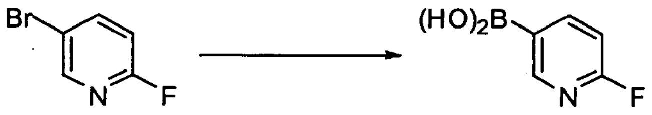

3 4

**Preparation of 6-fluoropyridin-3-ylboronic acid (4):**

To stirred and cooled (dry ice-acetone bath) anhydrous [TBME] (620 mL; in a 3 L three-necked round-bottomed flask equipped with mechanical stirrer, temperature probe with adapter, and nitrogen inlet) was added (via syringe) 2 M BuLi (352 mL, 0.704 mol, 1.2 eq). To this rapidly stirred and cooled (< -75 °C) mixture was added a solution of **3** (102.2 g, 0.581 mol) in anhydrous TBME (100 mL) over a period of 13 min during which time the internal temperature rose to -62 °C. The reaction was stirred for another 45 min (the temperature was maintained between -62 °C and -80 °C), followed by the rapid and sequential addition of four portions of triisopropylborate (total of 180 g, 0.957 mol, 1.65 eq). At the end of the addition the internal temperature had risen to -33 °C. After stirring an additional 45 min over the cold bath (internal temperature lowered from -33 °C to -65 °C), the cold bath was removed and the stirred mixture on its own rose to -22 °C over a period of 50 min. After warming (via water bath) to 6 °C over a period of 15 min, the stirred reaction mixture was placed in an ice-water bath and then quenched under nitrogen with a cooled solution of NaOH (160 g) in water (500 mL). Once the addition was complete, the internal temperature was 20 °C. This mixture was stirred at room temperature for 1.5 h. The aqueous layer was removed, neutralized to pH 7 with ~350 mL concentrated HCl, and then extracted with EtOAc (3 x 1 L). Because the pH was now 8-9, the aqueous layer was adjusted to pH 7 using ~15 mL concentrated HCl and extracted further (2 x 1 L) with ethyl acetate. The combined EtOAc extracts were dried ($Na_2SO_4$), filtered, and concentrated to a volume of ~150 mL. With swirling of the concentrate, heptane was added in portions (total volume of 300 mL) resulting in the precipitation/crystallization of the product. Filtration, washing of the solid with heptane (100 mL, 300 mL, then another 300 mL), and air drying gave the title product as an off-white solid (68.6 g, yield of 79-90%*; LC purity of 96.4%, NMR showed an estimated 5.5% w/w of heptane), which was used successfully without further purification. LC/MS showed it to be a mixture of the two following entities, the intensity of the higher molecular weight entity being major (*Note: yield of reaction is 79% if the boronic acid is assumed to be the only constituent and is 90% if it is assumed that the cyclic borate is the only constituent):

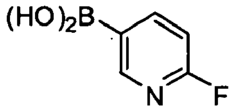

Exact Mass: 141.04

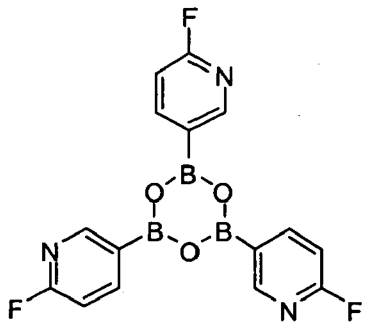

Exact Mass: 369.09

$^1H$ NMR ($CDCl_3$) δ 7.14 (dd, 1 H), 8.27 (ddd, 1 H), 8.39 (br s, 2 H, 2 O*H*), 8.54 (fine d, 1 H). MS (from LC/MS): *m/z* 143.0 [M + 1; for boronic acid] and 370.0 [M + 1; for cyclic borate above].

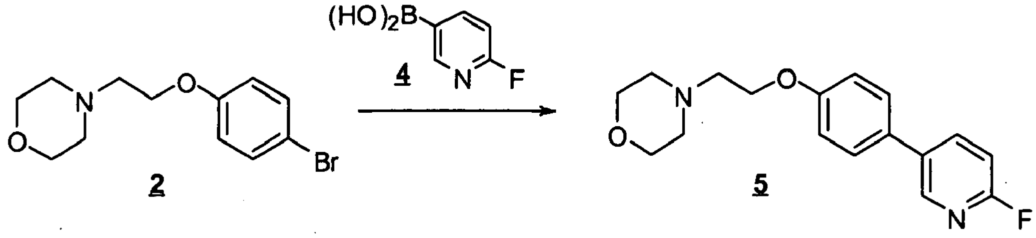

2 4 5

**Preparation of 4-(2-(4-(6-fluoropyridin-3-yl)phenoxy)ethyl)morpholine (5):**

A 2 L three-necked round-bottomed flask equipped with mechanical stirrer, thermometer and adapter, condenser, and nitrogen inlet (at top of condenser) was charged with 2 (110.7 g, 0.387 mol), **4** (71.05 g, 0.477 mol, 1.23 eq) and DME (700 mL). The resulting stirred solution was degassed by passing a rapid stream of nitrogen through the stirred solution over a period of 5 min followed by the addition of a degassed solution of $Na_2CO_3$ (121.06 g, 1.142 mol, 3 eq) in $H_2O$ (250 mL) and also solid $Pd(PPh_3)_4$ (19.8 g, 0.044 eq). Immediately after the last addition, the head space above the reaction mixture was purged with nitrogen and the mixture then stirred at 80-85 °C (internal temperature) for 7 h, followed by cooling to room temperature. Because of the lack of an aqueous layer, the supernatant was decanted, leaving behind the inorganic salts (with adsorbed water). The reaction flask with the inorganic salts was washed with 50% dichloromethane/ethyl acetate (2 x 250 mL), the washes being added to the decanted supernatant. These combined organics were dried ($Na_2SO_4$), filtered, and evaporated to dryness to a dark brown oil (148 g). To this oil was added 150 g of 50% heptane/isopropyl alcohol (IPA) and after swirling and cooling (via ice water bath), crystallization began. Additional heptane (50 g) was added and the resulting solid was filtered, washed, and air dried to give 48 g of a light brown solid. After evaporating the filtrate to dryness, the resulting mixture was swirled in 100 mL of 50% heptane/IPA followed by the addition of more heptane (~100 mL), stoppering and placing in the freezer for crystallization. The resulting solid was filtered, washed with heptane, and air dried to give 61 g of a gummy solid. Evaporation of the resulting filtrate gave an oil (34 g) which contained significant less polar impurities including $Ph_3P=O$ and so it was partitioned between 2 N HCl (240 mL) and EtOAc (220 mL). The bottom aqueous layer was removed and then stirred with EtOAc while neutralizing with $K_2CO_3$ to a pH of 7-8. The EtOAc layer was dried, filtered, and evaporated to dryness (22 g). The 48 g, 61 g, and 22 g portions were chromatographed over silica gel (1.1 Kg) packed in DCM. Elution with DCM (400 mL), 50% DCM/EtOAc (5 L), and then 50% DCM/EtOAc (8 L) containing increasing amounts of MeOH/$Et_3N$ (beginning with 1.5% MeOH/1% $Et_3N$ and ending with 5% MeOH/3% $Et_3N$) gave 77.68 g of a viscous oil (purity 98.0%) which immediately crystallized upon swirling in heptane (300 mL). Filtration, washing with heptane and air drying gave 75.55 g (98.7% AUC) of solid **5.** Additional pure **5** (total of 3.9 g, 98.6-99.3% AUC) was obtained from earlier chromatographic fractions containing $Ph_3P=O$ by cleaning them up as done for the above 34 g sample, followed by evaporative crystallization. The total yield of 5 was 79.5 g (68%).

$^1H$ NMR ($CDCl_3$) δ 2.59 (t, 4 H), 2.84 (t, 2 H), 3.75 (t, 4 H), 4.16 (t, 2 H), 6.97 (dd, 1 H), 7.01 (d, 2 H), 7.46 (d, 2 H), 7.92 (ddd, 1 H), 8.37 (fine d, 1 H). MS (from LC/MS): *m/z* 303.2 [M + 1].

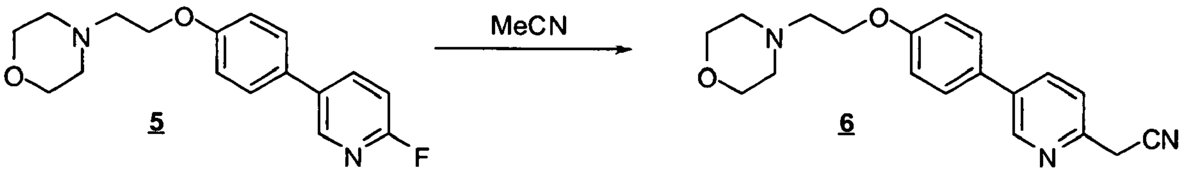


**Preparation of 2-(5-(4-(2-morpholinoethoxy)phenyl)pyridin-2-yl)acetonitrile (6):**

A 3 L three-necked round-bottomed flask was equipped with mechanical stirrer, thermometer and adapter, additional funnel, and nitrogen inlet (on top of addition funnel, positive pressure through a bubbler). With a rapid stream of nitrogen going through the bubbler, the stopper was removed and the flask was charged with KHMDS (415.8 g, 2.08 mol) and then anhydrous THF (1 L). To the stirred and cooled (ice/methanol bath, internal temperature of solution was -8 °C) KHMDS/THF solution was added dropwise a solution of MeCN (70 g) in THF (110 mL) over a period of 22 min followed immediately by the relatively rapid (4 min) addition of a solution of **5** (79.06 g, 0.262 mol) in THF (400 mL), after which time the internal temperature of the reaction mixture had reached 10 °C. With continued cooling (1 h) the internal temperature was -6 °C and by TLC the reaction appeared complete. After an additional 30 min (internal temperature of -3 °C), the reaction mixture was quenched with saturated brine (1 L) and diluted with EtOAc (500 mL). After removing the aqueous layer, the organic solution was dried ($Na_2SO_4$), filtered, and evaporated to dryness (to an oil) followed by completely dissolving in IPA (150 mL), diluting with heptane (300 mL), adding seed crystals (prepared by dissolving ~100 mg of crude oil in IPA (~150 mg) and diluting with heptane (~2.5 mL)), and allowing to stand overnight. After stirring to break up the crystalline solid, the solid was filtered, washed with 250 mL 2:1 heptane/IPA and then multiple washes with heptane and air dried to give 64.38 g (yield of 76%) of title product **6** as a crystalline tan solid (LC purity of 99.3%). Another 5.88 g of less pure material was obtained from the filtrate.

$^1H$ NMR ($CDCl_3$) δ 2.59 (t, 4 H), 2.84 (t, 2 H), 3.74 (t, 4 H), 3.97 (s, 2 H), 4.17 (t, 2 H), 7.02 (d, 2 H), 7.46 (d, 1 H), 7.51 (d, 2 H), 7.87 (dd, 1 H), 8.77 (fine d, 1 H). MS (from LC/MS): *m/z* 324.4 [M + 1].

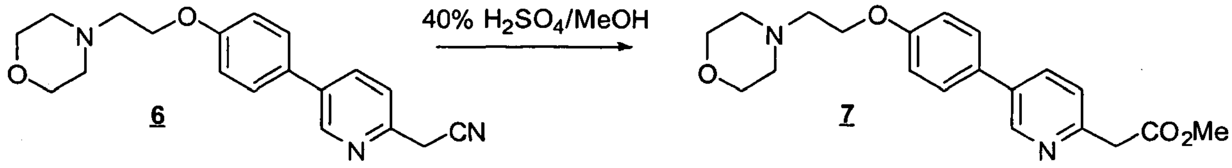


**Preparation of methyl 2-(5-(4-(2-morpholinoethoxy)phenyl)pyridin-2-yl)acetate (7):**

A 2 L single-necked round-bottomed flask was charged with **6** (64.00 g, 0.198 mol) and MeOH (360 g) followed by the slow, careful, and dropwise addition of $H_2SO_4$ (240 g) and the resulting homogeneous solution stirred at reflux (115 °C oil bath) until the reaction was complete (25 h with 0.8% unreacted starting material) with 3.5% $ArCH_2CO_2H$. After brief cooling, $MgSO_4$ (75 g) was added and the mixture swirled and allowed to stand an additional 45 min (composition now 96.3% product, 0.8% unreacted starting material, and 2.5% $ArCH_2CO_2H$). The reaction mixture was then added slowly to a rapidly stirred and cooled (ice-water bath) mixture of DCM (2 L) and a solution of $K_2CO_3$ (450 g) in $H_2O$ (600 mL). The resulting emulsion was allowed to stand overnight. The clear portions of organic solution were siphoned off and the remainder portions were treated iteratively with water and DCM, the clear organics being combined with the original portion that was siphoned off. The combined organics were dried ($Na_2SO_4$), filtered, and concentrated to a volume of ~1.2 L followed by the addition of 300 mL of 5% EtOAc (in heptane) and then heptane (300 mL) and the mixture concentrated (rotovap with heat) again to remove the DCM. At this point 15 mL EtOAc was added and the hot mixture swirled until crystallization had begun, swirling continued until crystallization was near complete, and then allowed to stand and cool to room temperature for complete crystallization. The solid was then filtered, washed with 300 mL 5% EtOAc (in heptane) and heptane (100 mL) and then fully air dried to give 57.74 g (yield of 82%) of **7** as a light yellow solid (98.9% AUC). Another 3.94 g of clean product (97.9% AUC) was obtained from the filtrate (total yield of 87%).

$^1H$ NMR ($CDCl_3$) δ 2.60 (t, 4 H), 2.84 (t, 2 H), 3.74 (overlapping t and s, 6 H), 3.89 (s, 2 H), 4.17 (t, 2 H), 7.01 (d, 2 H), 7.34 (d, 1 H), 7.49 (d, 2 H), 7.80 (dd, 1 H), 8.74 (fine d, 1 H). MS (from LC/MS): *m/z* 357.4 [M + 1].

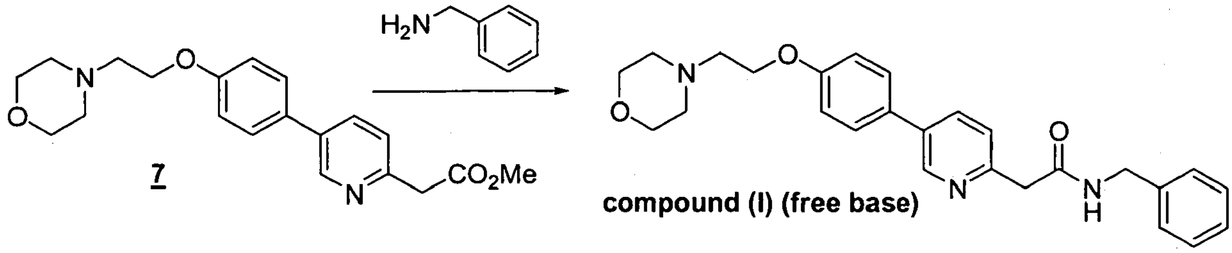

**Preparation of 2-(5-(4-(2-morpholinoethoxy)phenyl)pyridin-2-yl)-N-benzylacetamide (Compound (I) free base).**

A 1 L single-necked round-bottomed flask was charged with **7** (61.4 g, 0.172 mol), benzyl amine (55.6 g, 0.519 mol, 3 eq), and anhydrous anisole (300 g) and then stirred at reflux until reaction was essentially complete (23 h, 165 °C oil bath temperature; internal temperature was 147 °C) and then allowed to cool to near room temperature. A portion (1 mL) of the reaction mixture was diluted with toluene (1 mL) resulting in the complete crystallization of that portion. This seed was then added to the reaction mixture and allowed to stand until the whole reaction mixture had crystallized to a single block. Toluene (150 mL) was added and the mixture swirled to break up the solid. Heptane/toluene (1:1, 100 mL) was added and the solid mixture broken up further. Finally, heptane (50 mL, then 25 mL) was added and the mixture broken up even further, allowing to stand an additional 30 min before filtering the solid. Filtration of the solid, washing with 2:1 toluene/heptane (300 mL), 1:2 toluene/heptane (300 mL), and then heptane (2 x 300 mL), and then drying (air, then high vac) gave 60.16 g (yield of 81%) of title product as a white solid (≥98.9% AUC). Another 2.5 g of less pure (97.4%) material was obtained from the mother liquors.

$^1H$ NMR ($CDCl_3$) δ 2.60 (t, 4 H), 2.83 (t, 2 H), 3.74 (t, 4 H), 3.82 (s, 2 H), 4.18 (t, 2 H), 4.49 (d, 2 H), 7.01 (d, 2 H), 7.2-7.35 (m, 6 H), 7.49 (d, 2 H), 7.64 (br t, 1 H), 7.81 (dd, 1 H), 8.69 (fine d, 1 H). MS (from LC/MS): *m/z* 432.5 [M + 1].

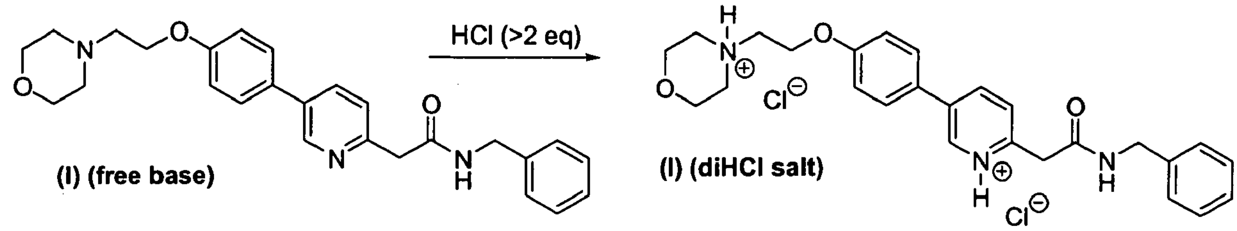


**Preparation of 4-(2-(4-(6-(2-(benzylamino)-2-oxoethyl)pyridinium-3-yl)phenoxy)ethyl)-morpholin-4-ium chloride (Compound (I), diHCl salt).**

To a stirred suspension of compound (I) (free base, 60.00 g) in absolute EtOH (600 mL) was added 170 mL of 2.5 M HCl (in ethanol), 25 mL EtOH being added to wash down the sides of the flask. The resulting homogeneous solution was stirred at room temperature (20 min) and then evaporated to near dryness (to frothing). After chasing with EtOH (2 x 150 mL), the residue was taken up again in EtOH (150 mL) and then was followed by the slow addition of heptane until the mixture appeared saturated (33 mL required for cloudiness to remain). After sitting overnight, two layers had formed. After adding additional heptane (250 mL) crystallization still could not be induced and so the reaction mixture was concentrated to a volume of ~200 mL at which time the mixture was homogeneous. This thick homogeneous solution was added dropwise to very rapidly stirred (mechanical) EtOAc (2 L). After the addition was complete, a 25 mL EtOH rinse of the original flask and addition funnel was added to the rapidly stirred mixture. The rapid stirring was continued for another ~1 h and then the mixture was filtered and the solid (partly gummy) was washed with EtOAc (300 mL) and then heptane. As soon as the heptane wash began, the solid got much gummier. The fritted Buchner funnel and its contents were covered (paper towel/rubber band) and immediately placed in the vacuum oven. After overnight vacuum at ~45 °C, the vacuum was released under nitrogen, and the Buchner funnel containing the product (foamy solid) was immediately placed in a zip-lock back and then, under nitrogen (glove bag), transferred to a bottle and the foamy solid broken up (spatula) to a powder. A second night under high vacuum (~45 °C) resulted in only 1.3 g of additional weight loss. Constant weight was essentially attained with the third night of high vacuum (~45 °C) where only 0.2 g of weight was lost. The final weight of material was 68.05 g (yield of 97%), containing 0.29 eq (4.8% w/w) of EtOAc, 0.035 eq (0.3% w/w) EtOH, and 0.03 eq (0.6% w/w) heptane. The purity was 99.6%.

$^1H$ NMR (DMSO-$d_6$) $\delta$ 3.1-3.3 (m, 2 H), 3.45-3.65 (m, 4 H), 3.8-4.0 (m, 4 H), 4.11 (s, 2 H), 4.32 (d, 2 H), 4.57 (t, 2 H), 7.19 (d, 2 H), 7.2-7.4 (m, 5 H), 7.88 (d, 2 H), 7.93 (d, 1 H), 8.68 (dd, 1 H), 8.99 (br t, 1 H), 9.10 (fine d, 1 H), 11.8 (br s, 1 H). MS (from LC/MS): *m/z* 432.5 [M + 1 of free base].

Elemental analysis (for $C_{26}H_{29}N_3O_3$ • 2 HCl • 0.035 EtOH • 0.29 EtOAc • 0.03 heptane • 0.8 $H_2O$):

a. Calculated (%): C, 60.03; H, 6.54; N, 7.65; Cl, 12.91
b. Observed (%):C, 59.85/59.97; H, 6.54/6.47; N, 7.67/7.67; Cl, 13.10/13.24

Calculated FW: 534.63 (does not take into account the 0.8 $H_2O$ which probably arose during handling of this very hygroscopic powder, since $^1H$ NMR shows no evidence for $H_2O$).

The ethyl chloride level in this material was measured and found to be 98 ppm. The sample was also analyzed and found to contain 5,800 ppm of heptane.

Analysis of another portion of this sample yielded the following results: 99.6% AUC, 1640 ppm ethanol, 41,480 ppm ethyl acetate, 5600 ppm heptane, no anisole detected, and 120 ppm ethyl chloride.

A procedure for recrystallizing the salt was also developed using the above dried salt. This procedure would work just was well on the highly pure crude salt (containing residual EtOH) obtained from concentrating the HCl salt-forming reaction mixture:

The salt (575 mg) was dissolved in twice the mass of absolute EtOH (1.157 g) and then heated under nitrogen. To this hot solution (stirred) was added 1.6 g of 25% EtOH (in EtOAc) followed by the addition of EtOAc (0.25 mL) resulting in a cloudiness that remained. The cloudy hot solution was allowed to cool to room temperature during which time crystallization occurred. After crystallization was complete (2 h), the crystalline solid was filtered, washed with anhydrous EtOAc (~40 mL), and vacuum dried to give 424 mg of the dihydrochloride salt of compound (I) as a free-flowing solid (tiny beads, 99.8% AUC) containing only 0.05 eq (0.45% w/w) of EtOH and 0.015 eq (0.26% w/w) of EtOAc. Slightly better recovery (460 mg from 586 mg) was attained using isopropanol/EtOAc but the level of solvent entrapment was higher [0.085 eq (1.0% w/w) of isopropanol and 0.023 eq (0.4% w/w) of EtOAc].

**Example 3: Large Scale Synthesis of Compound (I) di-HCl**

Reagents and solvents were used as received from commercial suppliers. Progress of the reactions was monitored by HPLC, GC/MS, or $^1$H NMR. Thin-layer chromatography (TLC) was performed using Analtech silica gel plates and visualized by UV light (254 nm). High pressure liquid chromatography (HPLC) was performed on an Agilent 1100 Series instruments. Proton and carbon nuclear magnetic resonance spectra were obtained using a Bruker AV 300 at 300 MHz for proton and 75 MHz for carbon. The solvent peak was used as the reference peak for proton and carbon spectra.

**Preparation of 4-(2-(4-Bromophenoxy)ethyl)morpholine (2)**

A 50 L jacketed reactor equipped with a reflux condenser and temperature probe was charged with 4-(3-chloropropyl)morpholine (2.44 kg, 0.54 mol), 4-bromophenol (2.27 kg, 0.54 mol, 1.0 equiv.), powdered potassium carbonate (6.331 kg, 1.88 mol, 3.50 equiv.), and DMF (12.2 L) and stirred. The reaction mixture was then heated to 60-65 °C and stirred overnight. After 17.5 h, the reaction mixture was cooled to 20-25 °C. The reaction mixture was charged to a different reactor equipped with bottom valve for the work-up. While maintaining a temperature between 20-30 °C, DI water (48.7 L) was charged to the reactor. The phases were separated. The aqueous layer was extracted with MTBE (3 × 24.4 L). To the combined organics, DI water (18.3 L) and then 6M sodium hydroxide (18.2 L) were added. The mixture was stirred for 2-5 minutes and the phases were separated. The organic phase was washed with water (24.4 L) and brine (24.4 L), dried over magnesium sulfate, filtered, and concentrated to give 3370g of a yellow oil (89% crude yield, 99.4% AUC by HPLC).

**Preparation of 6-fluoropyridin-3-ylboronic acid (4)**

A 72 L reactor equipped with reflux condenser, and temperature probe. To the reactor 5-bromo-2-fluoropyridine (1.17 L, 0.568 mol), toluene (18.2 L), and triisopropyl borate (3.13 L, 0.68 mol, 1.2 equiv.) were charged and stirred. Tetrahydrofuran (4.4 L) was added to the reactor and the reaction mixture was cooled to between -35 to -50 °C. While maintaining a temperature between -35 to -45 °C, n-butyl lithium (2.5 M solution of hexanes, 5.44 L, 0.68 mol, 1.2 equiv.) was cautiously added to the reactor. After 5 h, the reaction was deemed complete and the reaction mixture was warmed to between -15 to -20 °C. To the reaction was added 2M HCl (11.80L) to the reactor while maintaining a temperature between -15 °C and 0 °C. The reaction mixture was stirred at 18 to 23 °C for (16 h) and the phases were separated. The organics were then extracted with 6 M sodium hydroxide (6.0 L). The acidic anbasic aqueous phases were mixed in the reactor and 6 M HCl (2.5 L) was added until pH 7.5 was achieved. Sodium chloride (6.0 kg) was then added to the aqueous phase. The aqueous phase was then extracted with THF (3 × 20 L). The combined organics were dried with magnesium sulfate and concentrated to give 1300 g of a tan solid (81% crude yield).

**Preparation of 4-(2-(4-(6-fluoropyridin-3-yl)phenoxy)ethyl)morpholine (5)**

A 72 L reactor equipped with reflux condenser, sparging tube, bubbler, and temperature probe was charged with 6-fluoropyridin-3-ylboric acid (2.84 kg, 1.24 equiv.), 4-(2-(4-bromophenoxy)ethyl)morpholine (4.27 kg, 1.0 equiv.), and DME (27 L). Agitation was started and sodium carbonate (4.74 kg, 3.0 equiv.) as a solution in DI water (17.1 L) was then charged to the reaction mixture. Argon was bubbled through the reaction mixture for 50 minutes. Under an argon atmosphere, tetrakis(triphenylphosphine)palladium (750 g, 0.04 equiv.) was added to the reaction mixture as a slurry in DME (1.0 L). The reaction mixture was heated to 75 - 85 °C and stirred overnight (17 h). The reaction mixture was cooled to between 18 - 22°C. DI water (26.681kg) and MTBE (26.681 L) were charged to the reactor and stirred for 5 minutes. The phases were separated and the aqueous phase was extracted with MTBE (2 × 26.7 L). The combined organics were extracted with 2M HCl (1 × 15.0 L, 3 × 21.8 L). The aqueous phase was then charged back to the reactor and ethyl acetate was added (26.7 L). The pH was adjusted to 6.2 using 6 M sodium hydroxide (26.7 L) while maintaining a temperature between 15 - 25 °C. The phases were separated and the aqueous phase was extracted with ethyl acetate (2 × 26.7 L). The combined organics were dried with magnesium sulfate and concentrated to give 4555 g of a residue (101% crude yield, 67.1% AUC by HPLC).

**Purification of 4-(2-(4-(6-fluoropyridin-3-yl)phenoxy)ethyl)morpholine (5)**

The crude product (575 g) was purified by silica gel chromatography by eluting with methanol/ethyl acetate/heptane (30% ethyl acetate/heptane, 50% ethyl acetate/heptane, 75% ethyl acetate/heptane, 100% ethyl acetate, and 5% methanol/ethyl acetate). Concentration of the pure fractions by TLC (10% methanol/dichloromethane, $R_f$ = 0.3) provided 420 g of a light brown solid (73% recovery, >99.9% AUC by HPLC).

**Preparation of 2-(5-(4-(2-morpholinoethoxy)phenyl)pyridin-2-yl)acetonitrile (6)**

A 1 M solution of NaHMDS (2.0 L, 5.0 equiv.) in THF was charged to a 5-L flask and cooled to -20 to -15 °C. While maintaining a temperature below -10 °C, fluoride (119.7g, 1.0 equiv.) in THF (500 mL) was charged to the flask over 20 minutes. Acetonitrile (82.5 mL, 4.0 equiv.) in THF (170 mL) was added to the flask over 20 minutes, while maintaining a temperature below -10 °C. The reaction mixture was then stirred for 1 h. To the reaction was added brine (1.5 L, 12.6 vol.) at a rate as to maintain a temperature below 10 °C. The solution was then warmed to room temperature and the layers were allowed to separate. The mixture was filtered over Celite and washed with THF (1 × 200 mL, 1 × 100 mL). The aqueous phase was extracted with toluene (750 mL). The combined organics were dried with magnesium sulfate, filtered, washed with toluene (2 × 250mL), and concentrated to dryness. Toluene (1L) was added and the solution was concentrated to dryness again to give 169.8 g of an oil. MTBE (1190 mL, 7 vol.) was added to the oil at 50. °C and stirred for 15 minutes. Heptane (850 mL, 5vol.) was added over ten minutes at 50 °C. The mixture was then cooled to room temperature over 1.5 h and stirred for 2 h. The slurry was filtered, washed with 1:4 MBTE/heptane (2 × 100 mL), and dried in an oven overnight at 45 °C to give 102.3 g of an off-white solid (80% yield, 98.8% AUC by HPLC).

**Preparation of methyl 2-(5-(4-(2-morpholinoethoxy)phenyl)pyridin-2-yl)acetate (7)**

Nitrile **6** (101 g) and methanol (1.01 L, 10 vol.) were charged to a 3-L flask equipped with stir bar and thermocouple. Concentrated $H_2SO_4$ (175 mL, 10.0 equiv.) was added drop wise to the solution over 15 minutes while maintaining a temperature below 60 °C. Followed by 30% fuming sulfuric acid (124 mL) was added drop wise to the solution while maintaining a temperature below 60 °C. The solution was then heated to reflux with a heating mantle and stirred overnight. When the reaction was deemed complete, it was cooled to 20 °C. In a second flask (22 L), saturated sodium bicarbonate (10.7 L) and dichloromethane (1.1 L) were charged and cooled to 15 °C. While maintaining a temperature below 20 °C, the reaction mixture was added to the sodium bicarbonate/dichloromethane mixture. The quench was stirred for 15 minutes and the phases were separated. The aqueous phase was extracted with dichloromethane (1 × 550mL, 1 × 300mL). The combined organics were dried with magnesium sulfate and concentrated to dryness to give 105 g of an orange solid (94% crude yield, 97.7% AUC by HPLC).

**Preparation of 2-(5-(4-(2-morpholinoethoxy)phenyl)pyridin-2-yl)-N-benzylacetamide (Compound (I))**

Ester **7** (103 g), anisole (513 mL, 5 vol.), and benzylamine (94 mL, 3.0 equiv.) were charged to a 3 L flask equipped with thermocouple and overhead stirrer. The reaction mixture was then heated to 142 °C and stirred for two days. The reaction mixture was cooled to 45-50 °C and stirred for 2 hours. To the mixture was added n-heptane (1.5 L) dropwise over an hour. The solution was cooled to room temperature over three hours and then stirred overnight. The resulting slurry was filtered, washed with 4:1 Anisole/n-heptane (200 mL) and n-heptane (3 ×100 mL). Drying in the oven overnight, the resulting product was 112.1g of a tan solid (90% yield, 99.6% AUC by HPLC). The use of a single isomer of heptane was essential to adequately quantitate the residual solvent.

**Preparation of 2-(5-(4-(2-morpholinoethoxy)phenyl)pyridin-2-yl)-N-benzylacetamide dihydrochloride salt (Compound (I)·2HCl)**

EtOH (1.0 L) was charged to a 2-L flask and acetyl chloride (62.5 mL, 3.0 equiv.) was added slowly to the flask and stirred for 40 minutes. The resulting solution was added to compound (I) (100 g) over 30 minutes while maintaining a temperature of 30 °C. The solution was concentrated to a mass of 270 g. The concentrated solution was added to ethyl acetate (2 L) over 20 minutes with rapid stirring. The mixture was stirred overnight and then filtered under nitrogen to give two distinct solid products, tan solids (73.5 g) and darker solids (42.2 g). The solids were dry blended to give a combined yield of 99%. The HPLC analysis indicated 99.0% purity (AUC).
Analysis indicated that ethanol was present at 2530 ppm, ethyl acetate at 48,110 ppm, ethyl chloride at 170 ppm, and no heptane and anisole were detected. Palladium content was assayed three times and measured to be 29 ppm, 2 ppm, and less than 1 ppm.

**Crystallization Study of Compound (I)·2HCl**

The experiments shown in the table below were conducted to explore different crystallization and precipitation conditions of compound (I)·2HCl.

**Crystallization Study of Compound (I) 2HCl**

| Salt Formation Conditions | | | | | Crystallization Conditions | | | | | Comments |
|---|---|---|---|---|---|---|---|---|---|---|
| Expt | Amide (g) | Lot | Solvent | Acid | Solvent (vol) | Lot | EtOAc (vol) | Temp (C) | Nice Solids (y/n) | |
| 02BP097 A | 0.1 | 02BP09 0D (off-white) | IPA | IPA-HCl (5M) | IPA (10) | -- | 10 | 60 | N | Gummy solids/ slurry formed as EtOAc added |
| 02BP097 B | 0.1 | 02BP09 1E (white) | IPA | IPA-HCl (5M) | IPA (10) | -- | -- | 60 | N | Gummed out w/ cooling |
| 02BP097 C | 0.1 | 02BP09 1E (white) | IPA | IPA-HCl (5M) | IPA (15) | -- | 6 | 65 | N | Dried w/ EtOAc first; product oiled out w/ cooling |
| 02BP097 | 0.1 | 02BP09 | -- | IPA- | EtOAc/ | -- | -- | 60 | N | IPA-HCl |
| D | | 1E (white) | | HCl (5M) | IPA | | | | | added to amide solution; gummed out during addition (2 drops) |
| 02BP097 E | 0.3 | 02BP09 0D (off-white) | EtOH | IPA-HCl (5M) | EtOH (3.3) | Acros | 6.3 | 30-60 | Y | Solids observed at 30°C after EtOAc added; slow filtering |
| 02BP097 F | 0.3 | 02BP09 3G (tan solid) | EtOH | IPA-HCl (5M) | EtOH (3.3) | Acros | 6.6 | 60 | Y | Solids observed during cooling after EtOAc added; slow filtering |
| 02BP097 G | 0.3 | 02BP09 3G (tan solid) | PrOH | IPA-HCl (5M) | PrOH (3.3) | -- | 1.7 | 60 | Y | Solids observed during cooling after EtOAc added; slow filtering |
| 02BP097 H | 0.3 | 02BP09 3G (tan solid) | BuOH | IPA-HCl (5M) | BuOH (5) | -- | 1.2 | 60 | Y | Solids observed during cooling after EtOAc added; very slow filtering |
| 02BP098 A,B,C | 1.0 | 02BP09 3G (tan solid) | EtOH | IPA-HCl (5M) | EtOH (3.3) | Ald | 4-6 | 60 | N | Cloudiness observed earlier than expected; oiled out |
| 02BP098 D | 1.0 | 02BP09 3G (tan solid) | EtOH | EtO H-HCl (2.5 M) | EtOH (3.3) | Aid | 4.6 | 60 | N | Oiled out upon cooling |
| 02BP098 E | 0.3 | 02BP09 0D (off-white) | EtOH | EtO H-HCl (2.5 M) | EtOH (3.3) | Ald | 5.3 | 60 | N | Oiled out from EtOAc addition |
| 02BP098 F | 0.3 | 02BP09 1E (white) | EtOH | IPA-HCl (5M) | EtOH (3.3) | Acros | 6 | 60 | N | Oiled out upon addition of EtOAc |
| 02BP098 G | 0.3 | 02BP09 1E (white) | PrOH | IPA- HCl (5M) | PrOH (3.3) | -- | 4 | 60 | N | Oiled out w/ cooling |

Precipitation was achieved by an inverse addition of Compound (I)·2HCl in a concentrated solution of ethanol to a large volume of rapidly stirring ethyl acetate. This precipitation procedure was implemented for the demonstration batch resulting in the formation of two distinct solid types. The two distinct solid types were physically separated and filtered separately. A less dense tan solid (lot 02BP111E, 74 g, 99.1% AUC by HPLC) was filtered first followed by a denser darker solid (lot 02BP111F, 43 g, 99.1% AUC by HPLC). After drying in a vacuum oven and before blending the two solids a sample of each was retained for analysis. The HPLC data for the two samples were comparable while the DSC and XRPD were different.

Both of the HPLC preparations were greater than 99.0% pure (by area %), the lot 02BP111E sample showed a single endothermic event at approximately 198 °C while the lot 02BP111F sample showed two endothermic events at 117 °C and 189 °C. The XRPD data for the two samples were also different the lot 02BP111E sample seemed crystalline while the lot 02BP111F sample appeared to be amorphous. The HPLC data, the XRPD data and the DSC data support that the two samples are different forms of the same material.

The two lots of compound (I)·2HCl (lot 02BP111E and 02BP111F) were dry blended resulting in a new lot of compound (I)·2HCl (lot 02BP111G). Compound (I)·2HCl (lot 02BP111G) contained 170 ppm of ethyl chloride.

**Example 4: Preparation of 2-(5-(4-(2-morpholinoethoxy)phenyl)pyridin-2-yl)-*N*-benzylacetamide mesylate (Compound (I)·MSA).**

**Preparation of 2-(5-(4-(2-morpholinoethoxy)phenyl)pyridin-2-yl)acetonitrile (6)**

To round bottom reactor 1 was charged sodium bis(trimethyldisilyl)amide (1.0 M in THF, 23.2 L) and the solution cooled to ≤-10 °C over 52 minutes. To a glass carboy, under nitrogen, was charged compound **5** (1400 g, 1 wt) and THF (7.0 L, anhydrous, 5 vol)). The batch was stirred with an air powered stirrer under nitrogen. The batch was not completely soluble and was a hazy solution. The solution of compound **5** was added to reactor 1 over 41 minutes via a 5-L addition funnel. A solution of acetonitrile (965 mL, anhydrous, 0.69 vol) in THF (2.0 L, anhydrous, 1.43 vol) was prepared and added to reactor 1 over 48 minutes at ≤-10 °C via the same addition funnel (a minor amount of a yellow solid was present on the reactor wall). After aging for 45 minutes at ≤-10 °C the batch was sampled for analysis and compound **5** was 0.03% by conversion (specification ≤1.5% by conversion). One hour 24 minutes after sampling, brine (17.6 L, 12.6 vol) was added to reactor 1 over 52 minutes and gave a poorly stirring batch (resembled an emulsion). A pad of diatomaceous earth was prepared on a 24-inch polypropylene funnel (1026 g Celite 545 slurried in 3.3 L water with the filtrate discarded). The batch was filtered under suction via the pad and the reactor rinsed with THF (1.75 L, 1.25 vol) and the rinse transferred to the cake. The cake was rinsed with a second portion of THF (1.75 L, 1.25 vol) and the total filtration time was 1 hour 17 minutes. The filtrate was transferred to reactor 2 and the phases separated and held overnight (the batch was held in the reactor under nitrogen). The organic phase (approximately 34.5 L) was drained and the aqueous phase extracted with toluene (8.1 L, 5.8 vol), stirring for 16 minutes and settling over 12 minutes. It is possible to omit the toluene extraction and simply add toluene directly to the organic phase after separation. The aqueous phase (approximately 19 L) was removed and the organic phases combined and dried in reactor 2 with magnesium sulfate (1400 g, 1 wt, anhydrous) over 55 minutes. The batch was filtered via a 24-inch polypropylene funnel equipped with an inline filter into a glass carboy. The batch was blanketed with argon and stored in the cold room (2-8 °C) pending concentration. The following day, the batch was concentrated to a residue and rinsed with toluene (11.8 L, 8.4 vol), which in turn was concentrated (water bath 50 ±5°C). At the point of the toluene addition, the batch was an orange slurry and remained so after concentration. The total concentration time was 5 hours 3 minutes.

To reactor 3 was charged MTBE (13.9 L, 9.9 vol, ACS) which was then heated to 45 ± 5 °C. The MTBE was drained and approximately 2 L of MTBE was used to slurry the batch from the bulb into reactor 3. The remaining MTBE was added to reactor 3 maintaining the batch at 45 ± 5 °C and the batch then aged for 33 minutes in this temperature range. *n*-Heptane (10 L, 7.1 vol, 99%) was then added to reactor 3 over 39 minutes maintaining the batch at 45 ± 5 °C. The heat source was disconnected the batch was cooled to 25 ± 5 °C over 4 hours 5 minutes and aged at that temperature range for 27 hours 4 minutes. The batch was then filtered under suction via a 24-inch polypropylene funnel (PTFE cloth), covered and sucked dry under nitrogen. The total filtration time was 20 minutes. The orange batch (net wet weight 1322 g) was dried to constant weight over 48 hours 3 minutes in a vacuum oven set at 45 ± 5 °C. The batch was transferred to two 80 oz amber glass jars (Teflon lined closure) and blanketed with argon (1217 g of **6,** 81% of theory).

**Preparation of methyl 2-(5-(4-(2-morpholinoethoxy)phenyl)pyridin-2-yl)acetate (7)**

To a 22-L reactor was charged compound **6** (900 g, 2.78 mol) and methanol (9.0 L, 10 vol, anhydrous). Sulfuric acid (1115 mL, fuming) was added to the suspension over 2 hours 11 minutes to give a dark solution. The maximum temperature was 65.5 °C (target <65 °C). Sulfuric acid (1565 mL, 1.74 vol, concentrated) was added to the batch over 1 hour 49 minutes and the batch then heated to visible reflux (74 °C) over 18 minutes. The batch was maintained at that

temperature for 16 hours 57 minutes. The visible gentle reflux was noted to be absent, so the batch was then heated again to reflux at 79-80 °C over 2 hours 15 minutes. The batch was maintained at that temperature (80 ± 5 °C) for 10 hours 57 minutes and the heat source then disconnected; an additional charge of methanol (0.75 L, 0.8 vol, anhydrous) was performed after 26 hours 4 minutes to replenish the lost solvent volume. It was estimated that 2.5-3.3 L of solvent was lost by evaporation. HPLC analysis after 42 hours 31 minutes from reflux indicated that the level of compound **6** was 0.6% by conversion (specification ≤1.0%). To each of reactor 1 and 2 was charged methylene chloride (4.8 L, 5.3 vol) and sodium hydrogen carbonate solution (48 L, 53.3 vol, saturated). The sodium hydrogen carbonate solutions were stored overnight at 2-8 °C and removed the next morning. Half the batch from the 22-L reactor was added in portions to each reactor over 47 and 44 minutes respectively (batch temperature was 12-13 and 14-15 °C, respectively). The quench was accompanied by evolution of carbon dioxide (vigorous at the vortex). The batches from each reactor were then transferred to a 200-L reactor and the batch stirred for 16 minutes, then settled over 25 minutes and the organic phase separated. The aqueous phase was extracted successively with two portions of methylene chloride (5 L, 5.6 vol and 2.7 L, 3 vol); each extraction took place over 15 minutes stirring with settling over 6 and 9 minutes respectively. The combined organic phase was transferred to reactor 3 and dried with magnesium sulfate (900 g, 1 wt, anhydrous) over 35 minutes. The batch was then filtered under suction via a 24-inch polypropylene funnel fitted with Sharkskin cloth and equipped with an inline filter (10 micron, Pall P/N 12077). The filtrate was concentrated on a rotary evaporator over a total of 2 hours 18 minutes at 40 ± 5 °C (water bath temperature). After 54 minutes the batch solidified and formed balls. These were broken up and concentration continued. The batch (a mixture of fine solids and brittle chunks) was then further ground and returned to the bulb and concentration continued. The batch was transferred to an 80-oz amber jar with a Teflon lined lid and blanketed with argon to give compound **7** (871 g, 88% of theory).

**Preparation of 2-(5-(4-(2-morpholinoethoxy)phenyl)pyridin-2-yl)-N-benzylacetamide (Compound (I))**

To a 22-L reactor was charged compound **7** (650 g, 1.82 mol), anisole (3.25 L, 5 vol, anhydrous) and benzylamine (600 mL, 0.92 vol, 3 equiv). The batch (approximately 18 °C) was heated to 142 ± 5 °C over 1 hour 44 minutes, with dissolution occurring at 30 °C. The batch was maintained at 142 ± 5 °C for 69 hours 30 minutes at which point HPLC analysis indicated that compound **7** was 0.9% by conversion (specification ≤1.7% by conversion). The batch was cooled to 45-50 °C over 5 hours 12 minutes (to aid cooling the nitrogen flow was increased once the batch was approximately 72 °C). At that temperature range, the batch was poorly stirring and on mixing, the batch temperature increased to 52 °C. It was >50 °C for ≤15 minutes. The batch was aged for 2 hours 2 minutes once initially <50 °C, then *n*-heptane (9.75 L, 15 vol, 99%) was added to the batch over 1 hour 56 minutes, maintaining the batch temperature at 45-50 °C. The heating was then discontinued and the batch cooled to 25 °C over 10 hours 32 minutes and then to approximately 20 °C over 20 minutes. The total time the batch was maintained ≤25 °C was 4 hours 50 minutes (2 hours 47 minutes at approximately 20 °C). The batch was filtered under suction via a 24-inch polypropylene filter funnel (fitted with a PTFE cloth) and the reactor rinsed with anisole/*n*-heptane (1.3 L, 4: 1) and the rinse transferred to the cake. The cake was then washed successively with two portions of n-heptane (1.3 L, 0.65 L). The total filtration time was 39 minutes. The batch (net wet weight 1004 g of KX2·391) was transferred to three glass trays and placed into a vacuum oven set at 50 °C and dried to constant weight over 96 hours 26 minutes.

**Preparation of 2-(5-(4-(2-morpholinoethoxy)phenyl)pyridin-2-yl)-N-benzylacetamide mesylate (Compound (I)·MSA)**

Compound (I) (520 g, 1.21 mol) was transferred to reactor 1 using acetone (41.6 vol, 80 vol, ACS) to facilitate the transfer. The batch was heated to 50 ± 5 °C over 33 minutes with dissolution occurring at 30 °C . The batch was clarified into a second reactor via a transfer pump fitted with an inline filter (Pall P/N 12077, 10 micron) and reheated from 46 °C to 50 ± 5 °C. Methanesulfonic acid (121.4 g, 1.05 equiv, 99% extra pure) was added to the pale yellow batch over 12 minutes and the heating then discontinued. After fourteen minutes, white solids were observed, which increased in number to give after 59 minutes a white suspension. The batch was in the range of 25 ± 5 °C after 7 hours 51 minutes and aged for a further 19 hours 21 minutes (10 hours 30 minutes at ≤27 °C). The batch was filtered under suction via a 24-inch polypropylene filter (PTFE cloth) and the reactor rinsed with acetone (2.0 L, clarified, ACS) and the rinse transferred to the cake. The cake was covered with a stainless steel cover and sucked dry under a flow of nitrogen. The total filtration time was 21 minutes. The batch (net wet weight 764 g) was transferred to three glass drying trays and dried in a vacuum oven to constant weight at 25 ± 5 °C over 21 hours 54 minutes (565 g, 89% of theory). A sample was removed for analysis and the batch maintained in vacuo at 25 ± 5 °C. The batch was then transferred to two 80-oz amber glass bottles (Teflon lined polypropylene closure), blanketed with argon and stored at -10 to -20 °C.

**Example 5: Dose Determination for Rising Single-Dose (RSD) and Rising Multiple-Dose (RMD) Study**

The starting dose was selected based on the results of the 28-day toxicity studies in dogs and rats. In these studies, dogs were found to be the more sensitive species. The minimal toxic level was 0.5 mg/kg/dose given by oral gavage BID. At this level, no clinical signs, changes in body weight or macroscopic findings were observed. The only finding considered potentially test article-related was minimally to mildly increased alanine aminotransferase. Many microscopic findings were noted in animals given 0.5 mg/kg/dose BID, but these were of lesser severity and affected fewer animals than the high dose group and were not associated with any clinical signs. Based on FDA guidance, the starting dose was calculated as one-tenth of the dose per meter squared that is severely toxic to 10% of rodents (STD10), i.e., 2 mg.

Three dose levels for the RSD part have been selected to determine the single-dose oral pharmacokinetics of compound (I) and to support or refine the dosing schedule for the RMD part of the study. Compound (I) dose levels selected for the RSD part of the study are 2, 5 and 10 mg (free base equivalents), administered as an oral solution.

The dose levels for the RMD part have been selected to expeditiously and cautiously reach the maximum tolerated dose for compound (I). In anticipation of toxicity at the higher dose levels, doses will be escalated by 40 mg increments after the 80 mg dose level. Twice a day dosing is supported by the half-life range of 5-8 hours as observed in oral dosing in dogs. Compound (I) dose levels selected for the RMD part of the study are 2, 5, 10, 20, 40, 80, 120, 160 mg or higher (in increments of 40 mg), depending on safety and tolerability, administered as an oral solution twice daily. Both the dose and the frequency of dosing may be modified depending on the single-dose pharmacokinetics of compound (I) and safety.

**Example 6: Rising Single-Dose (RSD) and Rising Multiple-Dose (RMD) Study**

To determine the single-dose pharmacokinetics (PK) of compound (I) a rising single-dose (RSD) study a rising single-dose study is conducted. Successive cohorts of 3 patients are enrolled into escalating dosing cohorts. Each patient enrolled receives a single oral dose of Compound (I) solution at 2, 5 or 10 mg (at least 2 hours of fasting is required prior to and post-dosing) and is observed for at least 7 days. If no toxicity develops (as defined below), the patients continue Compound (I) on a twice daily dosing schedule for 2 cycles in the RMD part of study.

To determine the maximum tolerated dose (MTD) of Compound (I) when administered as multiple oral solutions to patients with multiple malignancies, a rising multiple-dose study is conducted. The conduct of the RMD part of the study is as follows:

First cycle

Successive cohorts of 3 patients receive Compound (I) as an oral solution at 2, 5, 10, 20, 40, 80, 120, 160 mg or higher (in increments of 40 mg) twice daily (about 10 hours apart; at least 2 hours of fasting is required prior to and post-dosing) for 21 days, with an additional dose given AM on Day 22 for the convenience of prolonged PK sampling. Only the first cycle has 22 days of dosing. All subsequent cycles have 21 days of dosing.

The dosing schedule may be modified based on concurrent PK findings and safety concerns.

If a clinically significant Grade 2 toxicity (as defined below) occurs within a cohort during Part 1 or Part 2 of the study, unless the adverse event is clearly the result of disease progression, dose escalation is slowed down. The dose increment for the next dosing cohort is reduced.

If 1 patient of 3 develops dose-limiting toxicity (DLT, as defined below) then the cohort will be expanded from 3 to 6 patients. If only 1 of 6 patients or none in the expanded cohort develops DLT, dose escalation will proceed to the next level (refer to Section 6.3.1). If $\geq$ 2 of 3 or 6 patients in the expanded cohort develop DLT, then the treatment at that dose level will be stopped. Another cohort of 3 patients is given a reduced dose twice daily. The process continues until the MTD is determined. MTD is defined as the highest dose level at which no more than 1 of 6 patients develops DLT. An additional 10 patients are dosed at the MTD to better characterize the safety pharmacokinetics and biologic effects of compound (I). If at any time the number of patients experiencing DLTs is > 33%, dosing will be stopped. The dose just lower than this level is considered the MTD and 10 additional patients are enrolled at this level.

Second cycle

When patients in a cohort complete the washout period of the first cycle with no DLT, they proceed to the second cycle of 21 days of dosing and 7 days of washout. After two cycles of dosing, patients who can tolerate Compound (I) and do not have disease progression receive additional cycles of compound (I) (21 days on and 7 days off).

Toxicity is defined as an adverse event that has an attribution of possibly, probably or definitely being related to the investigational treatment.

Dose-limiting toxicity (DLT) is assessed during the first treatment cycle and will be defined as:

- Any non-hematological toxicity ≥ Grade 3 according to NCI Common Terminology Criteria for Adverse Events (CTCAE) version 3.0. Nausea, vomiting, diarrhea and electrolyte imbalances will be considered DLT only if these are ≥ Grade 3 despite adequate supportive care;
- Grade 4 neutropenia lasting > 5 days;
- Febrile neutropenia (defined as absolute neutrophil count [ANC] < 1.0 x 109/L and fever > 38.5o C) or documented grade >3 infection with ANC < 1.0 x 109/L;
- Grade 4 thrombocytopenia or thrombocytopenia requiring platelet transfusion;
- Delay of dosing in the second cycle for > 14 days due to toxicity.

**Study Duration**

The study described above includes a maximum of 18 scheduled visits over 14 weeks per patient starting from Screening through the completion of RSD and the first 2 cycles of RMD dosing. Fewer visits are required of cohorts only enrolling for the RMD part of the study. The visits are used for evaluation of study endpoints. The evaluation of 7 dose levels for 2 cycles of dosing, the study lasts about 12 months. After completion of the first 2 cycles of RMD, additional cycles of dosing are permitted in patients who tolerate compound (I) and have no disease progression.

**Study Endpoints**

Study endpoints are assessed as described below. Safety is assessed by adverse events and laboratory evaluations (i.e., hematology, serum chemistry, and urinalysis).

Pharmacokinetics are assessed as follows: plasma levels are analyzed for compound (I), utilizing a validated LC/MS/MS bioanalytical method. Urine is collected and analyzed to provide a semiquantitative assessment of elimination and metabolism. Biological effects are determined as follows: samples are taken to measure plasma levels of vascular endothelial growth factor (VEGF). In addition, levels of phospho-Src Tyr419 and trans-phosphorylation of selected substrates are assessed in peripheral blood mononuclear cells and in tumor biopsies. Analysis of biological effects in biopsiesis performed in the subset of patients who receive the MTD of compound (I) and who have accessible tumors. Safety parameters are evaluated at the end of the first cycle to allow for dose escalation. All the above parameters collected within the first 2 cycles are analyzed as study endpoints at the end of the study.

**Patient Selection**

The following are inclusiont criteria requirements for patient entry into the study:

1. Signed written informed consent
2. Adults over age 18 years of age
3. Confirmed advanced solid tumor or lymphoma that may be metastatic or unresectable and for which standard curative or palliative measures do not exist or are no longer effective; patients with treated brain or ocular metastases are also eligible
4. ECOG performance status of 0-2
5. Life expectancy of at least 14 weeks
6. Adequate bone marrow reserve as demonstrated by absolute neutrophil count (ANC)≥ 1.5 x 109/L, platelet count (PLT) ≥ 100 x 109/L or hemoglobin (Hgb) ≥ 10 g/L
7. Adequate liver function as demonstrated by serum bilirubin, alanine aminotransferase (ALT), aspartate transaminase (AST) and alkaline phosphatase (ALP) ≤ 2.5 x upper limit of normal (ULN)
8. Adequate renal function (serum creatinine ≤ 1.5 x ULN or calculated creatinine clearance > 60 ml/min)
9. Normal coagulation profile (PT/INR and aPTT within institutional normal limits) for those who give consent to tumor biopsy, within 1 week prior to the procedure.
10. Negative pregnancy test for females at Screening, preferably done within 1 week before Day 1 of dosing (not applicable to patients with bilateral oophorectomy and/or hysterectomy)
11. Willing to abstain from sexual activity or practice physical barrier contraception 28 days before Day 1 of dosing and 6 months after the last dose for the patient
12. Signed written informed consent for tumor biopsy for the additional 10 subjects that will be dosed at the MTD and who have accessible tumors.

The following are criteria for exclusion of patients from participating in the study:

1. Unresolved toxicity of higher than Grade 1 severity from previous anti-cancer treatment or investigational agents
2. Receiving or having received investigational agents or systemic anti-cancer agents within 14 days of Day 1 of dosing or 28 days for those agents with unknown elimination half-lives or half-lives of greater than 50 hours
3. Received extensive radiation therapy including sternum, pelvis, scapulae, vertebrae or skull, ≤ 4 weeks or low dose palliative radiation therapy limited to limbs ≤ 1 week prior to starting study drug, or who have not recovered from side effects of such therapy
4. Currently taking hormones (i.e., estrogen contraceptives, hormone replacement, anti-estrogen), anti-platelet agents or anti-coagulants, e.g. coumadin, except for those who are on prophylactic doses of anti-coagulants for indwelling venous catheters
5. Use of strong inhibitors or inducers of cytochrome P450 3A4 enzymes 2 weeks or 5 half-lives prior to Day 1 of dosing and during the study
6. Pregnant or breast-feeding
7. Major surgery within 4 weeks prior to Day 1 of dosing
8. Major surgery to the upper gastrointestinal tract, or inflammatory bowel disease, malabsorption syndrome or other medical condition that may interfere with oral absorption
9. Signs or symptoms of end organ failure, major chronic illnesses other than cancer, or any severe concomitant conditions which, in the opinion of the investigator, makes it undesirable for the subject to participate in the study or which could jeopardize compliance with the protocol
10. History of angina pectoris, coronary artery disease or cerebrovascular accident, transient ischemic attack or cardiac arrhythmia requiring medical therapy
11. Evidence of hepatitis B or C, human immunodeficiency (HIV) infection, coagulation disorders, or hemolytic conditions, e.g. sickle cell anemia

**Study Procedures**

The following procedures are conducted at scheduled patient visits.

Informed Consent and Complete Medical History

Informed consent and complete medical history is taken at Screening.

RSD Pharmacokinetic (PK) sampling

Blood samples are collected for pharmacokinetic analysis on:
Day 1 at 0 hr (prior to dosing), and at 1, 2, 3,4, 6, 9, 11, 24 (Day 2), 48 (Day 3), 96 hrs (Day 5) and 168 hrs (Day 8) post-dose (12 samples). Urine is collected for pharmacokinetic analysis on: Day 1 at 0 hr (prior to dosing), 0-6 hrs, 6-12 hrs, 12-24 hrs and 24-48 hrs (5 samples).
Plasma sample collection and preparation is as follows: blood samples (approximately 2.0 mL) are drawn from indwelling catheters or by direct venipuncture into a Vacutainer collection tube with potassium (K3) EDTA (size - 3 mL) as the anticoagulant and maintained on ice until centrifugation. Samples are centrifuged (~2,000 rpm at 4 °C for 10 minutes) within 30 minutes of collection. The plasma are immediately harvested using polypropylene transfer pipettes to split the plasma into two, approximately equal volumes (about 400 microliters) in the pre-labeled polypropylene transport tubes. The resulting plasma samples are capped and immediately placed in a freezer maintained at -70 °C.
Urine sample collection and preparation is as follows: urine is collected in urine bags over each specified time interval. The urine bags are stored at ~4 °C (refrigeration or on ice) until completion of the collection period. After collection, each urine sample is mixed well by shaking. At the end of each collection time interval, the volume is measured and documented on the CRF. For urinalysis, an aliquot of ~2 mL of urine is collected by transfer pipette and tested by dipstick. For PK measurement, urine aliquots of ~5 mL from each collection are transferred to each of two pre-labeled polypropylene transport tubes. The resulting urine samples are capped and immediately placed in a freezer maintained at -70 °C.

RMD Pharmacokinetic sampling

Blood samples are collected (as described above) for pharmacokinetic analysis on:
First cycle (Patients dosing at 2, 5 and 10 mg have 20 samples taken; patients dosing at >10 mg have 25 samples taken): Day 1 at 0 hr (prior to first AM dose), and at 1, 2, 3, 4, 6, 10 (prior to PM dose), 11 hrs (1 hr after PM dose); Day 2 at 0 hr (prior to AM dose), 1 hr after; Day 3 at 0 hr (prior to AM dose), 1 hr after; Day 8 at 0 hr (prior to AM dose), 1 hr after; Day 15 at 0 hr (prior to AM dose), 1 hr after; Day 22 at 0 hr (prior to AM dose, i.e., the last dose), and at 1, 2, 3,

4, 6, 9, 11, 24 hrs (Day 23), and 48 hrs (Day 24).

Patients in the first 3 cohorts (i.e., 2, 5 or 10 mg) who have undergone RSD have PK sampling on Day 1 as follows: Day 1 at 0 hr (prior to AM dose), and 11 hrs (1 hr after PM dose).

Second cycle (5 samples): Day 29 at 0 hr (prior to AM dose); Day 36 at 0 hr (prior to AM dose); Day 43 at 0 hr (prior to AM dose); Day 50; and Day 57. Patients who can tolerate further dosing and do not have disease progression, and who elect to continue dose-cycling after the first two cycles have PK sampling just before starting dose and at the end of dosing (2 hours after last dose) for each of the subsequent cycles.

Vital Signs (RSD and RMD)

Pulse rate, systolic and diastolic blood pressure, respiration, and body temperature are measured at: Screening; RSD and RMD: Day 1 at 0 hr (prior to dosing), 2 and 8 hrs post-dose; At each clinic visit.

Pulse rate is obtained with patient in resting state (seated for at least 5 minutes), pulse counted for 30 seconds, multiplied by 2 and recorded in beats per minute. Systolic/diastolic blood pressure is measured using a sphygmomanometer with the patient in resting state (seated upright for at least 5 minutes) using the same arm each time. Blood pressure is recorded in mm Hg. Respiration is obtained with patient in resting state (seated for at least 5 minutes), number of breaths are counted for 30 seconds, multiplied by 2 and recorded in breaths per minute. Temperature is obtained with patient in resting state (seated upright for at least 5 minutes) using an oral or aural thermometer.

Body Weight and Height

The patient's weight in kilograms and height in inches is obtained at: Screening; RMD: Days 1, 22, 29, 50 and 57.

Laboratory Evaluations for Safety

Blood for hematology, serum chemistry and urinalysis are collected at: Screening; RSD: Days 2, 3, and 8; RMD: Days 2, 3, 8, 15, 22, 29, 36, 43, 50 and 57. Patients on additional cycling after the first two cycles have laboratory evaluations for safety just prior to the starting dose and at the end of dosing for each of the subsequent cycle. Blood for PT/INR and aPTT is tested in those patients who are having tumor biopsies performed, within a week prior to the procedure.

Physical Exam

A complete physical examination will be conducted at: Screening. A partial physical examination is done to update any changes on: RSD: Days 1 and 8; RMD: Days 1, 22, 29, 50 and 57.

ECG Testing

12-Lead ECG and long Lead II is conducted at: Screening; RSD: Day 1 at 1 and 4 hrs post-dose and Day 8; RMD: Day 1 at 1 and 4 hrs post-dose, and Days 8, 22, 50 and 57. Patients in the first 3 cohorts (i.e., 2, 5 or 10 mg) who have undergone RSD will have ECG done only on Days 8, 22, 50 and 57 of the RMD part.

Pregnancy Testing

Blood for serum pregnancy testing is collected at Screening from female patients, preferably within 1 week before Day 1 of dosing (not applicable to patients with bilateral oophorectomy and/or hysterectomy).

Adverse Events

Adverse Events are monitored throughout the study. At each visit, the Investigator begins by querying for adverse events by asking each patient a general, non-directed question such as 'How have you been feeling since the last visit?' Directed questioning and examination is done as appropriate.

Concomitant Meds

At each visit the use of any concurrent medication, prescription or over the counter, is recorded along with the reason the medication was taken.

Assessment of Biological Effects

Blood is collected for measurement of VEGF in plasma; Src and selected substrate phosphorylation is assessed in PBMC in RMD on Days **1,** 22 and 50.

For patients who meet the criteria for biopsy, a pre-dose biopsy is performed within 4 weeks before Day 1 of dosing and the post-dose biopsy is performed between Days -20-22. Concurrent with the timing of the biopsy, blood is collected for the measurement of biological effects. In addition, blood is collected on Day 50 for biological effect analysis.

**Concomitant Therapy**

Patients are not allowed to use any chronic concomitant medications that are strong inhibitors or inducers of cytochrome P450 3A4 or coagulation. For example, the systemic use of the following CYP3A4 modulators is prohibited within 14 days or 5 half-lives (whichever is the longer time) prior to Day 1 of dosing and throughout the study:

CYP3A4 Inducers: barbiturates, carbamazepine, efavirenz, glucocorticoids, modafinil nevirapine, phenobarbital, phenytoin, rifampin, St. John's wort, troglitazone, oxcarbazepine, pioglitazone, rifabutin

CYP3A4 Inhibitors: amiodarone, aprepitant, chloramphenicol, cimetidine, clarithromycin, diethyl-dithiocarbamate, diltiazem, erythromycin, fluconazole, fluvoxamine, gestodene, grapefruit juice, imatinib, itraconazole, ketoconazole, mifepristone, nefazodone, norfloxacin, norfluoxetine, mibefradil, star fruit, verapamil, voriconazole.

Anti-coagulants used sparingly to maintain patency of intravenous ports or catheters is allowed. Concurrent use of hormones (i.e., estrogen contraceptives, hormone replacement, anti-estrogen) is prohibited (see below, Washout Periods).

**Washout Periods**

There is a washout or observation period of at least 7 days after single-dose administration in RSD. The washout period after the first cycle of the RMD part is 6 days. All other cycles have washout periods of 7 days between 2 consecutive cycles.

**Treatment Compliance**

Patients who are found to be inadvertently enrolled with significant deviation from the protocol-specified criteria are discontinued from the study. Patients are assessed for adherence to dosing schedule. They are instructed to complete a study calendar at home to track their dosing. At the scheduled weekly visits, patients bring this calendar together with all used and unused dosing bottles to the clinical site(s). These are checked by site personnel before patients are dispensed with a new supply of study drugs. Investigators ask patients at each return visit if they have used any concomitant medication since the previous visit, determine whether such use is a protocol violation, and record the data and conclusion.

**Study Medication**

Compound (I) is provided in this study as the mesylate salt of the free base N-benzyl-2-{5-[4-(2-morpholin-4-yl-ethoxy)-phenyl]-pyridin-2-yl}-acetamide. Clinical dosing is calculated as the weight of the free base in the solution. The compound (I) mesylate salt is a white crystalline powder with an empirical formula of $C_{26}H_{29}N_3O_3 \cdot HO_3SCH_3$ and a molecular weight of 527.63 Daltons. The molecular weight of the free base is 431.53 Daltons.

Compound (I) mesylate powder is supplied on a cohort-by-cohort basis in unit dose bottles containing different amounts of study drug corresponding to the dose: 2, 5, 10, 20, 40, 80, 120, 160 mg or higher (free base equivalents). Dose levels that are modified for safety are also be prepared as unit dose bottles and delivered to the clinical site(s). Upon dissolution, the resulting compound (I) mesylate solution is clear and is dispensed to patients in the unit dose bottles at concentrations ranging from 0.2 to 4.0 mg/mL (free base equivalents).

**Dosage and Dosage Regimen**

Compound (I) mesylate is administered orally according to the patient's dose cohort, i.e., RSD: 2, 5 or 10 mg; RMD: 2, 5, 10, 20, 40, 80, 120, 160 mg (free base equivalents), or higher, in increments of 40 mg. For the RMD part, compound (I) is administered twice daily (about 10 hours apart, given after at least 2 hours of fasting, and followed by 2 hours of fasting) for 21 days followed by 7 days of washout per cycle. The only exception is the first cycle where an

additional dose is given on Day 22 for the ease of prolonged PK sampling. There are 6 days of washout for this cycle. Patients who can tolerate the study drug and do not have disease progression may elect to receive additional cycles of dosing after the first two RMD cycles.

A fixed volume of sterile water is added to a unit dose bottle of compound (I) mesylate and shaken well (inverted approximately 10 times) until a clear liquid is obtained.

| Dose Level (mg, free base) | Amount of Compound (I) Powder in Bottle (mg, free base) | Volume of Water added to each Bottle (mL) | Resulting Concentration (mg/mL) | Volume of a Single Dose (mL) |
|---|---|---|---|---|
| 2 | 2 | 10 | 0.2 | 10 |
| 5 | 5 | 20 | 0.25 | 20 |
| 10 | 10 | 20 | 0.50 | 20 |
| 20 | 20 | 20 | 1.0 | 20 |
| 40 | 40 | 20 | 2.0 | 20 |
| 80 | 80 | 20 | 4.0 | 20 |
| 120 | 120 | 40 | 3.0 | 40 |
| 160 | 160 | 40 | 4.0 | 40 |

Compound (I) is taken after at least 2 hours of fasting. Drinking water is allowed at all times. Under site supervision, with the administration of the first dose, the patient administers the entire unit dose bottle to himself or herself. An aliquot of 20 mL sterile water is provided to rinse out the bottle and the contents are taken orally to chase down the initial dose. This process is repeated. No food is taken until 2 hours afterwards.

For RMD, a 7-day supply of compound (I) solutions is prepared and dispensed at the site pharmacy to the patients. Patients return every week on Days 8, 15, 22, 36, 43 and 50 to return used bottles. On Days 8, 15, 36 and 43, the patients obtain a new 7-day supply.

**Dose Modification**

**Slowing of Dose Escalation**

When Grade 2 toxicity occurs, dose escalation may be slowed. The dose level of the next cohort will have smaller increments, as follows:

| Dose Level When Grade 2 Toxicity occurs (mg) | Next Escalated Dose (mg) | Amount of compound (I) in Bottle (mg) | Volume of Water added to Each Bottle (mL) | Resulting Concentration (mg/mL) | Volume of a Single Dose (mL) |
|---|---|---|---|---|---|
| 2 | 3.5 | 3.5 | 10 | 0.35 | 10 |
| 5 | 7.5 | 7.5 | 20 | 0.375 | 20 |
| 10 | 15 | 15 | 20 | 0.75 | 20 |
| 20 | 30 | 30 | 20 | 1.5 | 20 |
| 40 | 60 | 60 | 20 | 3.0 | 20 |
| 60 | 70 | 70 | 20 | 3.5 | 20 |
| 80 | 100 | 100 | 40 | 2.5 | 40 |
| 100 | 110 | 110 | 40 | 2.75 | 40 |
| 120 | 140 | 140 | 40 | 3.5 | 40 |
| 140 | 150 | 150 | 40 | 3.75 | 40 |

(continued)

| Dose Level | Next | Amount | Volume | Resulting | Volume |
|---|---|---|---|---|---|
| When Grade 2 Toxicity occurs (mg) | Escalated Dose (mg) | of compound (I) in Bottle (mg) | of Water added to Each Bottle (mL) | Concentration (mg/mL) | of a Single Dose (mL) |
| 160 | 180 | 180 | 40 | 4.5 | 40 |

If no more Grade 2 or higher toxicity occurs at the escalated dose level, the initial dose escalation schedule may be resumed.

**Dose Modification at DLT**

When DLT occurs in ≥ 2 of 3 or 6 patients in a cohort, dose escalation is stopped. Further dosing at a reduced dose in the next cohort is as follows:

| Dose Level When DLT Occurs (mg) | Reduced Dose (mg) | Amount of Compound (I) in Bottle (mg) | Volume of Water Added to Each Bottle (mL) | Resulting Concentration (mg/mL) | Volume of a Single Dose (mL) |
|---|---|---|---|---|---|
| 5 | 2.5 | 2.5 | 10 | 0.25 | 10 |
| 10 | 7.5 | 7.5 | 20 | 0.375 | 20 |
| 20 | 15 | 15 | 20 | 0.75 | 20 |
| 40 | 30 | 30 | 20 | 1.5 | 20 |
| 80 | 60 | 60 | 20 | 3.0 | 20 |
| 120 | 100 | 100 | 40 | 2.5 | 40 |
| 160 | 140 | 140 | 40 | 3.5 | 40 |

**Pharmacokinetic Analysis**

Noncompartmental pharmacokinetic analysis is performed on individual plasma Compound (I) concentration-time data using WinNonlin Professional (Pharsight Corp., Mountain View, CA Version 4.1) or other suitable software. When data from individual patients cannot be analyzed, mean plasma compound (I) concentration-time data is used to calculate pharmacokinetic parameters. The following pharmacokinetic parameters are calculated from the plasma concentrations: Cmax (Maximum serum concentration), tmax (Time to reach maximum concentration), $AUC_T$ (Area under concentration-time curve from time zero to last measurable concentration (CT) at time T, $AUC_{0-\infty}$ (Area under concentration-time curve from time zero to infinity), t½ (Terminal phase half-life), Ae (Amount of drug excreted in the urine). Additional parameters deemed appropriate for description and interpretation of the pharmacokinetic data are determined at the discretion of the study pharmacokineticist.

**Assessment of biological effects**

Plasma levels of vascular endothelial growth factor (VEGF) are measured by ELISA. Levels of phospho-Src Tyr419 and trans-phosphorylation of selected substrates are determined in peripheral blood mononuclear cells. The objective of performing tumor biopsies before and after treatment at MTD is to determine the biological effects of compound (I) in inhibiting phosphorylation of Src kinase that may be involved in tumor proliferation. Paired biopsies are performed in the 10 patients in the expansion cohort at MTD. Tissues are split in half with one portion being evaluated by routine pathology and the other half evaluated for levels of phospho-Src Tyr419 and trans-phosphorylation of selected substrates.

**Assessment of Disease Progression**

For measurable disease, tumor response is assessed according to the RECIST criteria (Therasse, P., et. al., New Guidelines to Evaluate the Response to Treatment in Solid Tumors. J Nat Can Inst. 2000, 92(3), p. 205-216). Measurements is obtained at baseline and after every other cycle (2 cycles). All responding patients (Complete Responders and Partial Responders) must have their response confirmed 4 weeks after the first documentation of response using the same method of measurement as the baseline measurement. In patients with non-measurable disease, response is assessed as clinically indicated (tumor markers, radiographic measurements, ultrasound, etc.) When bone metastases are the only site of disease, the WHO Criteria for Assessment of Disease Response in Bone is used to assess response. Progression of other non-measurable disease is defined as a 25% rise in tumor markers on 2 successive monthly determinations or significant radiographic progression of disease. Reassessment of tumor response is done by the same methods used to establish baseline tumor measurements. Assessment of tumor response is as follows:

Target Lesions

- Complete response (CR): disappearance of all target lesions
- Partial response (PR): decrease of at least 30% in the sum of the longest diameter (LD) of target lesions, taking as reference the baseline sum LD
- Progressive disease: increase of at least 20% in the sum of the LD of target lesions, taking as reference the smallest sum LD recorded since initiation of treatment, or the appearance of one or more new lesions
- Stable disease: neither sufficient shrinkage to qualify for partial response nor sufficient increase to qualify for progressive disease, taking as reference the smallest sum LD since initiation of treatment

Non-target Lesions

- Complete response: disappearance of all non-target lesions
- Incomplete response/stable disease: persistence of one or more non-target lesions
- Progressive disease: appearance of one or more new lesions or unequivocal progression of existing non-target lesions, or both

A clear progression of only non-target lesions is exceptional. However, if the investigator believes progression of only non-target lesions has occurred, this progression is verified through a confirmatory CT scan 4 weeks later. Tumor responses ≥ PR are confirmed 4 weeks later using the same method of measurement as baseline assessment.

The overall clinical response for all possible combinations of tumor responses in target and non-target lesions is determined according to the following table:

Overall Clinical Response

| Response in Target Lesions | Response in Non-target Lesions | New Lesions | Overall Clinical Response |
|---|---|---|---|
| CR | CR | | |
| CR | IR/SD | | |
| PR | Any except PD | | |
| SD | Any except PD | | |
| PD | Any | | |
| Any | PD | | |
| Any | Any | | |
| CR = complete response; IR = incomplete response PD = progressive disease; PR = partial. response; SD = stable disease. | | | |

**Example 7: Cell Growth Inhibition**

The drug concentration required to block net cell growth by 50% relative to a control sample is measured as the $GI_{50}$. The $GI_{50}$s for compound (I) was assayed as described herein.

The HT29 cell line is a NCI standard human colon carcinoma cell line. HT-29 cells were obtained from ATCC at passage 125 and were used for inhibition studies between passage 126 -151. HT29 cells were routinely cultured in

McCoy's 5A medium supplemented with Fetal Bovine Serum (1.5% v/v) and L-glutamine (2 mM).

The c-Src 3T3 is a mouse fibroblast NIH 3T3 normal cell line that has been transfected with a point-mutant of human c-Src wherein tyrosine 527 has been converted to a phenylalanine. This mutation results in "constitutively active" c-Src because phosphorylation on tyrosine 527 results in auto-inhibition of Src by having it fold back on its own SH2 domain. With a Phe there, this phosphorylation can't occur and therefore auto-inhibition can't occur. Thus, the always fully active mutant Src then converts the normal mouse fibroblasts into rapidly growing tumor cells. Since the hyperactive Src is the main factor driving growth in these cells (particularly when cultured under low growth serum conditions), compounds active in blocking this growth are thought to work by blocking Src signaling (*e.g.* as a direct Src kinase inhibitor or as an inhibitor acting somewhere else in the Src signaling cascade). The cells were routinely cultured in DMEM supplemented with Fetal Bovine Serum (2.0% v/V), L-glutamine (2 mM) and Sodium Pyruvate (1 mM).

In the BrdU Assay for cell growth inhibition, quantitation of cell proliferation was based on the measurement of BrdU incorporation during DNA synthesis. The Cell Proliferation ELISA BrdU assay kit (colorimetric) was obtained from Roche Applied Science and performed as per vendor instructions.

Growth inhibition was expressed as a $GI_{50}$ where the $GI_{50}$ is the sample dose that inhibits 50% of cell growth. The growth inhibition (GI) is determined from the formula GI= ($T_0$-$T_n$ x100/$T_0$-$CON_n$) where $T_0$ is the BrdU growth of untreated cells at time "0", $T_n$ is the BrdU growth of treated cells at day "n" and $CON_n$ is the control BrdU growth of control cells at day "n". The $GI_{50}$ was extrapolated and the data plotted using XL-Fit 4.0 software.

Actively growing cultures were trypsinized and cells were resuspended in 190μL of appropriate culture medium supplemented with 1.05% FBS in each well of a 96-well culture plate (1000 HT-29 cells; 2500 c-Src 3T3 cells). For 96 well culture plate experiments, c-Src 3T3 medium was supplemented with 10 mM HEPES buffer. HT-29 cells were seeded in standard tissue culture 96-well plates and c-Src 3T3 cells were seeded in 96-well plates coated with Poly-D-lysine (BIOCOAT™). To increase $CO_2$ diffusion, c-Src 3T3 96-well plates were incubated with their lids raised by ~2 mm using sterile rubber caps.

Seeded 96 well plates were allowed to attach overnight for 18-24 hours, either at 37 °C and 5% $CO_2$ for HT-29 or at 37 °C and 10% $CO_2$ for c-Src 3T3. Approx 18-24 hours after seeding, the initial growth of cells ($T_0$) was determined for untreated cells using the BrdU assay. Samples were reconstituted in DMSO at 20 mM and intermediate dilutions made using DMEM containing 10% FBS. The final assay concentrations were 1.5% for FBS and 0.05% for DMSO. Samples were added as 10 μL aliquots in triplicate and plates were incubated as above for ~72 hours. Negative (vehicle) and positive controls (*e.g.*, AZ28 (KX2-328)) were included. Plates were assayed for BrdU and the data analyzed as above for $GI_{50}$.

The results are shown in the table below. In this table, the data is listed as Growth % of Control, such that a lower number at an indicated concentration indicates a greater potency of the compound in blocking growth of that tumor cell line. All compounds were initially prepared as 20 mM DMSO stock solutions and then diluted into buffer for the *in vitro* tumor growth assays. NG means no cell growth beyond the control and T means the number of cells in the drug treated wells was less than in the control (*i.e.* net cell loss). NT indicates that the test was not performed. Compound AZ28 (KX2-328) is an ATP-competitive tyrosine kinase inhibitor, as described in Plé et al., J. Med. Chem, 47:871-887 (2004).

As shown in the table below, $GI_{50}$s were obtained for compound (I) in other cell lines. These GI50's were determined using the standard tumor growth inhibition assays, similar to that described in detail for the HT29 cell line above, and the following cell lines: colon tumor cell lines KM12, lung cancer cell line H460 and lung cancer cell lineA549 (all are NCI standard tumor cell lines).

| | | HT-29 Growth, % of Control Mean, n=3 | | | | c-Src 3T3 Growth, % of Control Mean, n=3 | | |
|---|---|---|---|---|---|---|---|---|
| compound | | 5 uM | 500 nM | 50 nM | $GI_{50}$ | 10 uM | 1.0 uM | 100 nM |
| KX2-328 | | T | 10.0 | 73.0 | 99 nM (c-Src 3T3), 794 nM (HT29) | T | T | 13.0 |
| (compound I) | | | | | 13 nM (c-Src 3T3); 23 nM (HT-29) | | | |
| NG =No growth, total growth inhibition; T = Cytotoxic Effect on Cells, negative growth; NT= Not tested | | | | | | | | |

The table below shows compound (I) inhibition of Src driven tumor cell growth in comparison to the ATP competitive Src inhibitors currently in clinical trials.

| Compound | c-Src527F/NIH3T3 $GI_{50}$ (nM) | HT29 (Colon) $GI_{50}$ (nM) |
|---|---|---|
| Compound (I) | 23 | 25 |
| KX2-328 | 87 | 647 |
| Dasatinib | 3 | 20 |
| SKI-606 | 208 | 173 |
| AZD0530 | 203 | 330 |

The table below shows compound (I) inhibition in brain tumor cell lines. These $GI_{50}$s were determined using standard tumor growth inhibition assays, similar to those described in detail in this Example 7.

**GI50 of compound (I) and Dasatinib in brain tumor cell lines:**

| Cell Line | Compound (I) IC50 | Dasatinib IC50 | Organism | Disease | Morphology | Tumorigenic |
|---|---|---|---|---|---|---|
| **Daoy** | 13.6 nM | 2927 nM | Human | Desmoplastic cerebellar medulloblastoma | Polygonal | Yes |
| **SK-N-MC** | 5.8 nM | 5114 nM | Human | Neuroepithelioma | Epithelial | Yes |
| **SW1088** | 76.1 nM | 897.3 nM | Human | Astrocytoma | Fibroblast | Yes |
| **LN-18** | 14.5 nM | 565.3 nM | Human | Glioblastoma; glioma | Epithelial | Yes |
| **SK-N-FI** | 1.7 nM | 12.6 nM | Human | Neuroblastoma | Epithelial | Yes |
| **U87** | 33.1 nM | 1586 nM | Human | Glioblastoma; astrocytoma | Epithelial | Yes |
| **GL261** | 13.7 nM | 17.7 nM | Mouse | Glioblastoma | Epithelial | Yes |

The table below shows compound (I) inhibition in renal tumor cell lines. These $GI_{50}$s were determined using standard tumor growth inhibition assays, similar to those described in detail in the Example section.

**GI50 of compound (I) and Dasatinib in renal tumor cell lines:**

| Cell Line | Compound (I) GI50 | Dasatinib GI50 | Organism | Disease | Morphology | Tumorigenic |
|---|---|---|---|---|---|---|
| **769-P** | 45.0 nM | 46.3 nM | Human | Renal cell adenocarcinoma | Epithelial | Yes |
| **786-O** | 378.4 nM | 2014 nM | Human | Renal cell adenocarcinoma | Epithelial | Yes |
| **Caki-2** | 39.2 nM | 14.2 nM | Human | Clear cell carcinoma | Epithelial | Yes |
| **ACHN** | 33.2 nM | 21.1 nM | Human | Renal cell adenocarcinoma | Epithelial | Yes |

The table below shows a summary of the results of compound (I) inhibition in five hepatocellular carcinoma cell lines. The table below shows the $IC_{50}$s and $IC_{80}$s of the mesylate salt of compound (I) and Dasatinib in hepatocellular

carcinoma cell lines (8.0 x 103 cells/wells, 1.5% FBS) @ 78Hr; results from normalized response data:

**GI50 of Compound (I) and Dasatinib in hepatocellular carcinoma cell lines:**

| | Compound (I) MSA | | Dasatinib | |
|---|---|---|---|---|
| Cell Line | $IC_{50}$ (nM) | $IC_{80}$ (nM) | $IC_{50}$ (nM) | $IC_{80}$ (nM) |
| HuH7 | 9 | 23 | 1972 | 7135 |
| WRL-68 | 15 | 25 | 5650 | 45,580 |
| PLC/PRF/5 | 13 | 24 | 15 | >50,000 |
| Hep 3B | 26 | 88 | 86 | >50,000 |
| Hep G2 | 60 | 3658 | NA | NA |

Samples of the test compounds were formulated in 100% DMSO to obtain 20 mM stock solutions; stored @ 4 °C. The $IC_{50}$s and $IC_{80}$s were determined as described below. Huh7, WRL-68, PLC/PRF/5, Hep 3B, and Hep G2 human cancer lines were routinely cultured and maintained in a basal medium containing 2% FBS @ 37 °C, 5% $CO_2$. Cells were seeded @ 4.0 x $10^3$/190 μl and 8.0 x $10^3$/190 μl per well of a 96-well plate. The assay medium was basal medium / 1.5% FBS. Cells were cultured overnight in 96-well plates at 37 °C, 5% $CO_2$ prior to the mesylate salt of compound (I) (Compound (I).MSA) and Dasatinib addition. The test article dilutions were prepared as follows: 20 mM stock solution samples were diluted serially in basal medium/1.5 % FBS using 1:3 dilutions, yielding 20x concentrations; 131 μM to 0.24 nM range. 10 μL of 20x dilutions were added to the appropriate wells (n = 3) containing 190 μL cancer cell line; 6561 nM to 0.012 nM range of final concentrations. Vehicle control contained cells, no sample. Medium control contained cells, no sample, 0.03% DMSO (highest DMSO concentration present in samples). The treated cells were incubated for 72 hours at 37 ° C, 5% $CO_2$. On day 3, 10 μL MTT (5mg/mL) were added to each well. Cells were incubated in the presence of MTT for 4 hours @ 37 °C, 5% $CO_2$. 90 μL 10% SDS(+HCl) was added to each well to lyse cells and solubilize formazan. Cells were then incubated overnight @ 37 °, 5% $CO_2$. $OD_{570}$ measurements were taken using BioTek Synergy HT multiplatform microplate reader. Growth inhibition curves $IC_{50}$s and $IC_{80}$s were determined using GraphPad Prism 5 statistical software.

**Example 8: Inhibition of Isolated Kinases**

It is believed that the conformation of Src outside cells vs. inside cells is markedly different, because inside cells, Src is embedded in multiprotein signaling complexes. Thus, because the peptide substrate binding site is not well formed in isolated Src (as shown by Src x-ray structures), it is believed that the activity against isolated Src for a peptide substrate binding inhibitor would be weak. Binding to this site will require the inhibitor to capture the very small percentage of total Src protein in the isolated enzyme assay that is in the same conformation that exists inside cells. This requires a large excess of the inhibitor to drain significant amounts of the enzyme from the catalytic cycle in the assay.

However, inside cells this large inhibitor excess is not needed because the SH2 & SH3 domain binding proteins have already shifted the Src conformation so that the peptide substrate binding site is fully formed. Now, low concentrations of the inhibitor can remove the enzyme from the catalytic cycle since all of the enzyme is in the tight binding conformation.

KX2-328 is AstraZeneca's published ATP-competitive Src inhibitor (AZ28) and is used as a positive control in many of the experiments described herein. KX2-328 is the compound having the structure:

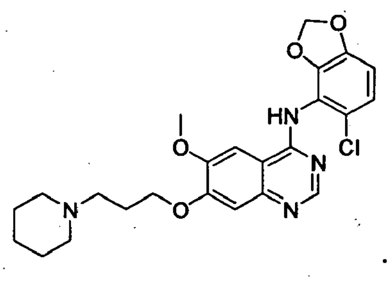

Note that compound (I) has weak activity against isolated kinases because the peptide binding site is not well formed outside of cells, but have very potent activity inside whole cells. Without wishing to be bound by theory, it is thought that the difference in activity is attributed to the fact that the peptide binding site is now fully formed in cells due to the allosteric effects of the binding protein partners in the multi-protein signaling complexes, relative to isolated kinase assays.

The table below illustrates percent activity of isolated kinases in the presence of the AstraZeneca ATP-competitive inhibitor (KX2-328, AZ28) or compound (I) relative to control (untreated) isolated kinases.

| **Target** | **AZ28 @ 10 μM** | **Compound (I) @ 10 μM** |
|---|---|---|
| Abl(h) | **1** | **120** |
| CHK1(h) | NT | **105** |
| EGFR(h) | **3** | **134** |
| FGFR2(h) | **94** | **94** |
| Fyn(h) | **2** | **85** |
| IGF-1R(h) | **110** | **101** |
| IR(h) | **125** | **112** |
| Lck(h) | **1** | **109** |
| Lyn(h) | **0** | **113** |
| MAPK2(h) | **105** | **112** |
| PDGFRβ(h) | **98** | **110** |
| PKCα(h) | **111** | **111** |
| Pyk2(h) | **43** | **97** |
| Yes(h) | **1** | **92** |
| ZAP-70(h) | **97** | **108** |
| PI3 kinase | **99** | **100** |

The AstraZeneca ATP competitive inhibitor shows the typical off target kinase inhibition activity for ATP-competitive inhibitors, poor selectivity as evidenced by strong inhibition of Abl, EGFRTK, Fyn, Lck, Lyn & Yes. In contrast, poor inhibition of these off-target kinases is seen with compound (I).

However, compound (I) is a more potent inhibitor of Src -driven cell growth, assayed as described in the example above. In the c-Src/NIH-3T3 engineered cell line, the $GI_{50}$ for AZ28 is 99 nM, vs. 13 nm for compound (I), and in the NCI human colon cancer cell line HT29, the $GI_{50}$ for AZ28 is 794 nM, vs. 23 nm for compound (I).

In separate examples, titration data indicate that AZ28 is a potent inhibitor of isolated Src ($IC_{50}$=8 nM). The titration data with FAK shows that AZ28 is at least ca. 100 X less potent against isolated FAK (IC50 >500 nM). Whereas, titration data indicate that compound (I) is a less potent inhibitor of isolated Src (IC50=46 μM). The titration data with FAK shows that compound (I) is similarly potent against isolated FAK ($IC_{50}$ >48 μM).

Note that AZ28 is 10-100 X less potent against cell growth than against isolated Src. This is typical of ATP competitive inhibitors since the concentration of competing ATP is much higher in whole cells than in the isolated enzyme assays. Compound I exhibited an $IC_{50}$ = 46 mM against cSrc.

**Example 9: Effect on Intracellular Phosphorylation Levels**

HT29 (colon cancer) and c-Src527F/NIH-3T3 (Src transformed) cell lines were treated with compound (I) or with AstraZeneca's ATP competitive Src inhibitor AZ28. AZ28 serves as a positive comparator to show what a validated Src inhibitor should do in these assays. After treatment with compound, cells were lysed, subjected to PAGE and probed with a battery of antibodies. The antibodies were selected to determine whether compounds caused changes in phosphorylation of known Src substrates. In addition, off-target protein phosphorylation was also investigated. Further, induction of apoptosis was evaluated *via* Caspase 3 cleavage. Multiple doses of each compound were tested because the trends in response to increasing drug concentration are the most reliable indicator of activity.

A dose response curve for compound (I) was generated using the GI50 for this compound in each of the two cell lines as the 1X concentration. Three additional doses 0.2X, 5X & 25X multiples the GI50's were also tested in addition

to a no drug control "C". The same range of multiples of Figure 1, the expected dose response for Src-Y416 autophosphorylation was obtained in both cell lines, and for both compounds. This data indicates that compound (I) is a Src inhibitor inside cells.

Figure 2 shows phosphorylation of FAK Tyr 925, a known Src transphorylation substrate within cells. Compound (I) and AZ28 inhibited Src trans-phosphorylation. This data indicates that compound (I) is a Src inhibitor inside cells.

Figure 3 shows phosphorylation of Shc Y239/240, a known Src transphorylation substrate within cells. Compound (I) and AZ28 inhibited Src trans-phosphorylation. This data indicates that compound (I) is a Src inhibitor inside cells.

Figure 4 shows phosphorylation of Paxillin Y-31, a known Src transphorylation substrate within cells. Compound (I) and AZ28 inhibited Src trans-phosphorylation. This data indicates that compound (I) is a Src inhibitor inside cells. Note: paxillin Y-31 was not detected in HT29 cells with or without added drug.

Cleavage of Caspase-3 is a good measure of induction of apoptosis. It is known that AZ28 is not effective in inducing apoptosis in HT29 (colon cancer) and c-Src527F/NIH-3T3 (Src transformed) cell lines. In contrast, as shown in Figure 5, compound (I) is very effective in inducing apoptosis.

Since Src activity is very high in both HT29 (colon cancer) and c-Src527F/NIH-3T3 (Src transformed) cell lines, one would expect to see a reduction in the total phosphotyrosine levels when Src activity is inhibited. Figure 6 indicates that this is true for both AZ28 and compound (I). This data indicates that compound (I) is a Src inhibitor inside cells.

PDGF receptor tyrosine kinase autophosphorylates on Y572/574. This is thought not to be a direct Src substrate in cells. It is known that AZ28 is not a potent inhibitor of isolated PDGF receptor tyrosine kinase (see the table in Example 8). Nevertheless, a dose response reduction in PDGF receptor autophosphorylation is seen with AZ28, as shown in Figure 7. This suggests that this is an indirect effect. Some effect is seen with compound (I), however it is somewhat less potent. Thus, compound (I) is less active than AZ28 against indirect PDGF autophoshorylation inhibition. PDGF receptor tyrosine kinase Y572/574 was not detected in HT29 cells with no drug added (as well as with drug added).

FAK Y397 is mainly a FAK autophosphorylation site and only a poor Src transphorylation site. AZ28 is not a potent FAK inhibitor (see isolated enzyme data in Example 8). Nevertheless, some inhibition of FAK autophosphorylation in c-Src527F/NIH3T3 cells with AZ28 is shown in Figure 8. However, no inhibition of FAK autophosphorylation in c-Src527F/NIH3T3 cells is seen with compound (I). The opposite is true in the NCI human colon cancer cell line HT29.

The isolated enzyme data shown in Example 8 demonstrated that AZ28 is a potent EGFR tyrosine kinase inhibitor. In agreement with this the tumor cell data in Figure 9 shows that AZ28 potently inhibits EGFR tyrosine kinase autophosphorylation. This site is not a direct Src phosphorylation site. The tumor cell data in Figure 9 also shows that compound (I) is less active against the off target autophosphorylation of EGFRTK.

The inhibition of autophosphorylation correlates with the $GI_{50}$'s of compound (I). Figures 10A and 10B show inhibition of Src autophophorylation (Y416) by compound (I) as compared to AZ28 in c-Src527F/NIH-3T3 cells and in HT-29 cells. The inhibition of transphosphorylation also correlates with the $GI_{50}$'s of compound (I). Figures 10C and 10D show inhibition of Src transphosphorylation of Shc Y239/240 by compound (I) as compared to Az28 in c-Src527F/NIH-3T3 cells and in HT-29 cells.

Compound (I) shows very high protein tyrosine kinase selectivity in whole cell assays. For example, Figure 11 shows compound (I) selectivity for protein tyrosine kinases in comparison to Dasatinib.

**Example 10: Protection Against Noise-Induced Hearing Loss**

Chinchillas (N=6) are used in studies of noise-induced hearing loss. The animals' hearing sensitivity is measured using standard electrophysical techniques before the experimental manipulation. In particular, hearing thresholds are measured through evoked potentials from recording electrodes chronically implanted in the inferior colliculus, following standard laboratory procedures. Animals are anesthetized, the auditory bullae are opened, and the left and right cochleas are visualized. The round window leading to the scala tympani of the cochlea was used as the access point for drug application. Animals are treated with Compound (I) or KX2-328 (a non-ATP competitive inhibitor from AstraZeneca, KX2-238), emulsified in DMSO, in 1000mM of saline solution, which is placed on the round window of one ear.

A control solution of 3 mM DMSO in 1000 mM of saline solution is placed on the round window of the other ear. The solution is allowed to set on the round window for 30 minutes, then the auditory bullae are closed. Subsequently, the animals are exposed to 4 kHz band noise at 105 dB SPL for four hours. Following the noise exposure, the animals' hearing is tested at day 1, day 7, and day 21 to determine evoked potential threshold shifts. Permanent threshold shift are assessed at day 21.

**Example 11: Protection Against Cisplatin-Induced Hearing Loss**

The effects of high level noise and ototoxic drugs, such as cisplatin or the class of aminoglycosides, share several common features in the inner ear. First, the noise and/or drugs alter the free radical/antioxidant levels in the cochlea (inner ear). The increase in free radicals has been shown to be a causative factor in the apoptotic death of the

sensory cells. Guinea pigs (e.g., N=7) are used in studies of cisplatin-induced hearing loss. The animals' hearing sensitivity is measured using standard electrophysical techniques before the experimental manipulation. In particular, hearing thresholds are measured through evoked potentials from recording electrodes chronically implanted in the inferior colliculus, following standard laboratory procedures. Animals are anesthetized and treated with cisplatin. Subsequently, the animals' hearing is tested to determine evoked potential threshold shifts.

**Example 12: Effect on Osteoclast Formation.**

To determine the effect of compound (I) on osteoclast formation, the compound is added to osteoclast precursors derived from spleen cells. For the generation of spleen-derived osteoclasts, spleen cells comprising osteoclast precursors are treated with Rapamycin, compound (I), or KX2-328 (AstraZeneca compound) for 5 days in the presence of receptor activator of nuclear factor-κB ligand (RANKL) and macrophage colony-stimulating factor (M-CSF). In *in vitro* murine or human osteoclast models, soluble RANKL enables osteoclast precursors to differentiate in the presence of M-CSF (Quinn, et al.; 1998, Endocrinology, 139,4424-4427; Jimi, et al.; 1999, J. Immunol., 163, 434-442). The untreated control cells were incubated in the presence of RANKL and M-CSF alone. Rapamycin is used as a positive control for the inhibition of osteoclast formation.

**Example 13: Effect on Osteoclast Survival.**

To determine the effect of compound (I) on osteoclast survival, osteoclasts are treated with Rapamycin, compound (I), or KX2-328 for 48 hours in the presence of RANKL and M-CSF. The untreated, control cells are incubated in the presence of RANKL and M-CSF alone. Rapamycin is used as a positive control for the inhibition of osteoclast survival.

**Example 14: Effect on Bone Resorption *in vitro.***

To determine the effects of compound (I) on osteoclast formation on bone slices, the bone slices are treated with Rapamycin, compound (I), or KX2-328 in increasing concentrations e.g., 0.1 nM, 1 nM, or 10 nM. The number of osteoclasts on the bone slices are counted.

During the resorption of bone, osteoclasts form resorption pits. To determine the effects of compound (I) on resorption pit formation on bone slices, the bone slices are treated with Rapamycin, compound (I), or KX2-328 as described above. The number of resorption pits on the bone slices are determined.

Bone slices are treated as indicated above and then stained with TRAP. The number of TRAP-positive osteoclasts is determined.

Bone slices are treated as indicated above and then stained with Toluidine Blue to reveal resorption pits, which are indicators of osteoclast-mediated resorption of bone.

**Example 15: Effect on Osteoblasts.**

The enzyme alkaline phosphatase has been used as an indicator of osteoblast activity, as it is involved in making phosphate available for calcification of bone. To determine the effects of compound (I) on osteoblast activity, osteoblasts are treated with compound (I) or KX2-328 at increasing concentrations and alkaline phosphatase expression is determined (nM alkaline phosphatase /μg protein/min. As controls, osteoblasts are treated with medium alone, dimethyl sulfoxide (DMSO), or bone morphogenic protein-2 (BMP2). BMPs, defined as osteoinductive by their ability to induce osteogenesis when implanted in extraskeletal sites, are thought to mediate the transformation of undifferentiated mesenchymal cells into bone-producing osteoblasts.

To determine the effects of compound (I) on osteoblast activity and protein expression, osteoblasts are treated with medium, DMSO, BMP2, compound (I) or KX2-328 as indicated above. The protein concentration in cell lysates is determined.

**Example 16: Effect on Obesity**

The following example illustrates that compound (I) could be used to treat obesity. Compound (I) is tested using a method described previously (Minet-Ringuet, et al.; 2006, Psychopharmacology, Epub ahead of print, incorporated herein by reference). Thirty male Sprague-Dawley rats initially weighing 175-200 g are housed in individual Plexiglas cages with an artificial 12:12-h light-dark cycle (lights on at 08:00 h) in a room maintained at 24±1°C and 55±5% humidity. Food and water are available ad libitum throughout. All rats are fed with a medium fat diet (metabolizable energy 17.50 kJ/g) composed of 140 g/kg of whole milk protein, 538.1 g/kg of cornstarch, 87.6 g/kg of sucrose, and 137 g/kg of soya bean oil, and this diet is supplemented with minerals and vitamins (mineral salts 35 g/kg, vitamins 10 g/kg,

cellulose 50 g/kg, and choline 2.3 g/kg). This food, named P14-L, which resembles the usual human diet (14% proteins, 31% lipids, and 54% carbohydrates) is prepared in the laboratory in the form of a powder.

Several doses of compound (I) are tested: 0.01, 0.1, 0.5, and 2 mg/kg, in addition to the control solution. The compound is solubilized in water and then incorporated into the diet. The basal food intake is recorded during the adaptation period and used to determine the daily quantity of the compound of the instant invention incorporated into food. The compound is mixed into the food in the laboratory. After 1 week of adaptation to the laboratory conditions, the rats are separated into five groups (n=6 per group) with homogenous weight and receive the compound of the instant invention in their food for 6 weeks. Weight is recorded three times per week. Body composition is measured at the end of the study by dissection and by weighing the main organs and tissues. Briefly, rats are deeply anesthetized by an intraperitoneal injection of an overdose of anesthetic (sodium pentobarbital 48 mg/kg) and heparinized (100 U heparin/100 g body weight). They are bled (to avoid coagulation in tissues) by sectioning the vena cava and abdominal aorta before removal and weighing of the main fresh organs (liver, spleen, kidneys, and pancreas) and tissues (perirenal and scapular brown adipose tissue, epididymal, retroperitoneal, visceral, and subcutaneous white adipose tissues (WATs), and carcass defined by muscles and skeleton).

**Example 17: Effect on Insulin-Induced GLUT4 Translocation in 3T3-L1 Adipocytes.**

The following example illustrates that compound (I) could be used to treat diabetes. Compound (I) is tested using a method described previously (Nakashima, et al.; 2000, J. Biol. Chem., 275, 12889-12895). Either control IgG, or the compound of the instant invention is injected into the nucleus of differentiated 3T3-L1 adipocytes on coverslips. Glutathione *S*-transferase fusion proteins are each coinjected with 5 mg/ml sheep IgG for detection purposes. Prior to staining, the cells are allowed to recover for a period of 1 h. Cells are starved for 2 hr in serum-free medium, stimulated with or without insulin (0.5 nM or 17 nM) for 20 min and fixed.

Immunostaining is performed using rabbit polyclonal anti-GLUT4 (F349) (1 μg/ml). Each fluorescein isothiocyanate-positive microinjected cell is evaluated for the presence of plasma membrane-associated GLUT4 staining. Control cells are injected with preimmune sheep IgG and then processed in the same way as experimentally injected cells. As quantitated by immunofluorescent GLUT4 staining, insulin leads to an increase in GLUT4 translocation to the plasma membrane. Cells are incubated with wortmannin as a control to block basal and insulin-induced GLUT4 translocation. The compound of the instant invention could stimulate insulin-induced GLUT4 translocation, which could indicate that administration of the compound of the invention inhibited kinase activity, e.g., PTEN function, resulting in an increase in intracellular phosphatidylinositol 3,4,5-triphosphate levels, which stimulates GLUT4 translocation.

**Example 18: Effect on Retinal Neovascularization**

The following example illustrates that compound (I) could be used to treat eye diseases, e.g., macular degeneration, retinopathy and macular edema. The effect of compound (I) on retinal neovascularization is determined using a model of retinal neovascularization as previously described (Aiello, et al.; 1995, Proc. Natl. Acad. Sci., 92, 10457-10461). Briefly, C57B1/6J mice are exposed to 75% $O_2$ from postnatal day 7 (P7) to P12 along with nursing mothers. At P12, the mice are returned to room air. Intraocular injections are performed at P12 and sometimes P14 as described below. At P17 the mice are sacrificed by cardiac perfusion of 4% paraformaldehyde in phosphate-buffered saline and the eyes are enucleated and fixed in 4% paraformaldehye overnight at 4 °C before paraffin embedding.

Mice are deeply anesthetized with tribromoethanol for all procedures. The lid fissure is opened (e.g., using a no. 11 scalpel blade) and the eye is proptosed. Intravitreal injections are performed by first entering the left eye with an Ethicon TG140-8 suture needle at the posterior limbus. A 32-gauge Hamilton needle and syringe are used to deliver the compound of the instant invention diluted in Alcon balanced salt solution through the existing entrance site. The eye is then repositioned and the lids are approximated over the cornea. Repeat injections are performed through a previously unmanipulated section of limbus 2 days later. As a control, equal amounts of saline are injected to the right eye.

Over 50 serial 6-μm paraffin-embedded axial sections are obtained starting at the optic nerve head. After staining with periodic acid/Schiff reagent and hematoxylin (Pierce, et al.; 1995, Proc. Natl. Acad. Sci. USA., 92, 905-909; Smith et al.; 1994, Invest. Ophthal. Vis. Sci., 35, 101-111), 10 intact sections of equal length, each 30 μm apart, are evaluated for a span of 300 μm. Eyes exhibiting retinal detachment or endophthalmitis are excluded from evaluation. All retinal vascular cell nuclei anterior to the internal limiting membrane are counted in each section by a fully masked protocol. The mean of all 10 counted sections yield average neovascular cell nuclei per 6-μm section per eye. No vascular cell nuclei anterior to the internal limiting membrane are observed in normal, unmanipulated animals (Smith et al.; 1994, Invest. Ophthal. Vis. Sci., 35, 101-111). Reduction in neovascularization could be observed in the eyes treated with compound as compared to the eyes in the saline control group.

**Example 19: Modulation of a Kinase Signaling Cascade Associated with Stroke**

Many animal models for stroke have been developed and characterized, *see e.g.,* Andaluz, et al., Neurosurg. Clin. North Am., vol. 13:385-393 (2002); Ashwal, S. and W. J. Pearce., Curr. Opin. Pediatr., vol 13:506-516 (2001); De Lecinana, et al., Cerebrovasc. Dis., vol. 11(Suppl. 1):20-30 (2001); Ginsberg and Busto, Stroke, vol. 20:1627-1642 (1989); Lin, et al., J. Neurosci. Methods, vol. 123:89-97 (2003); Macrae, I. M., Br. J. Clin. Pharmacol., vol. 34:302-308 (1992); McAuley, M. A., Cerebrovasc. Brain Metab. Rev., vol. 7:153-180 (1995); Megyesi, et al., Neurosurgery, vol. 46:448-460 (2000); Stefanovich, V. (ed.)., Stroke: animal models. Pergamon Press, Oxford (1983); and Traystman, R. J., ILAR J. 44:85-95 (2003), each of which is hereby incorporated by reference in its entirety. For a review of animal models of focal (stroke) and global (cardiac arrest) cerebral ischemia, *see e.g.,* Traystman, ILAR J., vol. 44(2):85-95 (2003) and Carmichael, NeuroRx®: The Journal of the American Society for Experimental NeuroTherapeutics, vol. 2:396-409 (2005, each of which is hereby incorporated by reference in its entirety.

Compounds that modulate cell death in stroke are identified using any of the art-recognized models for stroke. In the studies described herein, intra-arterial suture occlusion of the middle cerebral artery (MCA), a procedure known as MCAo, through the internal carotid artery is used as a model for cell death in stroke. In the control and test group of rats, the external carotid artery is transected, the common carotid artery is tied off, and the external carotid artery is then used as a pathway to pass a suture through the internal carotid artery, wherein the suture lodges in the junction of the anterior and middle cerebral arteries. To reduce subarachnoid hemorrhage and premature reperfusion, the suture is preferably coated with an agent such as silicone. The suture is used to occlude the MCA, *e.g.,* for a duration of 60, 90, or 120 minutes and to permanently occlude the MCA.

In the test group, rats are administered compound (I) at a variety of times prior to, during and after occlusion of the MCA with the suture. The effect of the compound on the test group is compared to the effects observed in the control group, for example, by measuring the extent of cell death in each MCAo group. Typically, in the control group, the pattern of cell death follows a progression from early infarction in the striatum to delayed infarction in the dorsolateral cortex overlying the striatum. Striatal is mostly necrotic and occurs rapidly. The pattern of cell-death in the test group is compared to that of the control group to identify compounds that modulate cell death in stroke.

**Example 20: Modulation of a Kinase Signaling Cascade Associated with Atherosclerosis**

Many animal models for atherosclerosis have been developed and characterized. For a review of animal models of atherosclerosis, restenosis and endovascular graft research, *see e.g.,* Narayanaswamy et al., JVIR, vol. 11(1): 5-17 (2000), which is hereby incorporated by reference in its entirety. Atherosclerosis is induced in a suitable animal model using a high fat/high cholesterol (HFHC) diet. The test animal is an animal that contains cholesterol ester transferase, such as the rabbit or the swine. The HFHC diet is produced, *e.g.,* using commercial chow supplemented with fat. Cholesterol intake is between 0.5-2.0% of the diet. A test group of animals, *e.g.,* rabbits or swine, receives compound (I). The effect of the test compound is compared to the effects of atherosclerosis in the untreated, control group of animals. Effects that are compared include, for example, the degree of plaque formation, the number and/or frequency of myocardial infarctions observed in each group of animals, and the extent of tissue damage secondary to myocardial infarction exhibited in coronary tissue.

Myocardial infarction is studied using a variety of animal models such as rats and mice. The majority of myocardial infarctions result from acute transbotic occlusion of pre-existing atherosclerotic plaques of coronary arteries, which is mimicked in animal models by ligation of the left coronary artery in *e.g.,* rats and mice. Myocardial infarction induces global changes in the ventricular architecture, a process called ventricular remodeling. The infarcted heart progressively dilates and accelerates the deterioration of ventricular dysfunction that eventually results in heart failure.

Myocardial ischemia is induced in test and control groups of animals, *e.g.,* mice or rats, by ligating the left anterior descending coronary artery. The affected heart tissue is contacted with compound (I) or a pharmaceutically acceptable salt thereof, for example, by intraperitoneal (i.p.) injections, after the induction of ischemia. High resolution magnetic resonance imaging (MRI), dry weight measurements, infarct size, heart volume, and area at risk are determined 24 hours postoperatively. Survival rates and echocardiography are determined at various times postoperatively in the rats receiving injections of compound (I) or a pharmaceutically acceptable salt thereof. Other effects of the test compound are compared to the control group of rats. For example, changes in left ventricular geometry and function are characterized using echocardiography to compare end-diastolic diameters, relative wall thickness, and the percentage of fractional shortening. In excised hearts, the infarct size calculated and expressed as a percentage of left ventricular surface area.

**Example 21: Modulation of a Kinase Signaling Cascade Associated with Neuropathic Pain**

Many animal models for neuropathic pain, such as chronic neuropathic pain, have been developed and characterized, *see e.g.,* Bennett & Xie, Pain, vol. 33, 87-107 (1988); Seltzer et al., Pain, vol. 43, 205-18 (1990); Kim & Chung,

Pain, vol. 50, 355-63 (1992); Malmberg & Basbaum, Pain, vol. 76, 215-22 (1998); Sung et al., Neurosci Lett., vol. 246, 117-9 (1998) ; Lee et al., Neuroreport, vol. 11, 657-61 (2000); Decosterd & Woolf, Pain, vol. 87, 149-58 (2000); Vadakkan et al., J Pain, vol. 6, 747-56 (2005), each of which is hereby incorporated by reference in its entirety. For a review of animal models used for neuropathic pain, *see e.g.,* Eaton, J. Rehabilitation Research and Development, vol. 40(4 Supplement):41-54 (2003), the contents of which are hereby incorporated by reference in their entirety.

Compounds that modulate neuropathic pain are identified using any of the art-recognized models for neuropathic pain. For example, the models for neuropathic pain generally involve injury to the sciatic nerve, although the method used to induce injury varies. For example, the sciatic nerve is injured due to partial constriction, complete transection, freezing of the nerve, and metabolic, chemical, or immune insults to the nerve. Animals with these types of nerve injury have been shown to develop abnormal pain sensations similar to those reported by neuropathic pain patients. In the studies described herein, the sciatic nerve of test and control groups of subjects, such as mice, are injured. In the test group, subjects are administered compound (I) at a variety of times prior to, during and after injury to the sciatic nerve. The effects of the compound on the test group are compared to the effects observed in the control group, *e.g.*, through physical observation and examination of the subjects. For example, in mice, the subject's hindpaw is used to test the response to non-noxious stimuli, such as tactile stimulation, or to test the subject's response to stimuli that would be noxious in the course of ordinary events, for example, radiant heat delivered to the hindpaw. Evidence of allodynia, a condition in which ordinarily nonpainful stimuli evoke pain, or a hyperalgesia, the excessive sensitiveness or sensibility to pain, in the test subjects indicates that test compound is not effectively modulating neuropathic pain in the test subjects.

**Example 22: Modulation of a Kinase Signaling Cascade Associated with Hepatitis B**

Many animal models for hepatitis B have been developed and characterized. For a review of animal models of hepatitis B, *see e.g.,* Guha et al., Lab Animal, vol. 33(7):37-46 (2004), which is hereby incorporated by reference in its entirety. Suitable animal models include, for example, the chimpanzee, tree shrews (non-rodent small animals that are phylogenetically close to primates, *see* Walter et al., Hepatology, vol. 24(1):1-5 (1996), which is hereby incorporated by reference in its entirety), and surrogate models such as the woodchuck, duck and ground squirrel. (*See e.g.,* Tennant and Gerin, ILAR Journal, vol. 42(2):89-102 (2001), which is hereby incorporated by reference in its entirety).

For example, primary hepatocytes are isolated from livers of the tree shrew species tupaia belangeri and are infected with HBV. *In vitro* infection results in viral DNA and RNA synthesis in hepatocytes and secretion hepatitis B surface antigen (HBsAg) and hepatitis B e antigen (HBeAg) into culture medium. Tupaias can also be infected with HBV *in vivo,* resulting in viral DNA replication and gene expression in tupaia livers. Similar to acute, self-limited hepatitis B in humans HBsAg is rapidly cleared from serum, followed by seroconversion to anti-HBe and anti-HBs.

Compounds that modulate hepatitis B are identified using any of the art-recognized models for hepatitis B. In the studies described herein, test and control groups of animals, *e.g.*, chimpanzees or tree shrews, are infected with HBV. In the test group, subjects are administered compound (I) at a variety of times prior to, during and after exposure to HBV. The effects of the compound on the test group are compared to the effects observed in the control group, *e.g.*, through physical observation and examination of the subjects and through blood or serum analysis to determine at what point in time the infection is cleared from the subject. For example, assays are run to detect the presence and/or amount of hepatitis B virus called surface antigens and fragments thereof. Alternatively or in addition, the subject's liver is analyzed. Liver function tests analyze levels of certain proteins and enzymes, such as, for example, aspartate aminotransferase (AST, formerly serum glutamic-oxaloacetic transaminase (SGOT)) and alanine aminotransferase (ALT, formerly serum glutamate-pyruvate transaminase (SGPT)).

**Example 23: The Effect on Tyrosine Kinase Inhibition**

The following example illustrates that compound (I) could be used to treat autoimmune diseases. Compound (I) is tested using a method described previously (Goldberg, et al.; 2003, J. Med. Chem., 46, 1337-1349). The kinase activity is measured using DELFIA (dissociation enhanced lanthanide fluoroimmunoassay), which utilizes europium chelate-labeled anti-phosphotyrosine antibodies to detect phosphate transfer to a random polymer, poly-Glu4-Tyrl (PGTYR). The kinase assay is performed, in a neutravidin-coated 96-well white plate in kinase assay buffer (50 mM HEPES, pH 7.0, 25 mM MgC12, 5 mM MnC12, 50 mM KCl, 100 μM Na3VO4, 0.2% BSA, 0.01% CHAPS). Test samples (compound (I)) initially dissolved in DMSO at 1 mg/mL are prediluted for dose response (10 doses with starting final concentration of 1 μg/mL, 1-3.5 serial dilutions) with the assay buffer. A 25 μL aliquot of this diluted sample and a 25 μL aliquot of diluted enzyme (lck) (0.8 nM final concentration) are sequentially added to each well. The reaction is started with a 50 μL/well of a mixture of substrates containing 2 μM ATP (final ATP concentration is 1 μM) and 7.2 ng/μL PGTYR-biotin in kinase buffer. Background wells are incubated with buffer and substrates only. Following 45 min of incubation at room temperature, the assay plate is washed three times with 300 μL/well DELFIA wash buffer. A 100 μL/well aliquot of europium-labeled anti-phosphotyrosine ($Eu^{3+}$-PT66, 1 nM, Wallac CR04-100) diluted in DELFIA assay buffer is added

to each well and incubated for 30 min at room temperature. Upon completion of the incubation, the plate is washed four times with 300 μL/well of wash buffer and 100 μL/well of DELFIA wash buffer. Enhancement solution (Wallac) is added to each well. After 15 min, timeresolved fluorescence is measured on the LJL's analyst (excitation at 360 nm, emission at 620 nm, EU 400 dichroic mirror) after a delay time of 250 μs. The compound of the instant invention could inhibit the kinase activity of lck, indicating that the compound may be used to treat autoimmune disease in a subject.

**Example 24: $IC_{50}$ of compound (I) and Dasatinib in Dasatinib-resistant Cell Lines; Twelve (12) concentrations of Inhibitor in Each Cell Line.**

Cancer cell lines reported in current literature to be Dasatinib-resistant (*i.e.,* COLO-320DM, H460, H226, and HCT-116) were cultured in the presence of the Compound (I) Src inhibitor or Dasatinib control in order to determine the effect of compound (I) on cell growth inhibition. Cell proliferation/growth inhibition was assessed using MTT colorimetric assay. Additionally, the $IC_{50}$ of both compound (I) and Dasatinib control was determined. The table below provides a list of the cell lines used in this growth inhibition study.

| NAME | ATCC No. | TYPE |
|---|---|---|
| H460 | HTB-177 | NSCLC |
| H226 | CRL-5826 | NSCLC |
| COLO-320DM | CCL-220 | colorectal adenocarcinoma |
| HCT116 | CCL-247 | colorectal carcinoma |

COLO-320DM, H460, H226, and HCT-116 human cancer cell lines were routinely cultured and maintained in basal medium containing 2% FBS at 37° C, 5% $CO_2$. For the experiments, cells are seeded at 4.0 x $10^3$/190 μL and 8.0 x $10^3$/190 μL per well of 96-well plate in basal medium/1.5% FBS. Cells cultured are overnight (16 h) in 96-well plates at 37° C in appropriate $CO_2$ conditions prior to compound (I) and Dasatinib addition.

For compound (I) and Dasatinib (BMS354825) dilutions, 20 mM stock solution samples were diluted serially in basal medium/1.5% FBS using 1:3 dilutions, yielding 20x concentrations in the 131 μM to 0.74 nM range. 10 μL of 20x dilutions are then added to appropriate wells (n=3) containing 190 μL cancer cell lines, yielding 6561 nM to 0.037 nM range of final concentrations. The following controls were used: Vehicle control of cells and no sample; Medium Control of cells, no sample, and 0.03% DMSO (highest DMSO concentration present in samples; not reported in results).

Treated cancer cells were incubated for 3 Days (78 hours) at 37 °C, appropriate $CO_2$ conditions. On Day 3, 10 μL MTT (5 mg/mL) was added to each well. Cells were then incubated in the presence of MTT for 4 hours at 37° C, appropriate $CO_2$ conditions. After this incubation period, 90 μL 10% SDS(+HCl) was added to each well to lyse cells and solubilize formazan. Cells were then incubated overnight at 37 °C, appropriate $CO_2$ conditions.

The $OD_{570}$ was measured using microplate reader. Growth inhibition curves and $EC_{50}$/ $IC_{50}$ were determined using GraphPad Prism 4 statistical software. Data was normalized to represent percent of maximum response.

The table below shows the $IC_{50}$s of compound (I) and Dasatinib in cancer cell lines (8.0 x $10^3$ cells/well, 1.5% FBS) at 78 Hr (results from normalized response data).

| Human Solid Tumor Cell Line | **Compound (I) nM** | | | **Dasatinib nM** | | | **Dasatinib nM** |
|---|---|---|---|---|---|---|---|
| **NAME** | **$IC_{50}$** | **$IC_{80}$** | **$IC_{90}$** | **$IC_{50}$** | **$IC_{80}$** | **$IC_{90}$** | **Literature $IC_{50}$** |
| H460 | 51 | 105 | 162 | 90 | 7,110 | 48,880 | 1,800* |
| H226 | 98 | 277 | 490 | 163 | 7,758 | 34,340 | 10,000* |
| COLO-320DM | 30 | 80 | 144 | 1 | 2 | 14 | 10,000** |
| HCT116 | 31 | 106 | 195 | 880 | NA | NA | 5,000** |
| * Johnson et al., Clin Cancer Res 2005;11(19) :6924-6932, October 1,2005<br>** Puputti et al., Mol Cancer Ther 2006;5 (12): 927-934, December 2006 | | | | | | | |

**Example 25: Effect of compound (I) on Dasatinib and Imatinib Resistant Leukemia Cells.**

Ba/F3 cells (*See e.g.,* Palacios et al., Nature 309: 126-131 (1984); Palacios et al., Cell 41: 727-734 (1985)) were cultured in 96-well plates in complete media + IL-3. Cultures of Ba/F3 cells were also transfected to express wild-type (WT) Bcr-Abl, E255K mutation of Bcr-Abl, or T315I mutation of Bcr-Abl and cultured in 96-well plates in complete media without IL-3. The Ba/F3 cell line is rendered Gleevec resistant when the mutation in the Bcr/Abl tyrosine kinase E225K is present. The Ba/F3 cell line is rendered both Gleevec and Dasatinib resistant when the Bcr/Abl tyrosine kinase T315I mutation is present. The cells of each group were then treated with no drug, 0.1 - 10,000 nM Dasatinib, or 0.1 - 10,000 nM compound (I) in 10-fold dilutions for 96 hrs. MTT assays were performed (plate read at 570 nM). All assays are in triplicate.

The results of this study, summarized in Figures 12-13 and the table below, show that compound (I) inhibits the T315I mutant of BCR-Abl at $GI_{50}$ = 35, whereas Dasatinib does not inhibit at 10,000 nM. Further, Dasatinib does not inhibit IL-3-induced proliferation of Ba/F3 cells whereas compound (I) is a potent inhibitor ($GI_{50}$ = 3.5 nM).

| Cell line | $GI_{50}$ values (nM) | |
|---|---|---|
| | Dasatinib | Compound (I) |
| Ba/F3 | --- | 3.5 |
| + WT BCR-Abl | 1 | 85 |
| + E225K | 1 | 80 |
| + T3151 | >10,000 | 35 |

**Example 26: $GI_{50}$s / BrdU Assay in five cell lines with compound (I) and BMS354825.**

Evaluation of the GI50s in five cell lines (SKOV-3, K562, HT-29, A549 & MDA-MB-231) with compound (I) and BMS354825 assayed at T=0 and T=72 using BrdU.

For these experiments, cells were seeded in two 96-well plates per cell line with the cell number indicated below in 200 μL growth media containing 1.5% FBS. Cell lines being evaluated are: SKOV-2, K562, HT-29, A549, and MDA231. All seeded at 1000 cells per well except HT-29 (2000 cells) and MDA MB 231 (5000 cells). The plates were incubated for 24 hours after seeding at 37 °C + 5% $CO_2$. Except MDA231, this line is grown at 37 °C and 0% $CO_2$.

After 24 hours post-seeding, compound (I) and BMS354825 were added at 128nM, 64nM, 32nM, 16nM, 8nM, 4nM, 2nM, and 1nM to 1 plate of each cell line (n=3). Compound (I) and BMS354825 treated sets of cell line plates were incubated for 72 hours at 37oC + 5% $CO_2$. Except MDA231, this line is grown at 37 °C and 0% $CO_2$. Brdu assay was performed at T=0 and T=72.

Growth Inhibition. The BrdU data was used to determine the % growth inhibition for each sample concentration using the formula:

$$GI= [\ (T_1-T_0) / (Con-T_0)\ ]x100$$

where $T_0$=Fluorescence of cells at time 0; $T_1$= Fluorescence of treated cells at x hours; Con = Fluorescence of control cells at x hours. $T_1$ values ≤ $T_0$ values were designated as T, cytotoxicity. The $GI_{50}$ was estimated using XLFit excluding $T_1$ values ≤ $T_0$ (cytotoxicity). The results of this study, summarized in Figures 14-18 and the table below.

| $GI_{50}$ Data Summary | | |
|---|---|---|
| | Compound (I) | BMS-354825 |
| **HT-29** | 1.54E-08 M | 2.05E-08 M |
| **SKOV-3** | 9.75E-09 M | 3.24E-09 M |
| **A549** | 9.39E-09 M | 1.25E-08 M |
| **K562** | 1.09E-08 M | <1.0E-9 M |
| **MDA-MB-321** | 1.98E-08 M | 6.02E-09 M |

**Example 27: Combination $GI_{50}$ of Gemzar® and compound (I) in the L3.6pl cell line using the BrdU assay.**

This study involved the evaluation of the $GI_{50}$ of Gemzar® ± compound (I) in the L3.6pl cell line assayed at

T=0 and T=72 using the BrdU Assay (Roche: Catalog Number, 11647229001). L3.6pl cells, a human pancreatic cancer cell line, were seeded in three 96-well plates with 2000 cells/well for L3.6pl in 190 μL growth media containing 1.5% FBS. L3.6pl cells are previously described in Trevino et al. Am J Pathol. 2006 Mar;168(3):962-72, hereby incorporated herein by reference in its entirety. The cells were incubated for 18-24 hours after seeding at 37°C + 5% $CO_2$. After 24 hours, Gemzar® + compound (I), Gemzar®, and compound (I) was added to the L3.6pl cells (n=3). Gemzar® was evaluated at concentrations of 8nM, 4nM, 2nM, 1nM, 0.5nM, 0.25nM, 0.125nM, 0.063nM. Compound (I) was evaluated at concentrations of 100nM, 50nM, 25nM, 12.5nM, 6.25nM, 3.125nM, 1.56nM, and 0.78nM. Each sample treated plate was incubated for 72 hours at 37 °C + 5% $CO_2$. The BrdU assay was perfomed at T=0 and again after 72 hours of incubation, T=72. The results of the study are provided in Figures 19 and 20. The table below provides a summary of the calculated $GI_{50}$ for Gemzar® ± compound (I).

| $GI_{50}$ Summary Table | | |
|---|---|---|
| | **Compound (I) nM** | **Gemzar® nM** |
| **Single** | 53.03 | 1.76 |
| **Combined** | 1.15 | 0.09 |

**Example 28: Orthotopic Prostate Model for Measuring *in Vivo* Metastases**

Nu/Nu mice (8-12 weeks of age) were injected with PC3-MM2 prostate cancer cells into the prostate as described previously in Pettaway et al., Clin Cancer Res 1996, 2:1627-1636, hereby incorporated herein by reference in its entirety. Fourteen days after orthotopic injection of PC3-MM2 cells, the mice were randomized into four groups: Dasatinib (15 mg /kg/day) treatment; compound (I) (5 mg /kg/day) treatment; compound (I) (10 mg /kg/day) treatment; and control (vehicle). Dasatinib, compound (I), and vehicle was administered by oral gavage. All mice were sacrificed by cervical dislocation on about day 42. Tumor volume (measured by caliper), weight, and incidence of regional (celiac or para-aortal) lymph node metastases were recorded. Results of the experiment are reported in the table below and shown in Figures 21 and 22.

| | **Tumor incidence** | **Tumor weight (g)** | | **LN metastasis** |
|---|---|---|---|---|
| | | **Median** | **(IQR)** | |
| **Control** | 5/6 | 2.27 | (1.94 ~ 2.61) | 5/5 |
| **Compound (I) (5.0mg/kg/day)** | 5/6 | 1.16 | (0.94 ~ 1.28) | 4/5 |
| **Compound (I) (10.0mg/kg/day)** | 5/6 | 0.35 | (0.24 ~ 0.56) | 2/5 |
| **Dasatinib (15mg/kg/day)** | 5/6 | 0.43 | (0.30 ~ 1.34) | 2/5 |

**Example 29: HBV Primary Assay**

The HBV primary assay developed was conducted similarly to that described by Korba et al., (Antiviral Res. 15: 217-228 (1991) and Antiviral Res. 19: 55-70 (1992)) with the exception that viral DNA detection and quantification have been improved and simplified (Korba et al., Antiviral Res. 19: 55-70 (1992)).

Compound (I) was evaluated for potential anti-HBV activity using a single high-test concentration of the compound in the standardized HepG2-2.2.15 antiviral assay. The HepG2-2.2.15 is a stable cell line producing high levels of the wild-type ayw1 strain of HBV. Briefly, HepG2-2.2.15 cells were plated in 96-well plates. Only the interior wells were utilized to reduce "edge effects" observed during cell culture; the exterior wells are filled with complete medium to help minimize sample evaporation. On the following day, the confluent monolayer of HepG2-2.2.15 cells was washed and the medium is replaced with complete medium containing test concentrations of a test article in triplicate. 3TC was used as the positive control, while media alone was added to cells as the untreated control. Three days later the culture medium was replaced with fresh medium containing the appropriately diluted test compound. Six days following the initial administration of the test compound, the cell culture supernatant was collected, treated with pronase and DNAse and then used in a real-time quantitative TaqMan PCR assay for direct measurement of HBV DNA copies using an ABI Prism 7900 sequence detection system (Applied Biosystems, Foster City, CA).

The antiviral activity of each test compound was calculated by comparing its HBV DNA copies against that of the untreated control cells (100%) to derive percent inhibition level. After removing the supernatant, the remaining cells

were subject to CellTiter 96 Aqueous One (Promega, Madison, WI) solution cell proliferation assay (MTS-based) to measure cell viability. Cytotoxicity of the compound was determined by comparing its cell viability with that of the untreated cell control to derive percentage of the cell control. Results of this study are provided in the table below and in Figure 23.

| Compound | Test Concentration | Antiviral Activity | Cytotoxicity | Interpretation |
|---|---|---|---|---|
| | | Percent inhibition of cell control | Percent of cell control | |
| 3TC | 1 μM | 92.0% | 103.3% | Active |
| Compound(I) : 2HCl | 10 μM | 48.2% | 51.3% | Cytotoxic |

**Example 30: Inhibition of Src Kinase Activity in Whole Cells**

Compound (I) inhibits Src kinase activity in whole cells as shown in Figures 10A, 10B, 10C, and 10D. Figure 10A is a graph depicting the effect of compound (I) on Src autophosphorylation in c-Src/NIH-3T3 cells; Figure 10B is a graph indicating the effect of compound (I) on Src autophosphorylation in HT-29 cells; Figure 10C is a graph depicting the effect of compound (I) on Src transphosphorylation in c-Src/NIH-3T3 cells; and Figure 10D is a graph indicating the effect of compound (I) on Src autophosphorylation in HT-29 cells. Compound (I) is a potent inhibitor of Src kinase activity in whole cells. As shown in Figures 10A-10D, compound (I) is a potent inhibitor of Src kinase activity in whole cells. In particular, compound (I) was a potent inhibitor of Src autophosphorylation (Figures 10A and 10B) and Src transphosphorylation (Figures 10C and 10D) in various cell lines. Similar whole cell inhibition results were obtained for additional transphosphorylation substrates, *i.e.,* FAK Y925 & paxillin Y31. Phosphorylations of PDGF Y572/574, EGF Y845, JAK1 Y1022/1023 & JAK2 Y1007/1008, Lck Y405 & ZAP70 Y319 were not inhibited in whole cells. Lyn Y416 and Bcr/Abl &245 were inhibited less potently.

**Example 31: Selectivity for Protein Tyrosine Kinases in Whole Cells**

Compound (I) is selective for protein tyrosine kinases (PTKs). Figure 11 is an illustration depicting the selectivity of compound (I) for protein tyrosine kinases (PTKs) in whole cells as compared to Dasatinib, an ATP-competitive Src inhibitor currently in clinical trials. SYF cells are mouse fibroblasts that lack the Src kinase family members Src, Yes and Fyn. Compound (I) demonstrated very high PTK selectivity in whole cells as compared to Dasatinib.

**Example 32: Oral Potency**

Compound (I) demonstrates high oral potency. For example, Figure 24 shows the oral potency of compound (I) in comparison to Dasatinib. Oral potency was determined using staged HT29 (human colon cancer) mouse Xenografts over a period of 28 days of treatment. Compound (I) was tested at 2.0 and 4 mg/kg bid. Dasatinib was tested at 25 mg/kg bid. At day 5, Dasatinib dose was lowered to 15 mg/kg bid due to weight loss.

**Example 33: HCV Primary Assay**

Compound (I) could be used to treat HCV. Compound (I) is tested using the method of Pietschmann, T., et al. J. Virol. 76:4008-4021. The ET call line is a human hepatoma cell line, Huh-7, harboring an HCV RNA replicon (genotype 1b) with a stable luciferase (Luc) reporter and three cell culture-adaptive mutations. The cells are grown in Dulbecco's modified essential media (DMEM), 10% fetal bovine serum (FBS), 1% penicillin-streptomycin (pen-strep), 1% glutamine, 5 mg/ml G418 in a 5% $CO_2$ incubator at 37°C. All cell culture reagents are from e.g., Mediatech (Herndon, VA).

**Example 34: Plasma and Brain Exposure**

Compound (I) demonstrates good plasma/brain exposure. For example, the plasma and brain exposure of compound (I) is described below. Plasma concentrations were measured in mice after oral administration. All doses were formulated in purified water. Male CD-1 mice were dosed after an overnight fast and fed 4 hours post-dose. Dosing was as follows:

| Group Number | Route | Compound | Dose (mg/kg)* | Dose Vol. (mL/kg) |
|---|---|---|---|---|
| 1 | PO | Compound (I) Mesylate | 10 | 10 |
| 2 | PO | Compound (I) Mesylate | 50 | 10 |
| *Note: Doses administered were mg free base/kg | | | | |

Protein was precipitated with 0.25 mL acetonitrile for plasma, 0.25 mL for brain. After centrifugation, supernatant was directly injected into an LC/MS system. The limit of quantitation was 1 ng/mL using a 50 μL aliquot for plasma and a 50 μL aliquot for brain. The standard curve was 1 to 1,000 ng/mL for both plasma and brain.
HPLC conditions were as follows:
HPLC System: Shimadzu SCL-10 System
Analytical Column: Aquasil C18 5 μm 100x2 mm column.
Column Temperature: Ambient temperature
Autosampler Temperature: Ambient temperature
Mobile Phase

A) 10 mM Ammonium formate in water (pH 4).
B) Acetonitrile.

Flow Rate: 0.6 mL/min
Injection Volume: 2 μL
Gradient:

| Time (Minute) | 0.0 | 1.6 | 2.6 | 3.8 | 3.9 | 4.1 | 4.4 | 4.6 | 4.65 | 7.0 |
|---|---|---|---|---|---|---|---|---|---|---|
| %B | 20 | 20 | 65 | 65 | 20 | 20 | 95 | 95 | 20 | Stop |

Mass Spectrometry Conditions were as follows:

Instrument: ABI Sciex API 4000
Mode: ESI+
Experiment: MRM (multiple reaction monitoring)
Transitions: Compound (I): m/z 432.4→114.2 (Rt = 3.11 minute)

The four tables directly below show plasma and brain concentrations following the administration of a single oral dose of compound (I) at 10 mg/kg and 50 mg/kg.

**Compound (I) Plasma Concentrations (ng/mL) in Male CD-1 Mice After a Single PO Dose of 10 mg/kg (Group 1)**

| Time (hr) | Group A | Group B | Group C | Mean | SD | %CV |
|---|---|---|---|---|---|---|
| 0 | BLQ | BLQ | BLQ | 0.00 | 0.00 | NA |
| 0.5 | 778.51 | 1096.62 | 737.37 | 870.83 | 196.62 | 22.58 |
| 1 | 516.97 | 328.28 | 243.96 | 363.07 | 139.79 | 38.50 |
| 2 | 328.47 | 271.89 | 261.57 | 287.31 | 36.02 | 12.54 |
| 5 | 133.38 | 147.74 | 160.62 | 147.25 | 13.63 | 9.26 |

NA: Not Applicable.
BLQ: Below Limit of Quantitation (1 ng/mL)
BLQ = 0 when calculating mean, SD and %CV

**Compound (I) Brain Concentrations (ng/g) in Male CD-1 Mice After a Single PO Dose of 10 mg/kg (Group 1)**

| Time(hr) | Group A | Group B | Group C | Mean | SD | %CV |
|---|---|---|---|---|---|---|
| 0 | BLQ | BLQ | BLQ | 0.00 | 0.00 | NA |
| 0.5 | 398.43 | 509.00 | 286.70 | 398.04 | 111.15 | 27.92 |
| 1 | 150.70 | 266.66 | 92.06 | 169.81 | 88:85 | 52.32 |
| 2 | 125.69 | 84.04 | 85.88 | 98.54 | 23.53 | 23.88 |
| 5 | 67.68 | 75.21 | 71.22 | 71.37 | 3.77 | 5.28 |

**Compound (I) Plasma Concentrations (ng/mL) in Male CD-1 Mice After a Single PO Dose of 50 mg/kg (Group 2)**

| Time(hr) | Group A | Group B | Group C | Mean | SD | %CV |
|---|---|---|---|---|---|---|
| 0 | BLQ | BLQ | BLQ | 0.00 | 0.00 | NA |
| 0.5 | 8511.88 | 8334.53 | 12315.31 | 9720.57 | 2248.85 | 23.13 |
| 1 | 2374.12 | 2442.20 | 1365.56 | 2060.62 | 602.91 | 29.26 |
| 2 | 1148.57 | 1546.09 | 1850.18 | 1514.95 | 351.84 | 23.22 |
| 5 | 424.48 | 1139.11 | 1201.91 | 921.83 | 431.86 | 46.85 |

**Compound (I) Brain Concentrations (ng/g) in Male CD-1 Mice After a Single PO Dose of 50 mg/kg (Group 2)**

| Time(hr) | Group A | Group B | Group C | Mean | SD | %CV |
|---|---|---|---|---|---|---|
| 0 | BLQ | BLQ | BLQ | 0.00 | 0.00 | NA |
| 0.5 | 2795.27 | 3190.42 | 5089.32 | 3691.67 | 1226.42 | 33.22 |
| 1 | 945.72 | 936.29 | 482.22 | 788.08 | 264.92 | 33.62 |
| 2 | 613.18 | 530.41 | 684.97 | 609.52 | 77.34 | 12.69 |
| 5 | 200.01 | 387.73 | 522.17 | 369.97 | 161.81 | 43.74 |

The brain and plasma pharmacokinetic parameters of compound (I) in mice after a single dose of 10 mg/kg (Group 1) are as follows:

| Sample ID | $T_{max}$ (hr) | $C_{max}$ (ng/mL) | AUClast (ng·hr/mL) |
|---|---|---|---|
| Brain | 0.50 | 398 | 631 |
| Plasma | 0.50 | 871 | 1503 |
| Note: Brain Cmax and AUClast are ng/g and ng-hr/g, respectively. | | | |

The AUClast Brain/AUClast Plasma Ratio is 0.42.

The brain and plasma pharmacokinetic parameters of compound (I) in mice after a single dose of 50 mg/kg (Group 2) are as follows:

| Sample ID | $T_{max}$ (hr) | $C_{max}$ (ng/mL) | AUClast (ng·hr/mL) |
|---|---|---|---|
| Brain | 0.50 | 3692 | 4211 |
| Plasma | 0.50 | 9721 | 10818 |
| Note: Brain Cmax and AUClast are ng/g and ng·hr/g, respectively. | | | |

The AUClast Brain/AUClast Plasma Ratio is 0.39.

**Example 35: Glioma Survival Studies**

A brain tumor mouse xenograft study was conducted comparing compound (I) to Temodar®. The studies were conducted in C57BL/6 mice. GL261 glioma cells (1 x $10^5$ in 5μl DMEM) were implanted intracranial coordinates: bregma, lateral 2.0mm, anterior 1.2mm, 3.0mm depth dura. Treatment was initiated 3 days post implantation. The groups were as follows (all doses in 100μl $H_2O$):

| | | |
|---|---|---|
| Vehicle ($H_2O$) | | |
| Compound (I) 2.5mg/kg | bid oral | |
| Compound (I) 5mg/kg | bid oral | |
| Temodar® 5mg/kg | once weekly oral | |

The table below shows a summary of the results. The median survival range and the log-rank (Mantel-Cox) statistical test results comparing the survival distributions of the samples.

| | Vehicle | Compound (I) 2.5mg/kg bid oral | Compound (I) 5mg/kg bid oral | Temodar® 5mg/kg weekly xl oral |
|---|---|---|---|---|
| Median survival Range: | **22 21-25** | **25 22-36** | **23 22-29** | 29 26-29 |
| vs. Vehicle | | P=0.1062 | P=0.1762 | P=0.0017 |
| vs. Temodar | **P=0.0017** | P=0.3649 | P=0.1366 | |
| vs. compound (I) 2.5 mg/kg | | | | P=0.8901 |
| vs. compound (I) 5mg/kg | | | | P=0.1366 |

Figures 24 and 25 A-D show the weight gain in each of the C57BL/6 mice in the different treatment groups. The average weight at endpoint for each of the treatment groups is shown in the table below. Figure 26 is a graph showing the average weights over a 40-day period for each of the treatment groups.

**Average weight at endpoint**

| | | |
|---|---|---|
| Vehicle | | 19.2 g |
| Compound (I) | 2.5 mg/kg | 16.0g |
| Compound (I) | 5 mg/kg | 14.3g |
| Temodar® | 5 mg/kg | 13.3 g |

**Example 36. Synergistic Cell Growth Inhibition Using a Combination**

The combination of compound (I) and tamoxifen was tested *in vitro* to determine the ability of the combination to inhibit cell growth in MCF-7 breast cancer cells. A range of concentrations was tested by MTT Assay as shown below. The first column corresponds to the concentration of compound (I).

| (I) (nM) | Tamoxifen (nM) | Fa | CI |
|---|---|---|---|
| 10 | 50 | 0.325758 | 0.819 |
| 10 | 100 | 0.47365 | 0.915 |

(continued)

| (I) (nM) | Tamoxifen (nM) | Fa | CI |
|---|---|---|---|
| 25 | 50 | 0.3521 | 0.900 |
| 25 | 100 | 0.4937 | 0.906 |
| 50 | 50 | 0.3715 | 1.048 |
| 50 | 100 | 0.5326 | 0.852 |
| 75 | 100 | 0.6913 | 0.505 |
| 75 | 50 | 0.4196 | 0.948 |

The MTT cell growth data was analyzed by the CalcuSyn software (Biosoft). This program uses the median-effect principle (77) to delineate the interaction between two drugs. For each dose combination, the program generates a combination index (CI). A combination index (CI) of <1, 1 or >1 denotes synergism, additivity or antagonism respectively. Figure 27 shows synergistic growth inhibitory effects with 100 nM tamoxifen + 75 nM compound (I). The CI value for this combination was calculated to be 0.505.

**Other Embodiments**

While the invention has been described in conjunction with the detailed description thereof, the foregoing description is intended to illustrate and not limit the scope of the invention, which is defined by the scope of the appended claims. Other aspects, advantages, and modifications are within the scope of the following claims. It will be understood by those skilled in the art that various changes in form and details may be made therein without departing from the scope of the invention encompassed by the appended claims.

The invention is further described with reference to the following clauses:

1. A pharmaceutical composition for oral, intravenous, intramuscular, or subcutaneous administration comprising an amount of compound (I) or a pharmaceutically acceptable salt thereof, ranging from 2 mg to 400 mg per dose administered two or three times daily and a pharmaceutically acceptable carrier.

2. The pharmaceutical composition according to clause 1 wherein the amount is from 10 mg to 300 mg.

3. The pharmaceutical composition according to clause 2 wherein the amount is from 20 mg to 250 mg.

4. The pharmaceutical composition according to clause 3 wherein the amount is from 40 mg to 200 mg.

5. The pharmaceutical composition according to clause 4 wherein the amount is from 60 mg to 160 mg.

6. A pharmaceutical composition for oral, intravenous, intramuscular, or subcutaneous administration comprising an amount of compound (I) or a pharmaceutically acceptable salt thereof ranging from 4 mg to 800 mg per dose administered once daily and a pharmaceutically acceptable carrier.

7. The pharmaceutical composition according to clause 6 wherein the amount is from 20 mg to 600 mg.

8. The pharmaceutical composition according to clause 7 wherein the amount is from 40 mg to 500 mg.

9. The pharmaceutical composition according to clause 8 wherein the amount is from 80 mg to 400 mg.

10. The pharmaceutical composition according to clause 9 wherein the amount is from 120 mg to 320 mg.

11. The pharmaceutical composition according to any one of clauses 1-10, wherein the composition comprises the mesylate salt of compound (I).

12. The pharmaceutical composition according to any one of clauses 1-11, wherein the administration is oral.

13. The pharmaceutical composition according to any one of clauses 1-11, wherein the administration is intravenous.

14. The pharmaceutical composition according to any one of clauses 1-11, wherein the administration is intramuscular.

15. The pharmaceutical composition according to any one of clauses 1-11, wherein the administration is subcutaneous.

16. The pharmaceutical composition according to any one of clauses 1-5 or 11-16, wherein the dose is administered two times daily.

17. The pharmaceutical composition according to any one of clauses 1-5 or 11-16, wherein the dose is administered three times daily.

18. The pharmaceutical composition according to any one of clauses 1-17, wherein the composition is administered in combination with one or more anti-cancer treatments or anti-cancer agents.

19. The pharmaceutical composition according to clause 18 wherein the composition is administered in combination with the anti-cancer agent gemcitabine.

20. The pharmaceutical composition according to clause 18 wherein the composition is administered in combination with the anti-cancer agent oxaliplatin.

21. A method of treating or preventing a condition or disorder selected from cancer, cell proliferative disorder, microbial infection, hyperproliferative disorder, macular edema, osteoporosis, cardiovascular disorder, eye disease, immune system disfunction, type II diabetes, obesity, transplant rejection, hearing loss, stroke, athrosclerosis, chronic neuropathic pain, hepatitis B, and autoimmune disease comprising administering the pharmaceutical composition according to any one of clauses 1-20.

22. The method according to clause 21 wherein said condition or disorder is cancer.

23. The method according to clause 22 wherein the cancer is selected from renal, prostate, liver, lung, pancreatic, brain, breast, colon, leukemia, ovarian, epithelial, and esophageal.

24. The method according to clause 22 wherein the cancer is selected from an advanced malignancy, a solid tumor, and lymphoma.

25. The method according to clause 21 wherein the condition or disorder is a cell proliferative disorder.

26. The method according to clause 25 wherein the cell proliferative disorder is selected from psoriasis, diabetic retinopathy, and macular degeneration.

27. The method according to clause 21 wherein the condition or disorder is a microbial infection.

28. The method according to clause 27 wherein the microbial infection is selected from bacterial, fungal, parasitic, and viral.

29. The method according to clause 21 wherein the disorder or condition is selected from hyperproliferative disorder, macular edema, osteoporosis, cardiovascular disorder, eye disease, immune system disfunction, type II diabetes, obesity, transplant rejection, hearing loss, stroke, athrosclerosis, chronic neuropathic pain, hepatitis B, and autoimmune disease.

30. A method of regulating immune system activity comprising administering the pharmaceutical composition according to any one of clauses 1-20.

## Claims

**1.** A combination of

(a) a pharmaceutical composition comprising

(i) compound (I):

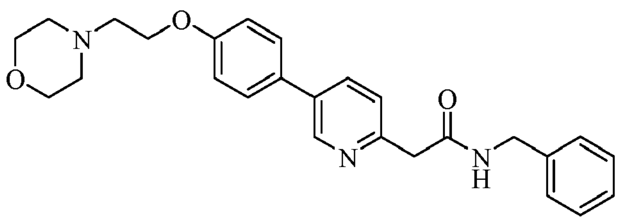

or a pharmaceutically acceptable salt thereof, and
(ii) a pharmaceutically acceptable carrier,
wherein the compound is present in an amount ranging from 10 mg to 300 mg per dose, and wherein the composition is formulated for oral, intravenous, intramuscular, or subcutaneous administration two or three times daily;

and
(b) oxaliplatin;

for simultaneous or sequential use in the treatment of a cancer or cell proliferation disorder.

**2.** The combination for use according to claim 1, wherein the amount is from 20 mg to 250 mg.

**3.** The combination for use according to claim 2, wherein the amount is from 40 mg to 200 mg.

**4.** The combination for use according to claim 3, wherein the amount is 40 mg.

**5.** The combination for use according to any one of claims 1-4, wherein the pharmaceutical composition is for administration two times daily.

**6.** A combination of

(a) a pharmaceutical composition comprising

(i) compound (I):

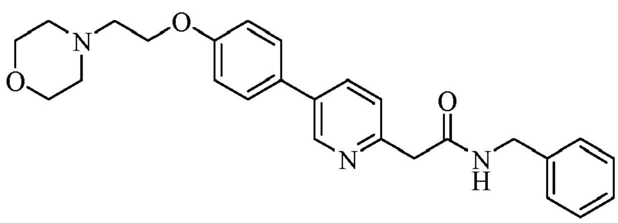

or a pharmaceutically acceptable salt thereof, and
(ii) a pharmaceutically acceptable carrier,
wherein the compound is present in an amount ranging from 20 mg to 600 mg per dose, and wherein the composition is formulated for oral, intravenous, intramuscular, or subcutaneous administration once daily;

and
(b) oxaliplatin;

for simultaneous or sequential use in the treatment of a cancer or cell proliferation disorder.

**7.** The combination for use according to claim 6, wherein the amount is from 40 mg to 500 mg.

**8.** The combination for use according to claim 7, wherein the amount is from 80 mg to 400 mg.

**9.** The combination for use according to claim 6, wherein the amount is 80 mg.

**10.** The combination for use according to any one of claims 1-9, wherein the pharmaceutical composition comprises the mesylate salt of compound (I).

**11.** The combination for use according to any one of claims 1-10, wherein said cancer or cell proliferation disorder is cancer selected from renal cancer, prostate cancer, liver cancer, lung cancer, pancreatic cancer, brain cancer, breast cancer, colon cancer, leukemia, ovarian cancer, epithelial cancer, esophageal cancer, an advanced malignancy, a solid tumor, and lymphoma.

**12.** The combination for use according to claim 11, wherein the cancer is leukemia.

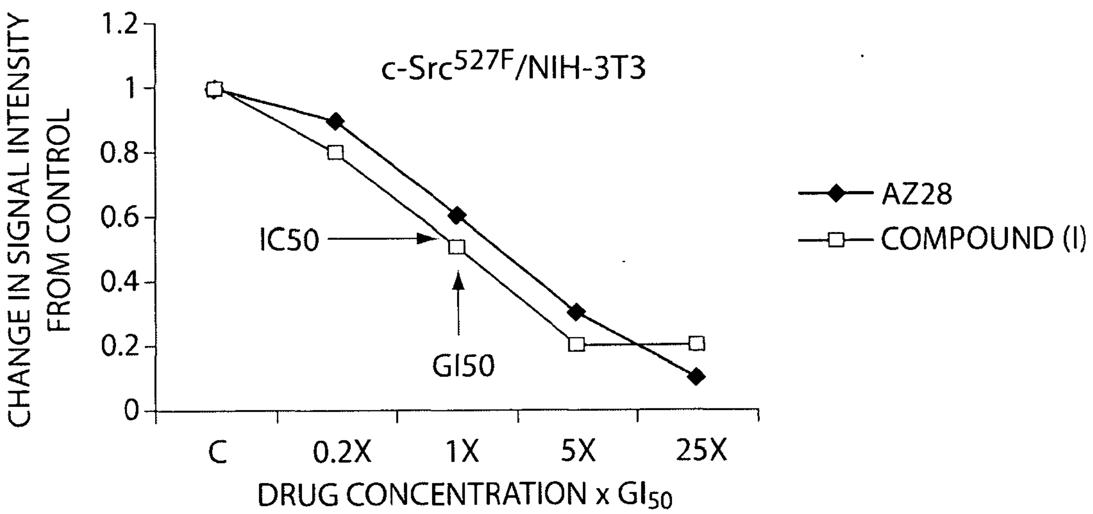
CHANGE IN SIGNAL INTENSITY
FROM CONTROL
1.2
1
0.8
0.6
0.4
0.2
0
c-Src527F/NIH-3T3
IC50
GI50
C
0.2X
1X
5X
25X
DRUG CONCENTRATION x GI50
AZ28
COMPOUND (I)

Fig. 1A

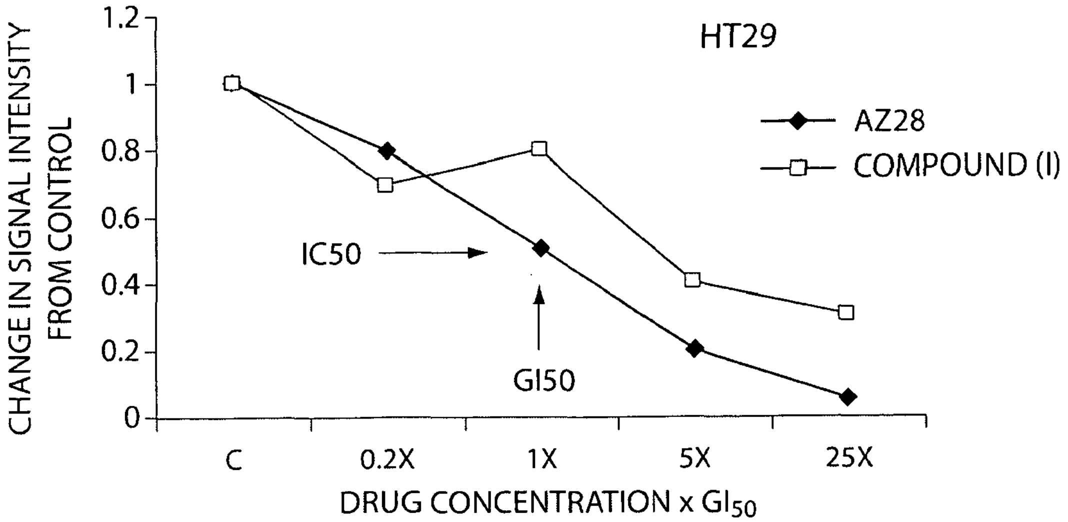
CHANGE IN SIGNAL INTENSITY
FROM CONTROL
1.2
1
0.8
0.6
0.4
0.2
0
HT29
IC50
GI50
C
0.2X
1X
5X
25X
DRUG CONCENTRATION x GI50
AZ28
COMPOUND (I)

Fig. 1B

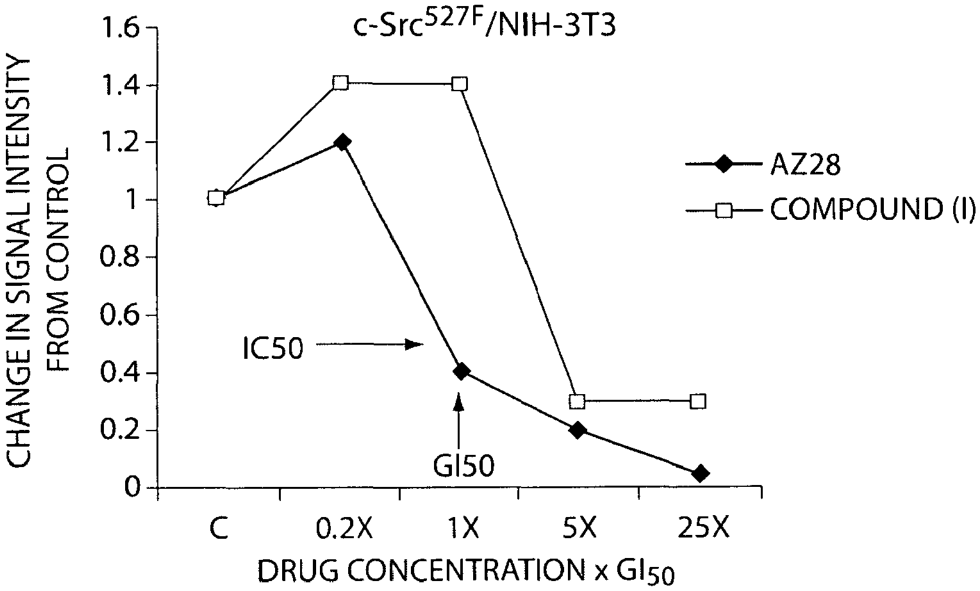
c-Src527F/NIH-3T3
CHANGE IN SIGNAL INTENSITY FROM CONTROL
1.6
1.4
1.2
1
0.8
0.6
0.4
0.2
0
AZ28
COMPOUND (I)
IC50
GI50
C
0.2X
1X
5X
25X
DRUG CONCENTRATION x GI50

Fig. 2A

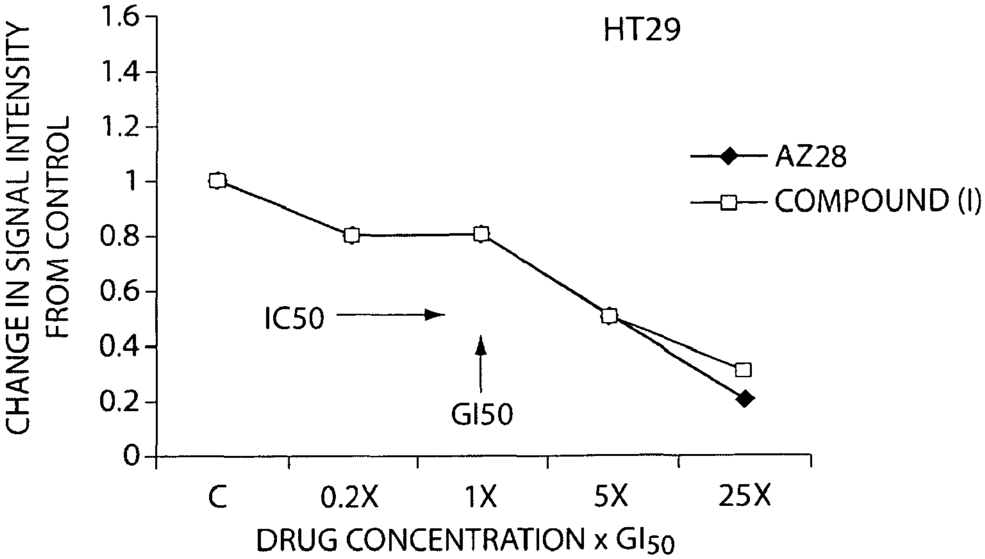
HT29
CHANGE IN SIGNAL INTENSITY FROM CONTROL
1.6
1.4
1.2
1
0.8
0.6
0.4
0.2
0
AZ28
COMPOUND (I)
IC50
GI50
C
0.2X
1X
5X
25X
DRUG CONCENTRATION x GI50

Fig. 2B

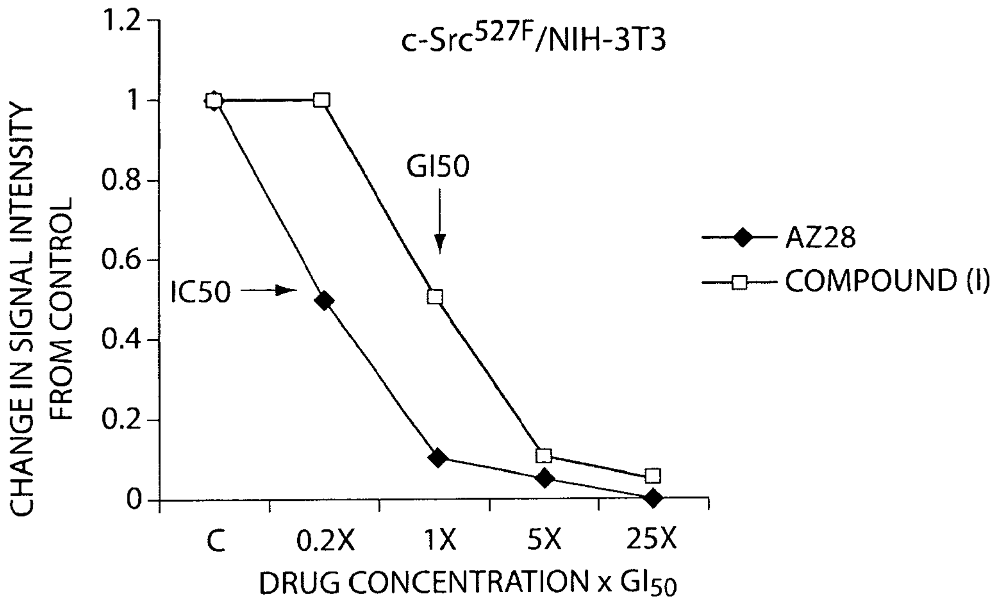
c-Src527F/NIH-3T3
CHANGE IN SIGNAL INTENSITY FROM CONTROL
1.2
1
0.8
0.6
0.4
0.2
0
GI50
IC50
C
0.2X
1X
5X
25X
DRUG CONCENTRATION x GI50
AZ28
COMPOUND (I)

Fig. 3A

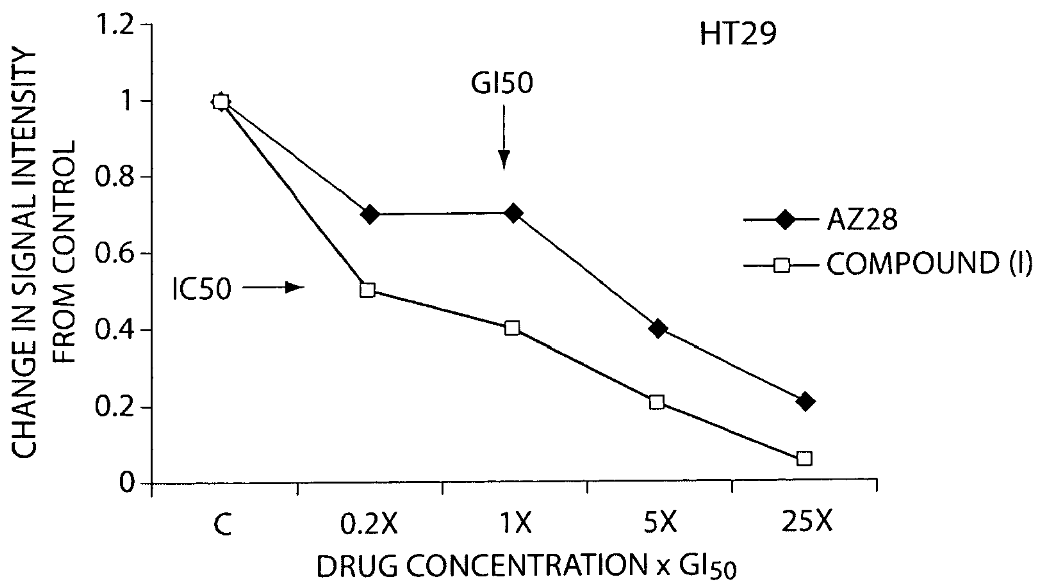
HT29
CHANGE IN SIGNAL INTENSITY FROM CONTROL
1.2
1
0.8
0.6
0.4
0.2
0
GI50
IC50
C
0.2X
1X
5X
25X
DRUG CONCENTRATION x GI50
AZ28
COMPOUND (I)

Fig. 3B

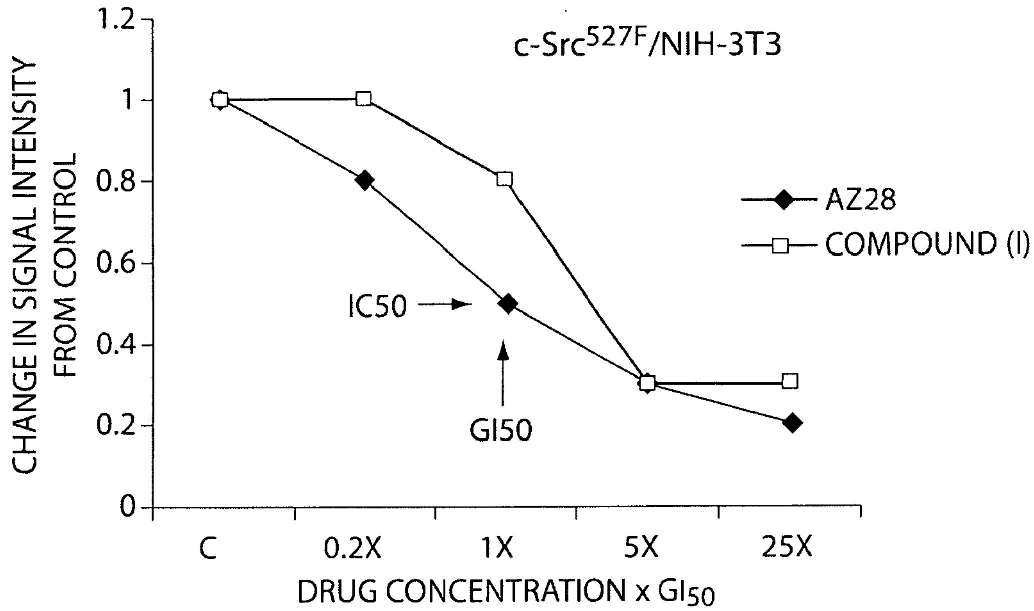

c-Src527F/NIH-3T3
CHANGE IN SIGNAL INTENSITY FROM CONTROL
1.2
1
0.8
0.6
0.4
0.2
0
AZ28
COMPOUND (I)
IC50
GI50
C
0.2X
1X
5X
25X
DRUG CONCENTRATION x GI50


Fig. 4

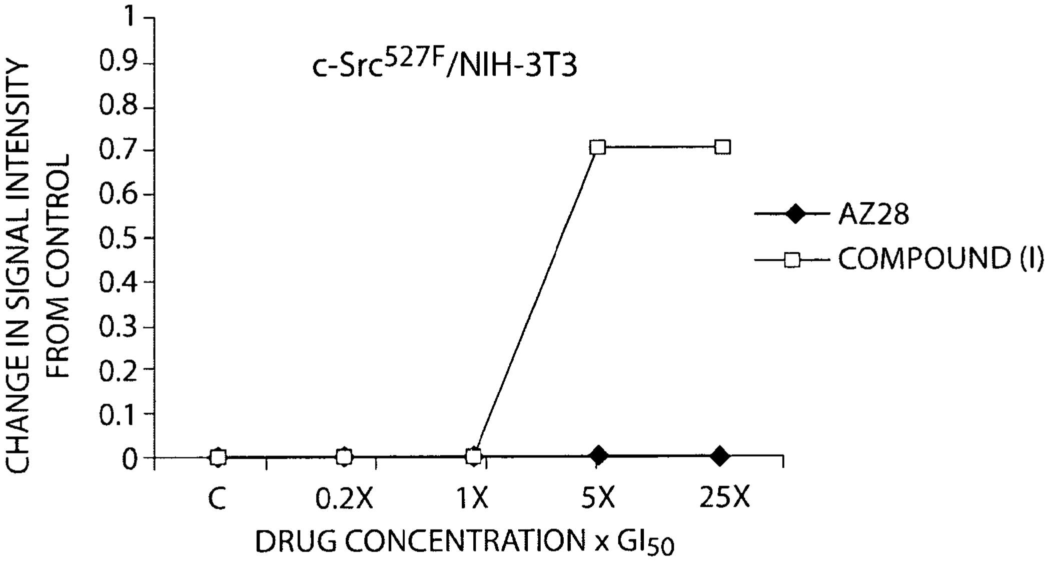

c-Src527F/NIH-3T3
CHANGE IN SIGNAL INTENSITY FROM CONTROL
1
0.9
0.8
0.7
0.6
0.5
0.4
0.3
0.2
0.1
0
C
0.2X
1X
5X
25X
DRUG CONCENTRATION x GI50
AZ28
COMPOUND (I)


Fig. 5A

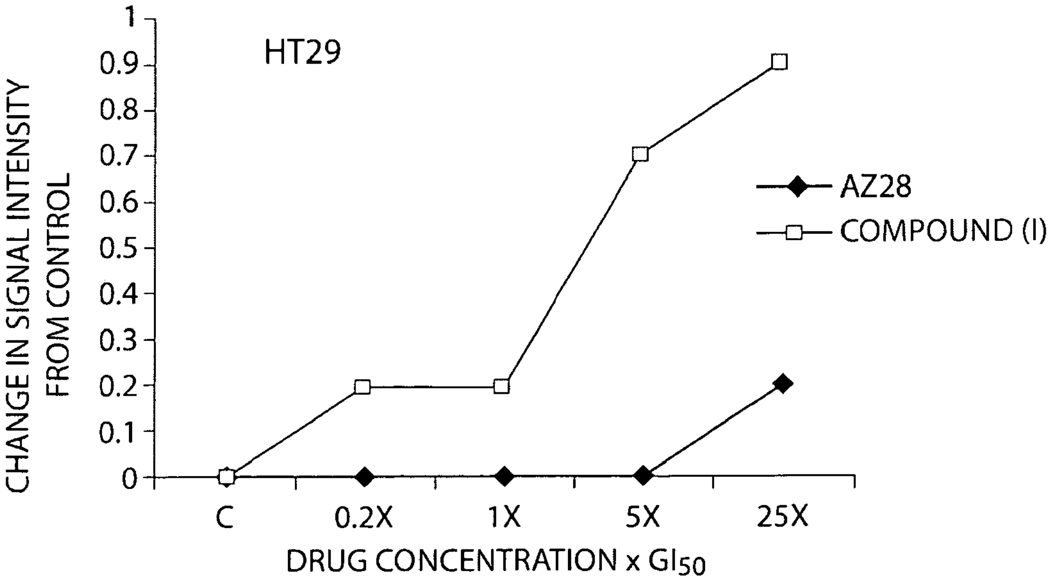

HT29
CHANGE IN SIGNAL INTENSITY FROM CONTROL
1
0.9
0.8
0.7
0.6
0.5
0.4
0.3
0.2
0.1
0
C
0.2X
1X
5X
25X
DRUG CONCENTRATION x GI50
AZ28
COMPOUND (I)


Fig. 5B

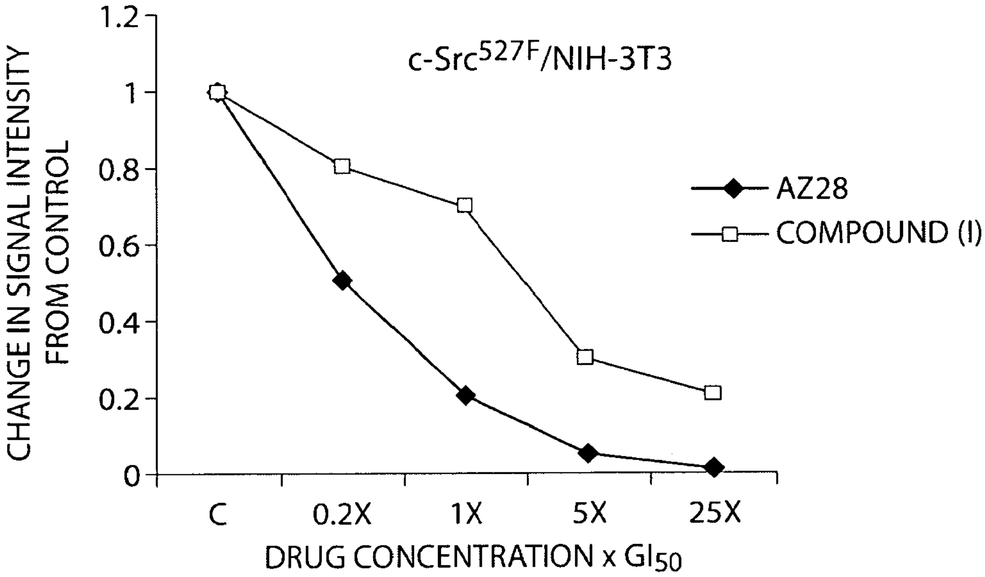
c-Src527F/NIH-3T3
CHANGE IN SIGNAL INTENSITY FROM CONTROL
1.2
1
0.8
0.6
0.4
0.2
0
AZ28
COMPOUND (I)
C
0.2X
1X
5X
25X
DRUG CONCENTRATION x GI50

Fig. 6A

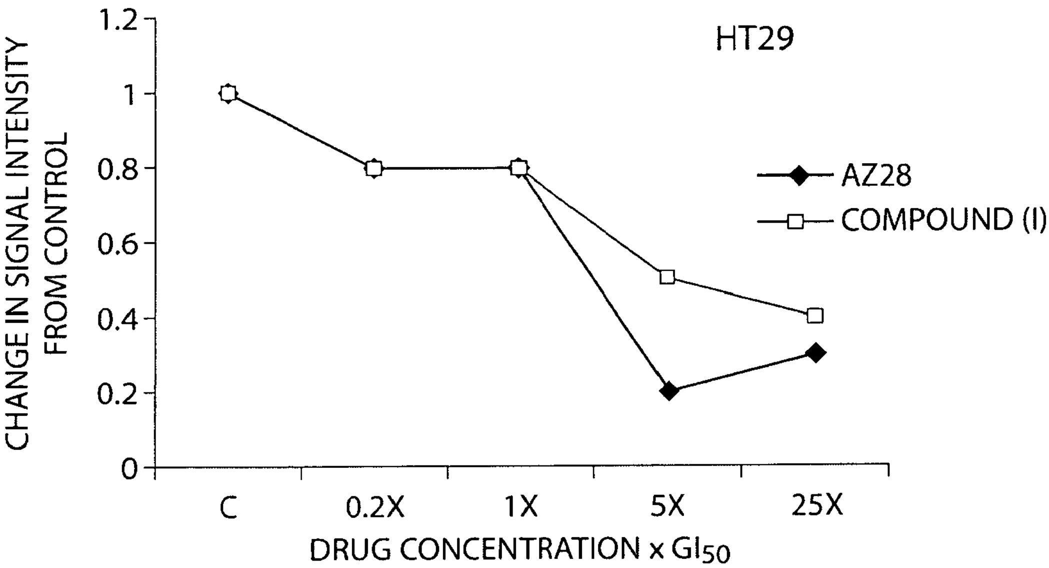
HT29
CHANGE IN SIGNAL INTENSITY FROM CONTROL
1.2
1
0.8
0.6
0.4
0.2
0
AZ28
COMPOUND (I)
C
0.2X
1X
5X
25X
DRUG CONCENTRATION x GI50

Fig. 6B

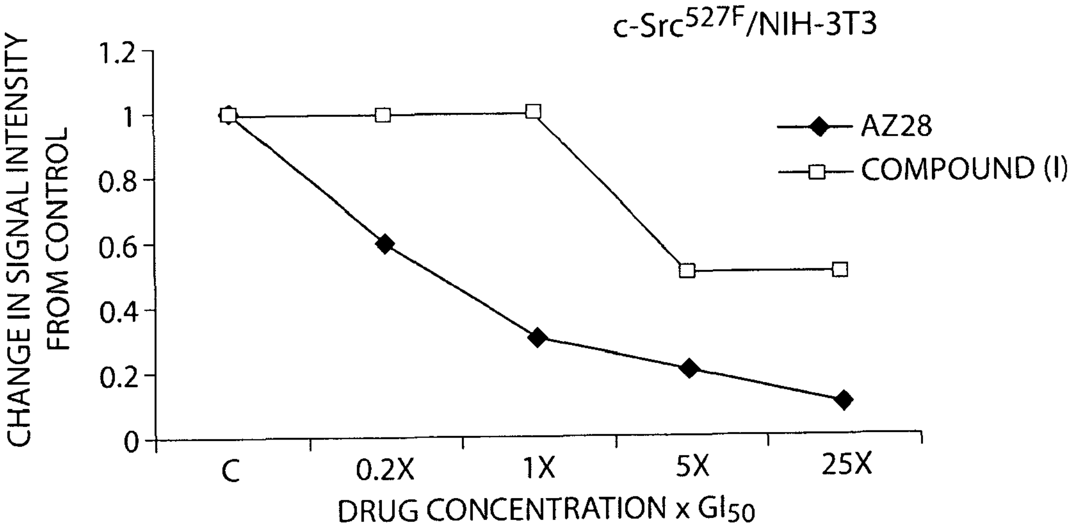
c-Src527F/NIH-3T3
CHANGE IN SIGNAL INTENSITY FROM CONTROL
1.2
1
0.8
0.6
0.4
0.2
0
AZ28
COMPOUND (I)
C
0.2X
1X
5X
25X
DRUG CONCENTRATION x GI50

Fig. 7

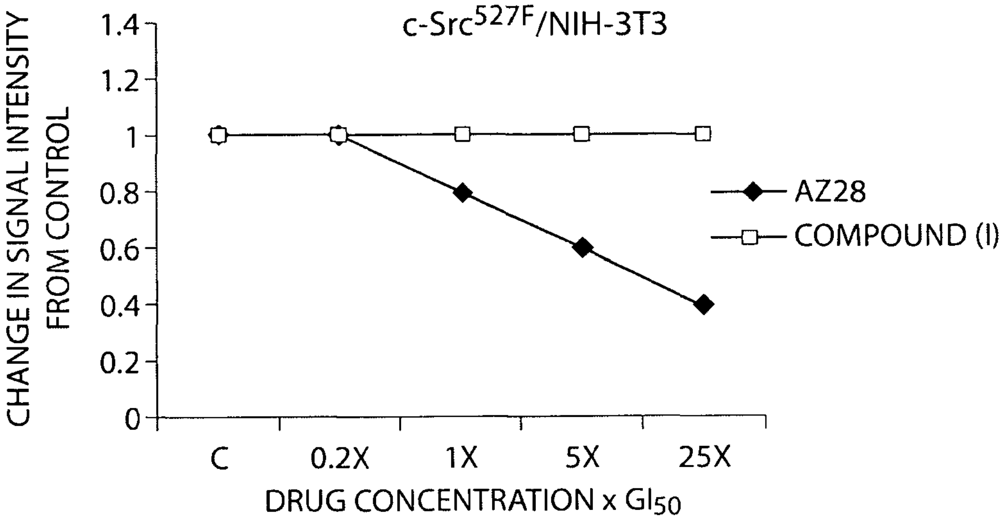
c-Src527F/NIH-3T3
CHANGE IN SIGNAL INTENSITY FROM CONTROL
1.4
1.2
1
0.8
0.6
0.4
0.2
0
C
0.2X
1X
5X
25X
DRUG CONCENTRATION x GI50
AZ28
COMPOUND (I)

Fig. 8A

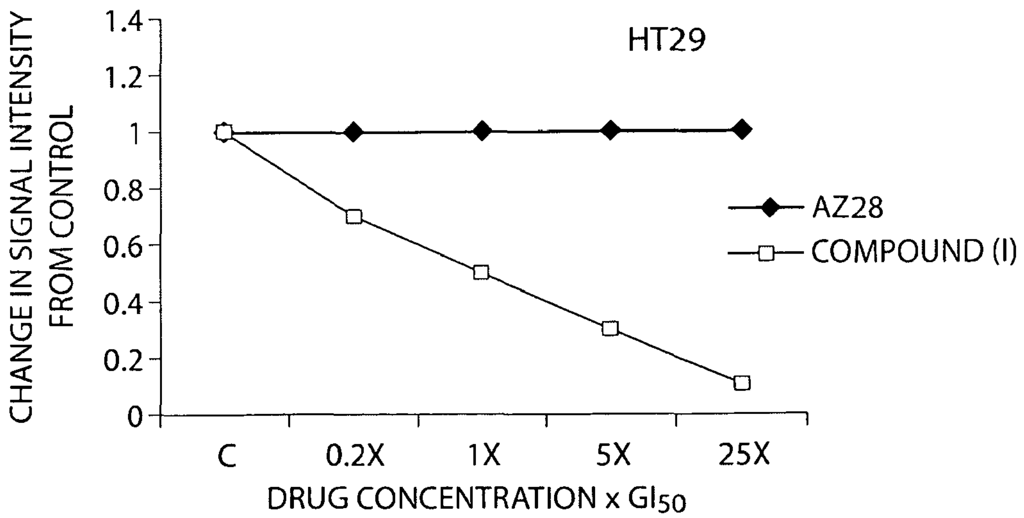
HT29
CHANGE IN SIGNAL INTENSITY FROM CONTROL
1.4
1.2
1
0.8
0.6
0.4
0.2
0
C
0.2X
1X
5X
25X
DRUG CONCENTRATION x GI50
AZ28
COMPOUND (I)

Fig. 8B

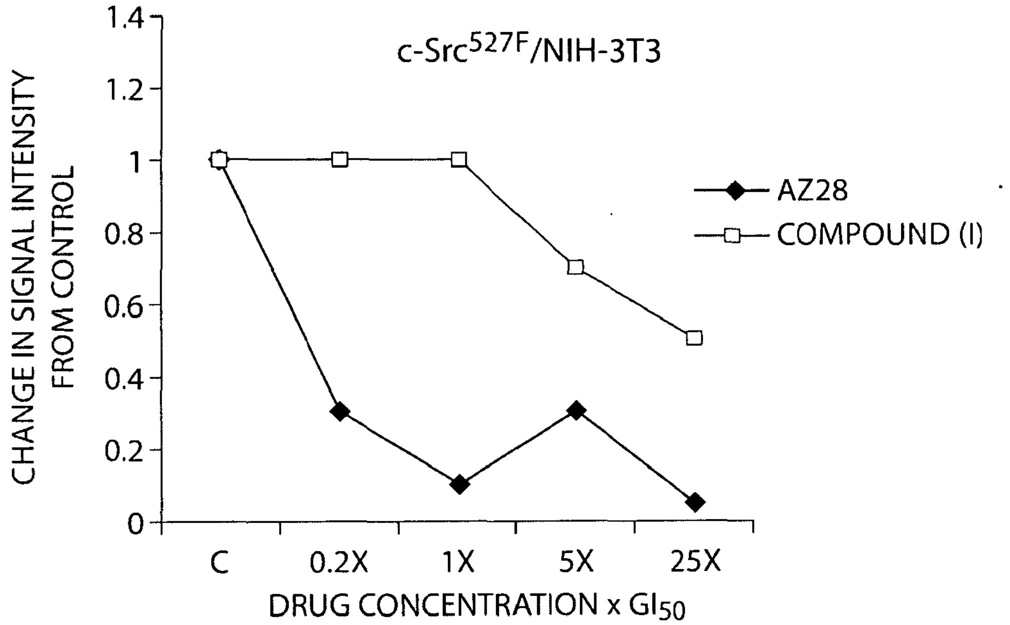
c-Src527F/NIH-3T3
CHANGE IN SIGNAL INTENSITY FROM CONTROL
1.4
1.2
1
0.8
0.6
0.4
0.2
0
AZ28
COMPOUND (I)
C
0.2X
1X
5X
25X
DRUG CONCENTRATION x GI50

Fig. 9A

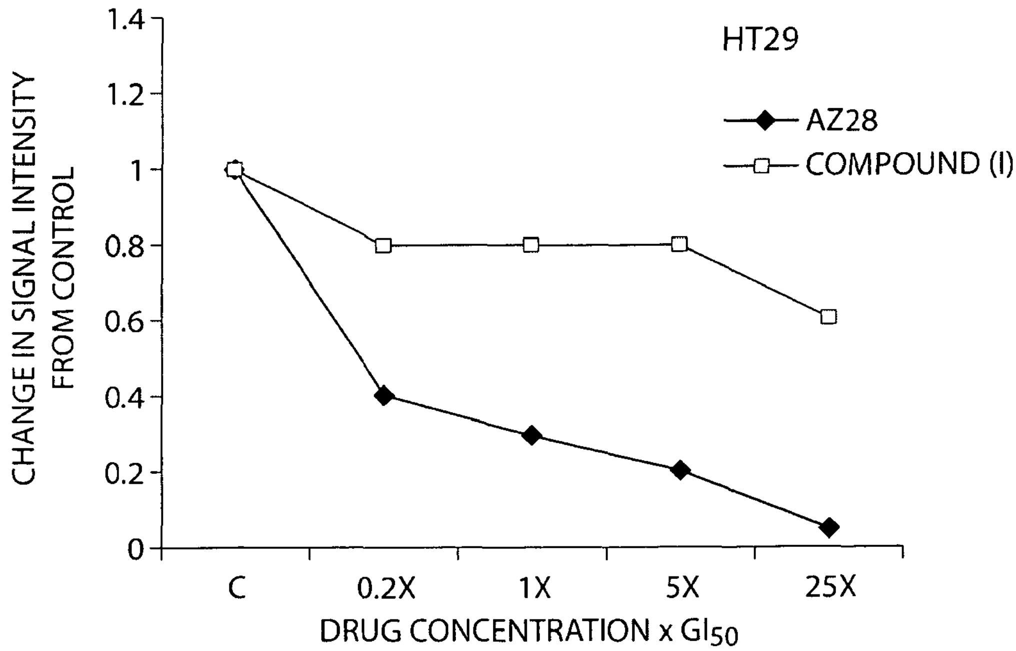
HT29
CHANGE IN SIGNAL INTENSITY FROM CONTROL
1.4
1.2
1
0.8
0.6
0.4
0.2
0
AZ28
COMPOUND (I)
C
0.2X
1X
5X
25X
DRUG CONCENTRATION x GI50

Fig. 9B

Src Autophosphorylation (Y416)
Inhibition Correlates with $GI_{50}$'s

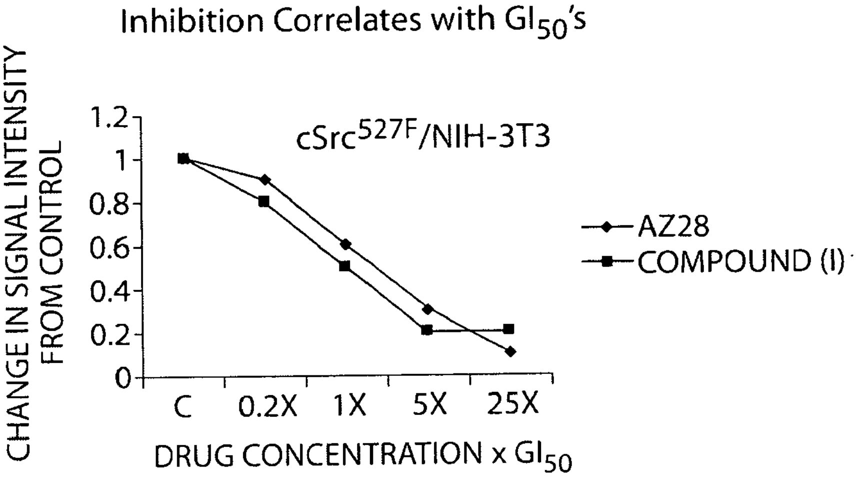


Fig. 10A

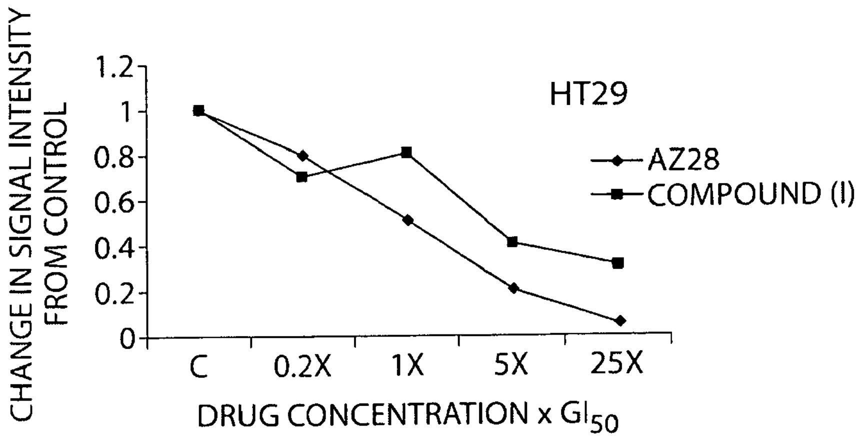


Fig. 10B

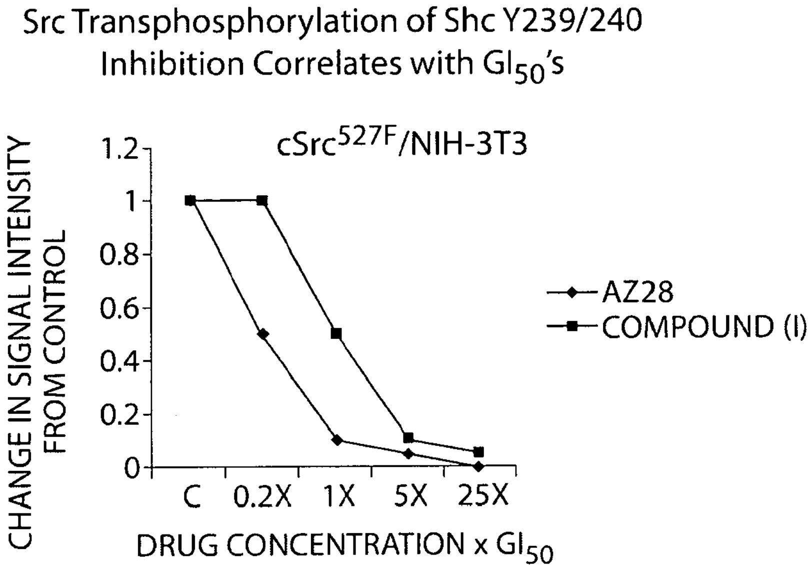


Fig. 10C

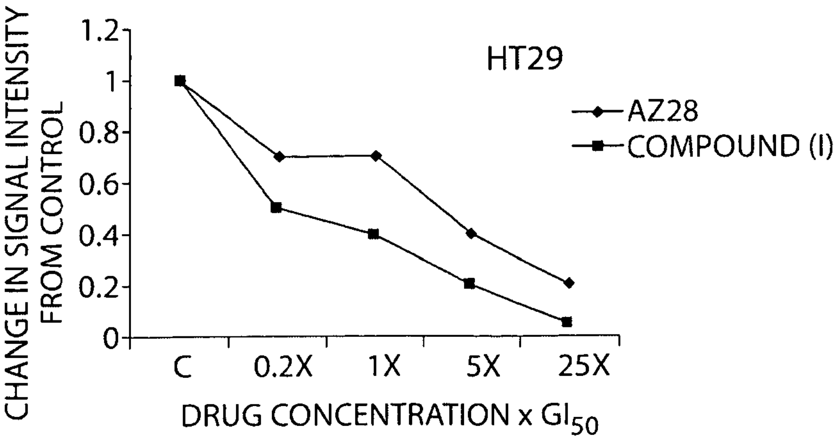


Fig. 10D

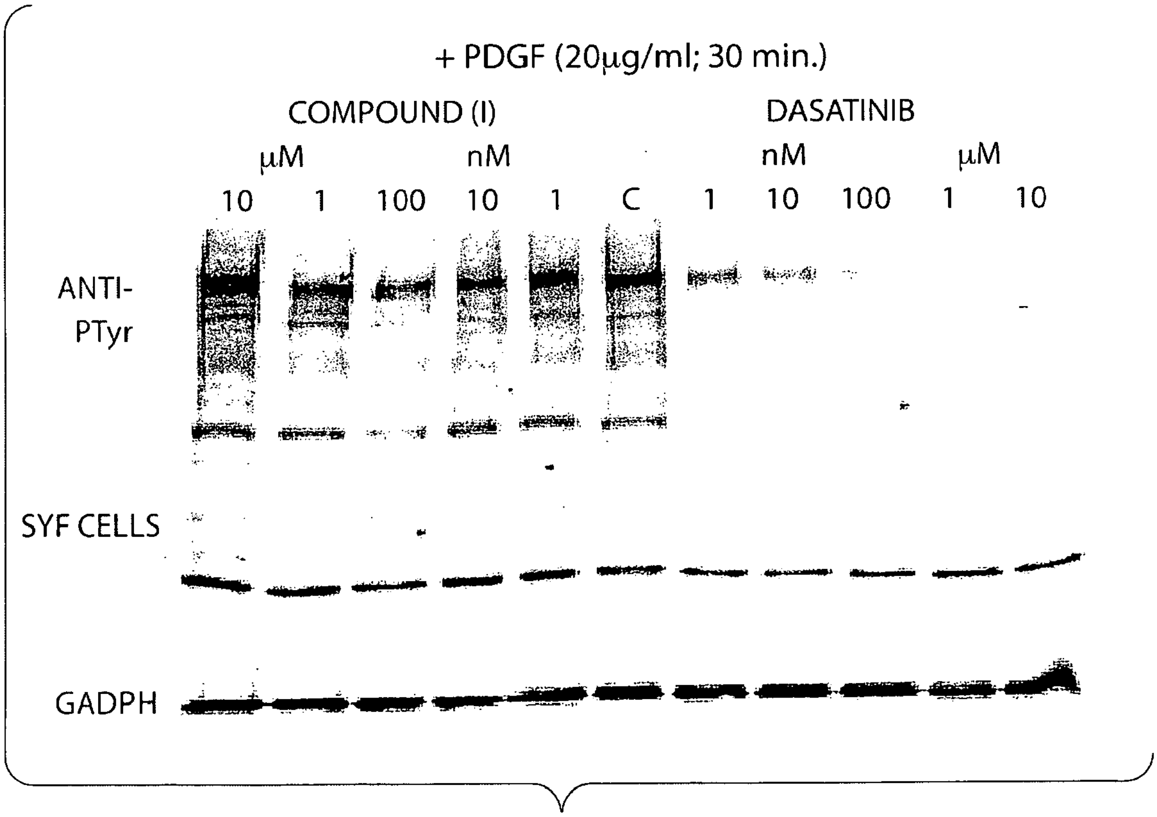
+ PDGF (20μg/ml; 30 min.)
COMPOUND (I)
DASATINIB
μM
nM
nM
μM
10 1 100 10 1 C 1 10 100 1 10
ANTI-PTyr
SYF CELLS
GADPH

Fig. 11

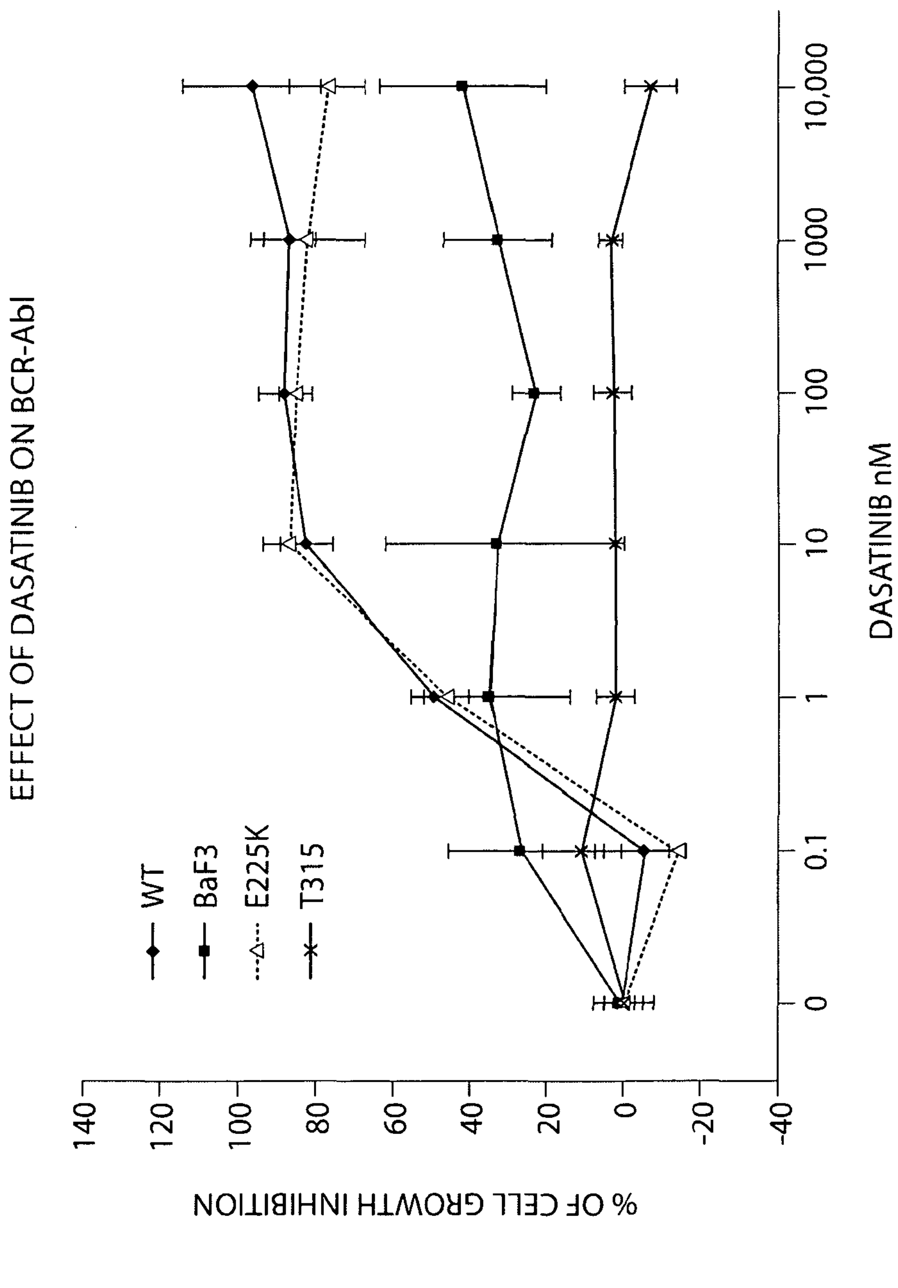
EFFECT OF DASATINIB ON BCR-Abl
WT
BaF3
E225K
T315
% OF CELL GROWTH INHIBITION
140
120
100
80
60
40
20
0
-20
-40
0
0.1
1
10
100
1000
10,000
DASATINIB nM

Fig. 12

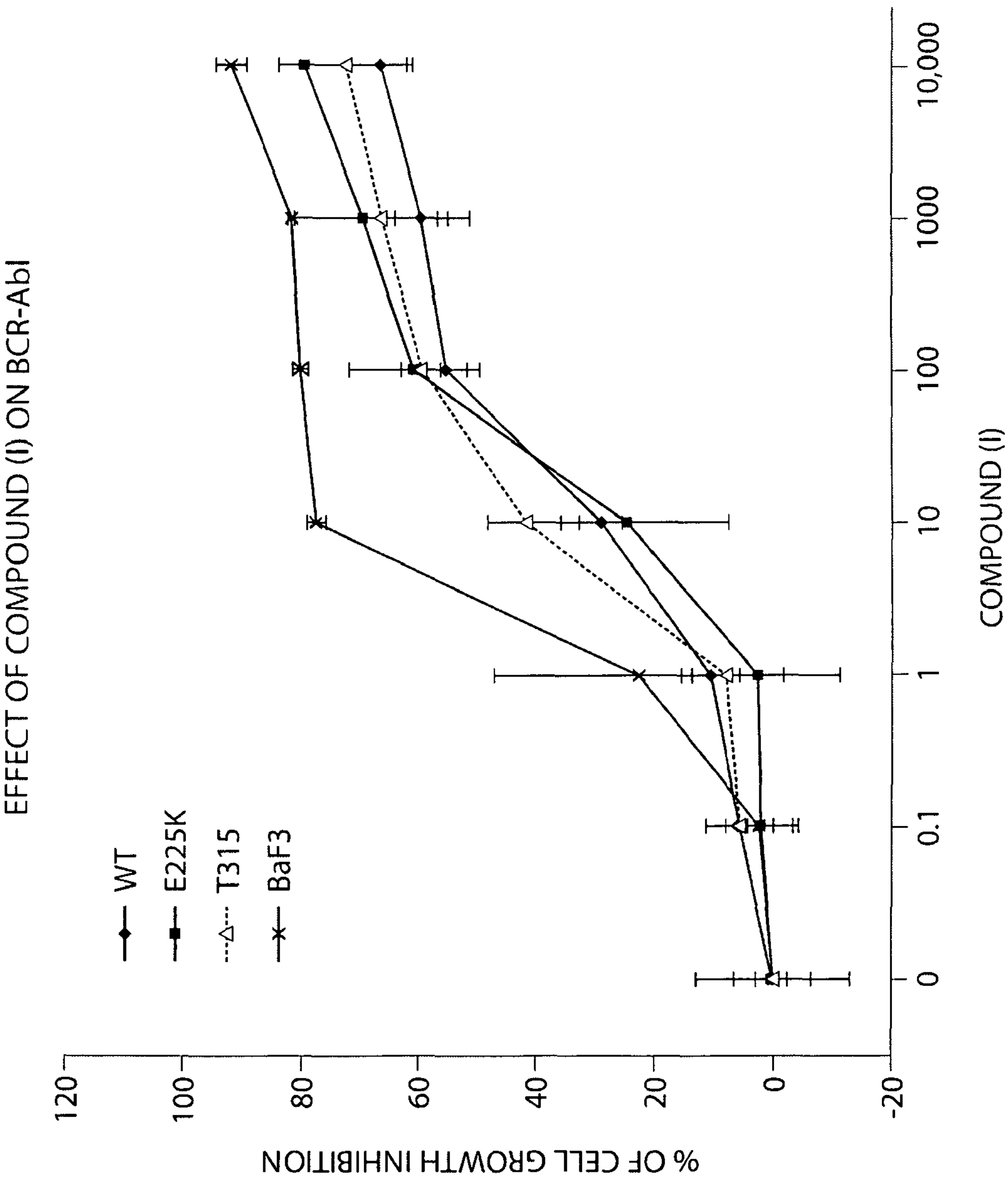
EFFECT OF COMPOUND (I) ON BCR-Abl
WT
E225K
T315
BaF3
% OF CELL GROWTH INHIBITION
120
100
80
60
40
20
0
-20
0
0.1
1
10
100
1000
10,000
COMPOUND (I)

Fig. 13

HT-29 COMPOUND (I)

| COMPOUND (I) | | GI50 nM |
|---|---|---|
| nM | %G | 15.44 |
| 128 | 0.00 | |
| 64 | 0.00 | |
| 32 | 0.00 | |
| 16 | 34.65 | |
| 8 | 112.84 | |
| 4 | 184.40 | |
| 2 | 166.43 | |
| 1 | 141.32 | |

#Ok
#Ok

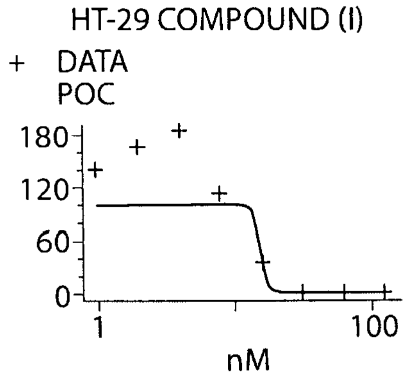


HT-29 BMS354825

| BMS354825 | | GI50 nM |
|---|---|---|
| nM | %G | 20.47 |
| 128 | 36.77 | |
| 64 | 35.55 | |
| 32 | 49.09 | |
| 16 | 41.02 | |
| 8 | 60.86 | |
| 4 | 75.88 | |
| 2 | 70.91 | |
| 1 | 72.24 | |

#Ok
#Ok

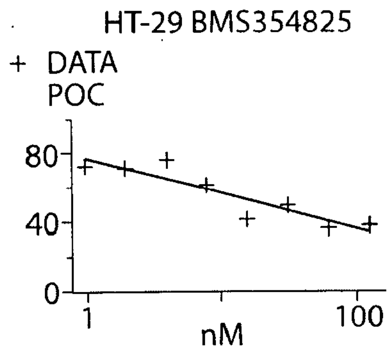


Fig. 14

SKOV-3 COMPOUND (I)

| COMPOUND (I) | | GI50 nM |
|---|---|---|
| nM | %G | 9.75 |
| 128 | 0.00 | |
| 64 | 0.00 | |
| 32 | 0.00 | |
| 16 | 0.45 | |
| 8 | 89.79 | |
| 4 | 101.54 | |
| 2 | 118.56 | |
| 1 | 114.65 | |

#Ok
#Ok

SKOV-3 BMS354825

| BMS354825 | | GI50 nM |
|---|---|---|
| nM | %G | 3.24 |
| 128 | 0.49 | |
| 64 | 1.25 | |
| 32 | 8.22 | |
| 16 | 24.63 | |
| 8 | 41.91 | |
| 4 | 48.62 | |
| 2 | 50.65 | |
| 1 | 73.20 | |

#Ok
#Ok

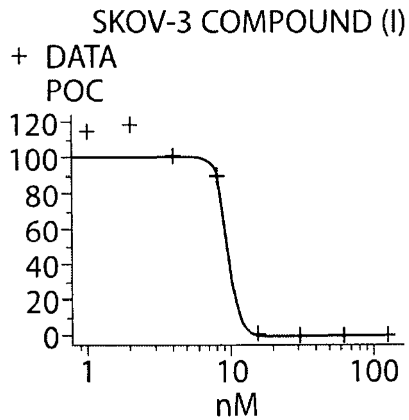


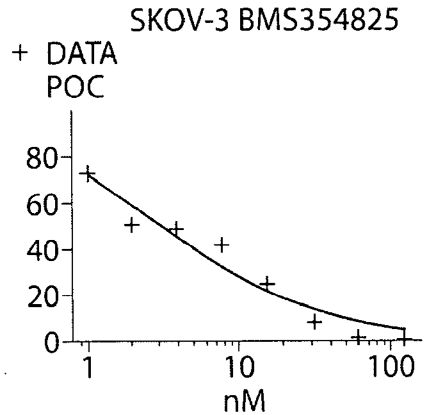


Fig. 15

A549 COMPOUND (I)

| COMPOUND (I) | | GI50 nM |
|---|---|---|
| nM | %G | 9.39 |
| 128 | 0.00 | |
| 64 | 0.00 | |
| 32 | 0.00 | |
| 16 | 0.00 | |
| 8 | 95.88 | |
| 4 | 106.28 | |
| 2 | 92.65 | |
| 1 | 117.54 | |

#Ok
#Ok

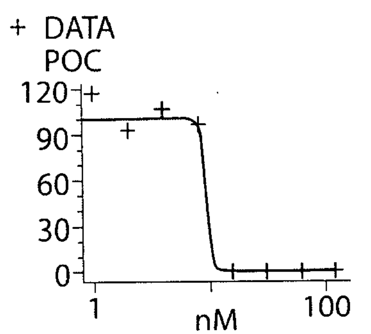


A549 BMS354825

| BMS354825 | | GI50 nM |
|---|---|---|
| nM | %G | 12.47 |
| 128 | 0.00 | |
| 64 | 0.00 | |
| 32 | 17.977 | |
| 16 | 44.93 | |
| 8 | 77.82 | |
| 4 | 82.92 | |
| 2 | 74.99 | |
| 1 | 73.46 | |

#Ok
#Ok

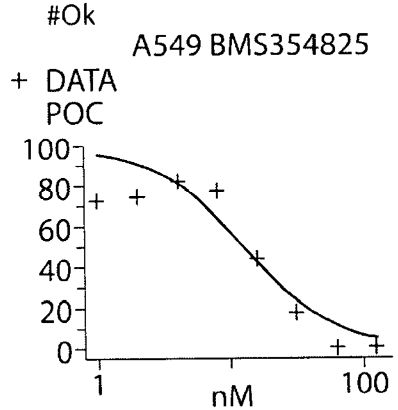


Fig. 16

K562 COMPOUND (I)

| COMPOUND (I) | | GI50 nM |
|---|---|---|
| nM | %G | 10.85 |
| 128 | 0.00 | |
| 64 | 0.00 | |
| 32 | 0.00 | |
| 16 | 6.34 | |
| 8 | 89.18 | |
| 4 | 119.03 | |
| 2 | 96.18 | |
| 1 | 119.23 | |

#Ok
#Ok

K562 COMPOUND (I)

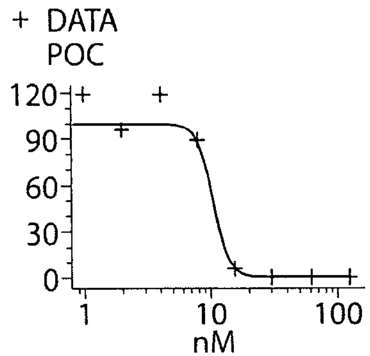


K562 BMS354825

| BMS354825 | | GI50 nM |
|---|---|---|
| nM | %G | <1nM |
| 128 | 0.00 | |
| 64 | 0.00 | |
| 32 | 0.00 | |
| 16 | 0.00 | |
| 8 | 0.00 | |
| 4 | 0.00 | |
| 2 | 0.00 | |
| 1 | 0.00 | |

#Ok
#Ok

K562 BMS354825

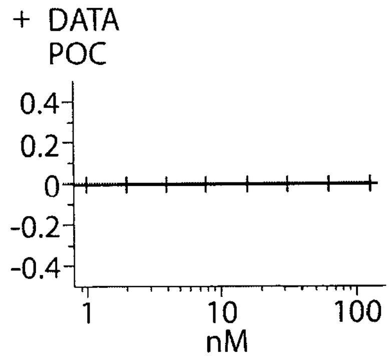


Fig. 17

MDA-MB-231 COMPOUND (I)

| COMPOUND (I) | |
|---|---|
| nM | %G |
| 128 | 0.00 |
| 64 | 0.00 |
| 32 | 8.16 |
| 16 | 74.47 |
| 8 | 88.15 |
| 4 | 81.50 |
| 2 | 98.43 |
| 1 | 111.44 |

GI50 nM
19.83

#Ok
#Ok

MDA-MB-231 BMS354825

| BMS354825 | |
|---|---|
| nM | %G |
| 128 | 0.00 |
| 64 | 17.27 |
| 32 | 36.38 |
| 16 | 34.18 |
| 8 | 46.40 |
| 4 | 54.31 |
| 2 | 58.88 |
| 1 | 83.87 |

GI50 nM
6.02

#Ok
#Ok

MDA-MB-231 COMPOUND (I)
POC

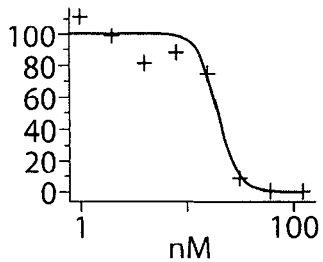


MDA-MB-231 BMS354825
POC

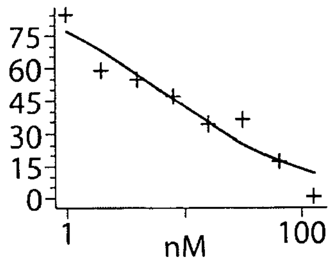


Fig. 18

COMBINED SAMPLES DATA

L3.6pl BLENDED SAMPLES

| GEMZAR | | CMPD (I) |
|---|---|---|
| nM | %G | nm |
| 8 | 0.00 | 100 |
| 4 | 0.00 | 50 |
| 2 | 0.00 | 25 |
| 1 | 0.00 | 12.5 |
| 0.5 | 0.00 | 6.25 |
| 0.25 | 10.02 | 3.12 |
| 0.125 | 32.43 | 1.56 |
| 0.0625 | 82.43 | 0.78 |

| GEMZAR | CMPD (I) |
|---|---|
| nM | nM |
| 0.09 | 1.15 |

#Ok #Ok

#Ok #Ok

L3.6pl BLENDED SAMPLES

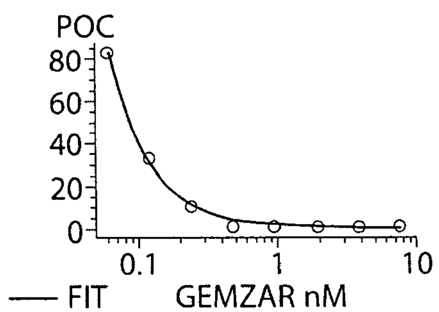


L3.6pl BLENDED SAMPLES

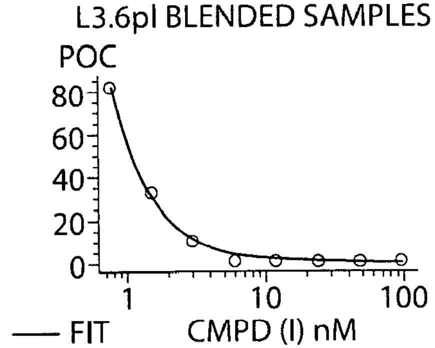


Fig. 19

SAMPLE DATA

L3.6pl GEMZAR

| GEMZAR | | GI50 nM |
|---|---|---|
| nM | %G | 1.76 |
| 8 | 0.00 | |
| 4 | 0.00 | |
| 2 | 41.78 | |
| 1 | 104.67 | |
| 0.5 | 106.93 | |
| 0.25 | 112.63 | |
| 0.125 | 107.50 | |
| 0.0625 | 106.80 | |

#Ok
#Ok

L3.6pl COMPOUND (I)

| COMPOUND (I) | | GI50 nM |
|---|---|---|
| nM | %G | 53.03 |
| 100 | 7.10 | |
| 50 | 54.21 | |
| 25 | 97.86 | |
| 12.5 | 101.70 | |
| 6.25 | 101.69 | |
| 3.12 | 96.96 | |
| 1.56 | 96.40 | |
| 0.78 | 99.89 | |

#Ok
#Ok

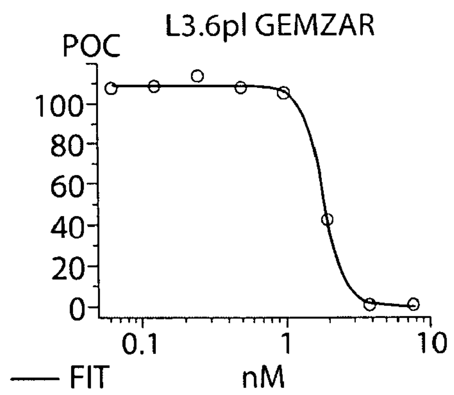


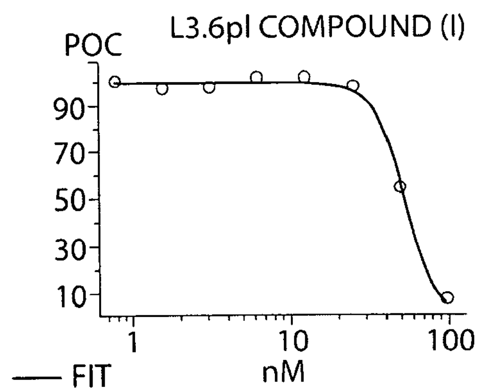


Fig. 20

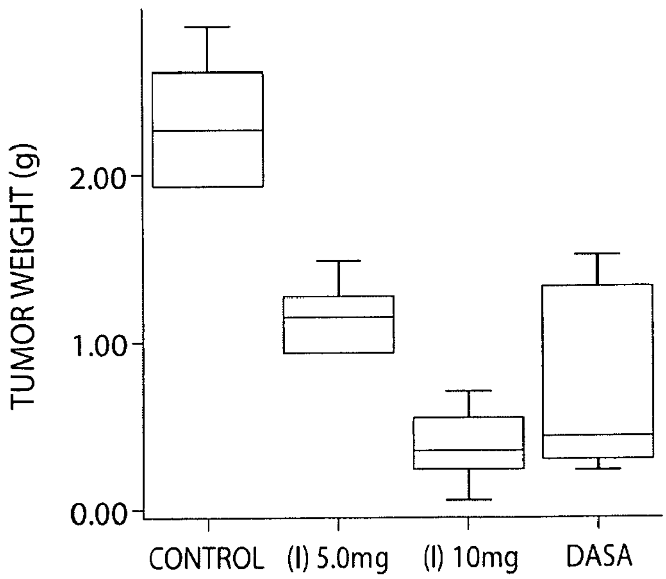


Fig. 21

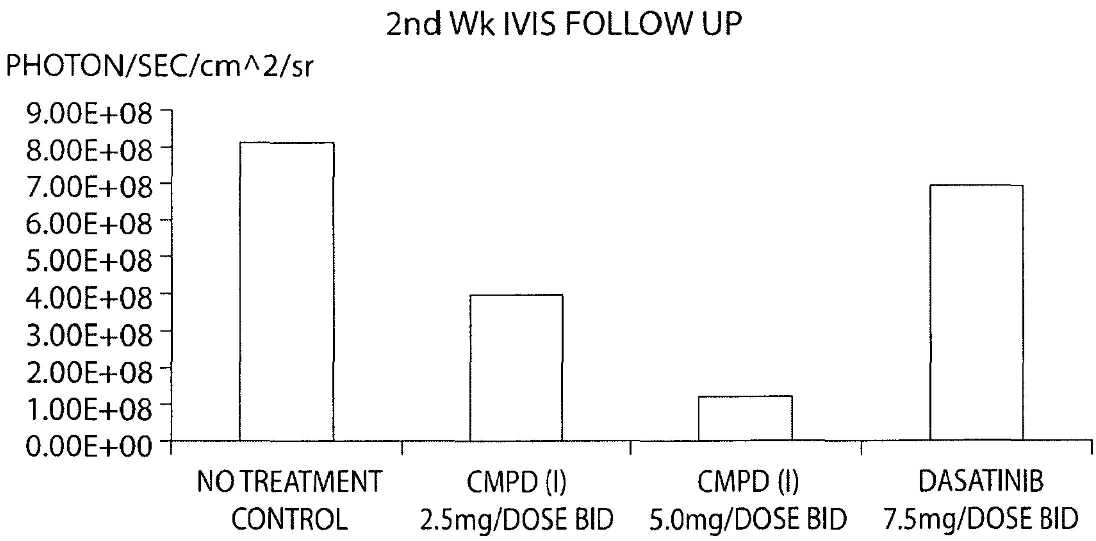
2nd Wk IVIS FOLLOW UP
PHOTON/SEC/cm^2/sr
9.00E+08
8.00E+08
7.00E+08
6.00E+08
5.00E+08
4.00E+08
3.00E+08
2.00E+08
1.00E+08
0.00E+00
NO TREATMENT CONTROL
CMPD (I) 2.5mg/DOSE BID
CMPD (I) 5.0mg/DOSE BID
DASATINIB 7.5mg/DOSE BID

Fig. 22

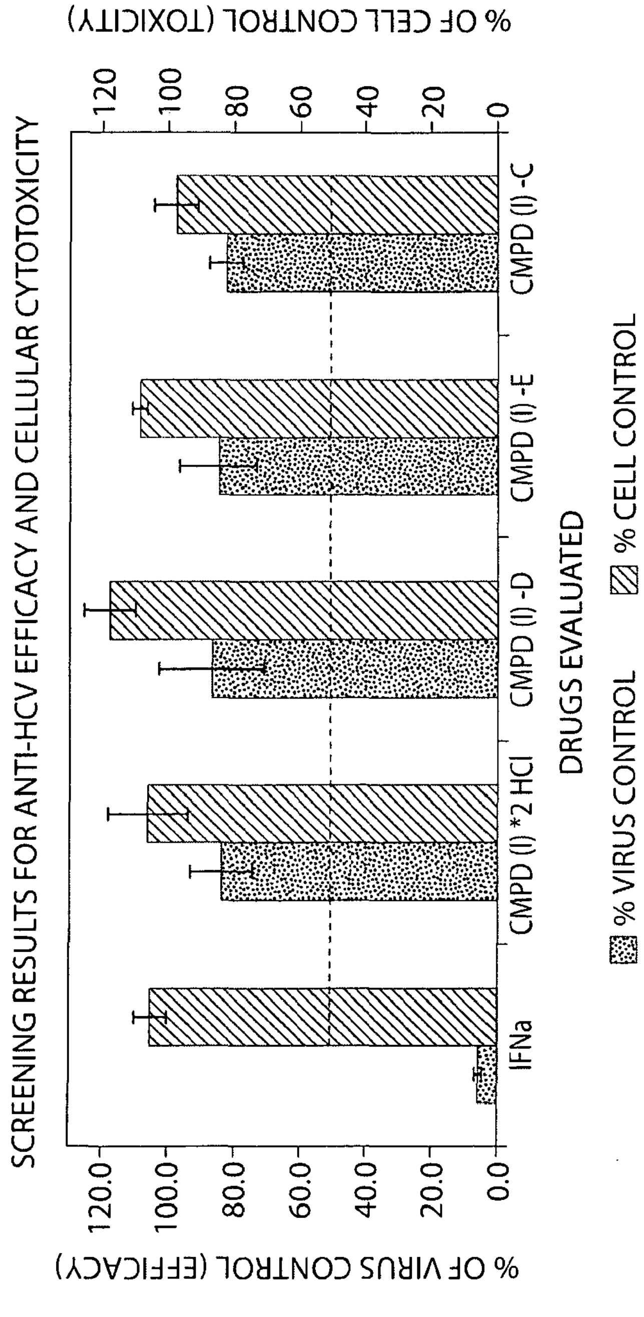
SCREENING RESULTS FOR ANTI-HCV EFFICACY AND CELLULAR CYTOTOXICITY
% OF VIRUS CONTROL (EFFICACY)
0.0
20.0
40.0
60.0
80.0
100.0
120.0
% OF CELL CONTROL (TOXICITY)
0
20
40
60
80
100
120
IFNa
CMPD (I) *2 HCl
CMPD (I) -D
CMPD (I) -E
CMPD (I) -C
DRUGS EVALUATED
% VIRUS CONTROL
% CELL CONTROL

Fig. 23

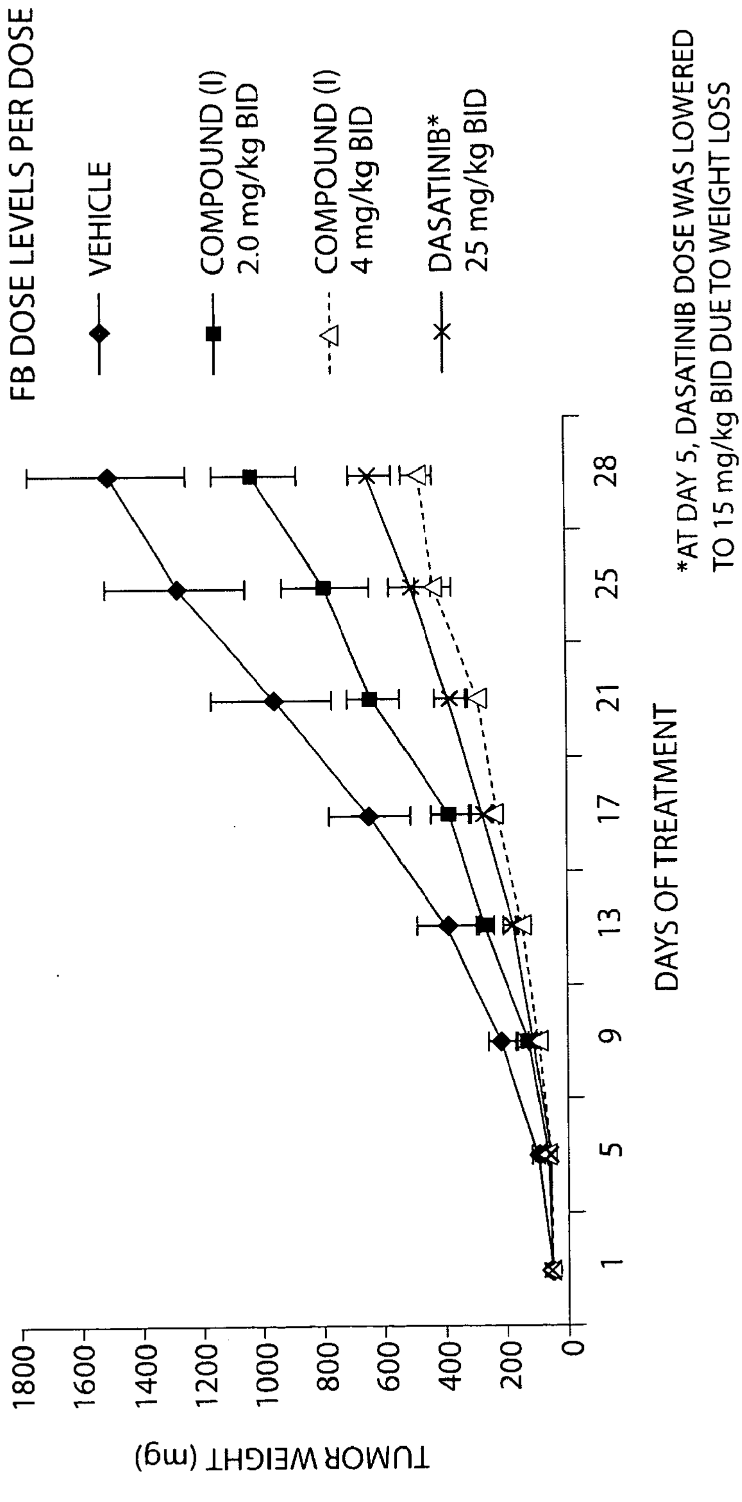

TUMOR WEIGHT (mg)
1800
1600
1400
1200
1000
800
600
400
200
0
DAYS OF TREATMENT
1
5
9
13
17
21
25
28
FB DOSE LEVELS PER DOSE
VEHICLE
COMPOUND (I) 2.0 mg/kg BID
COMPOUND (I) 4 mg/kg BID
DASATINIB* 25 mg/kg BID
*AT DAY 5, DASATINIB DOSE WAS LOWERED TO 15 mg/kg BID DUE TO WEIGHT LOSS


Fig. 24

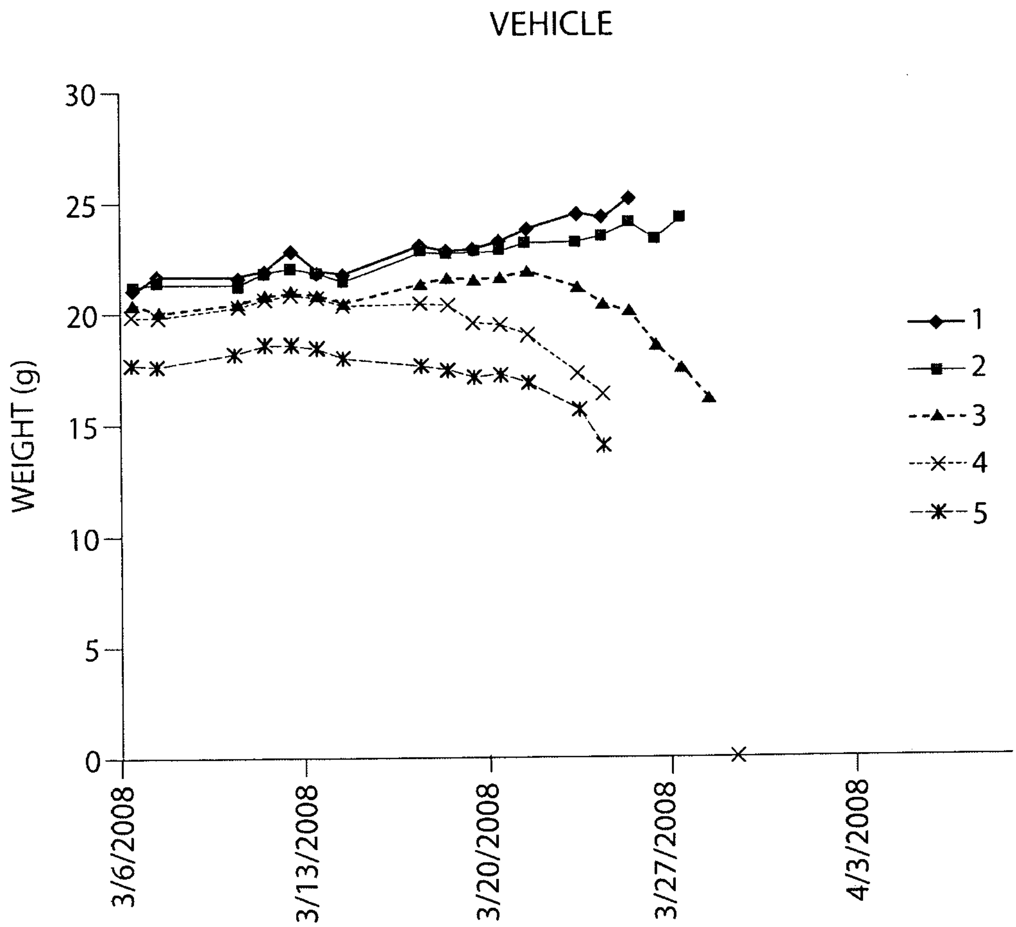
VEHICLE
WEIGHT (g)
30
25
20
15
10
5
0
3/6/2008
3/13/2008
3/20/2008
3/27/2008
4/3/2008
1
2
3
4
5

Fig. 25A

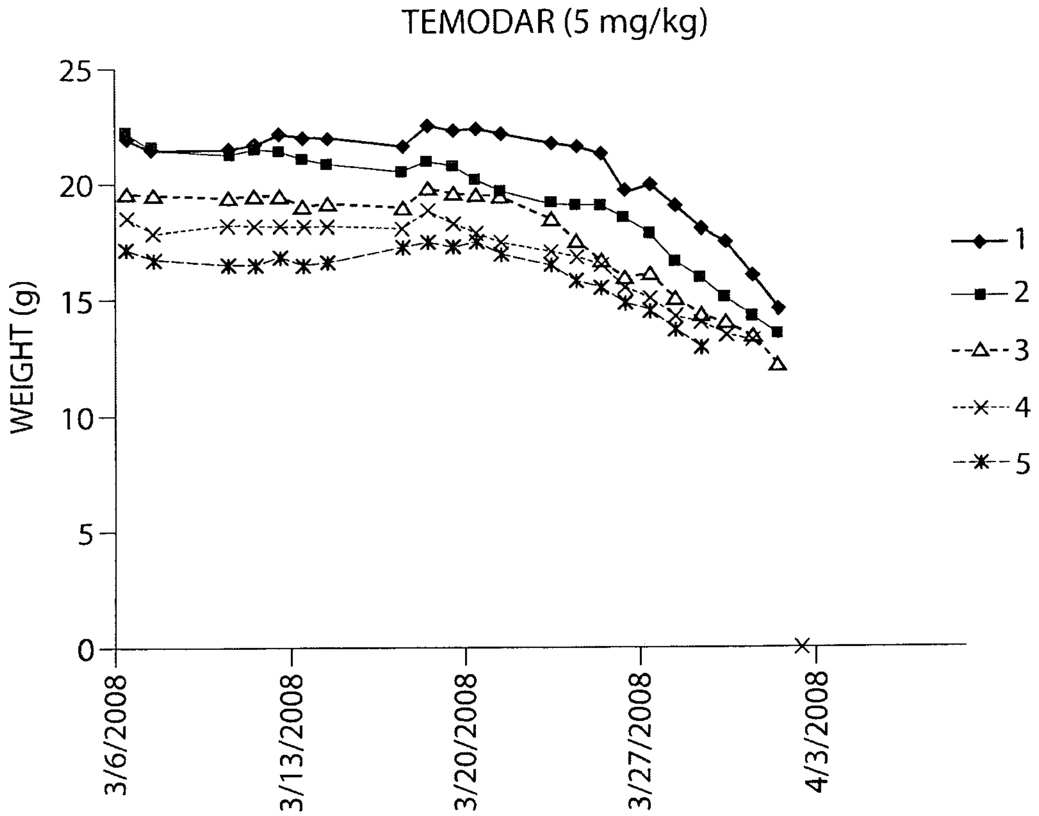
TEMODAR (5 mg/kg)
WEIGHT (g)
25
20
15
10
5
0
3/6/2008
3/13/2008
3/20/2008
3/27/2008
4/3/2008
1
2
3
4
5

Fig. 25B

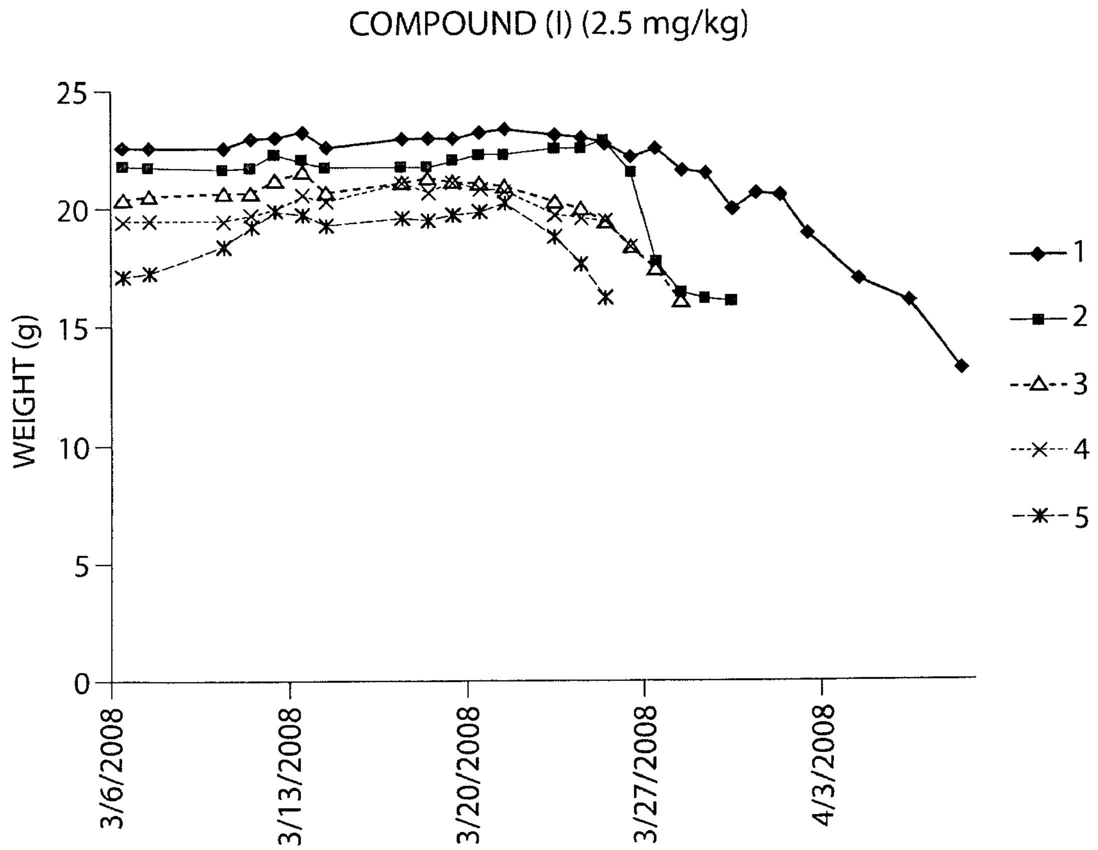
COMPOUND (I) (2.5 mg/kg)
WEIGHT (g)
25
20
15
10
5
0
3/6/2008
3/13/2008
3/20/2008
3/27/2008
4/3/2008
1
2
3
4
5

Fig. 25C

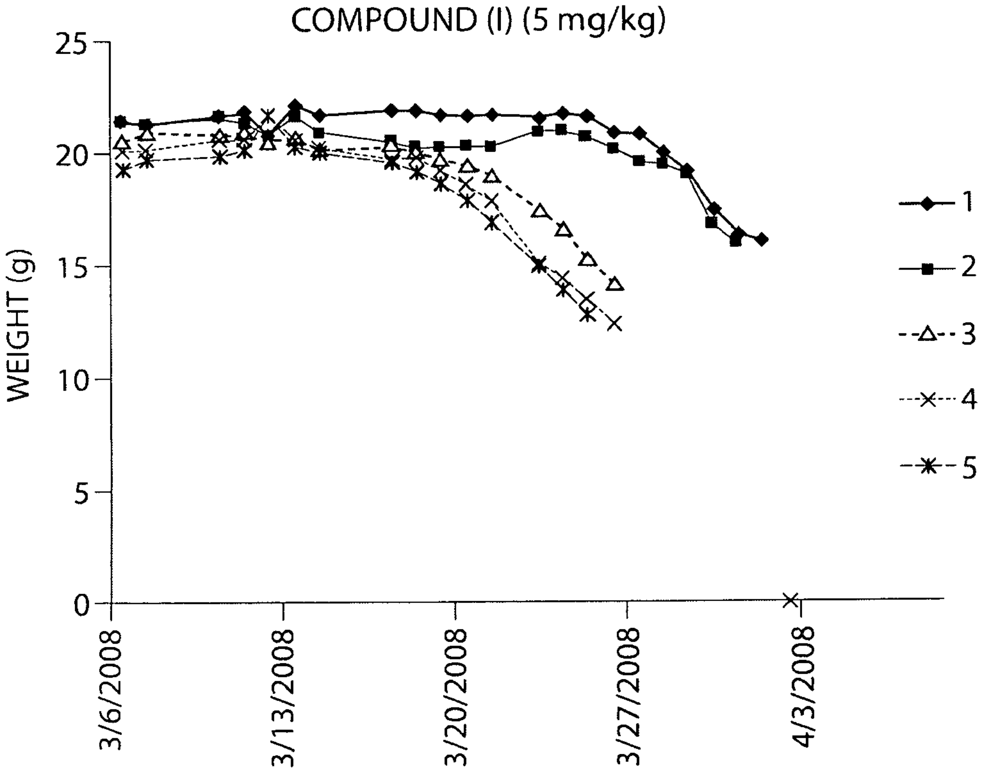
COMPOUND (I) (5 mg/kg)
WEIGHT (g)
25
20
15
10
5
0
3/6/2008
3/13/2008
3/20/2008
3/27/2008
4/3/2008
1
2
3
4
5

Fig. 25D

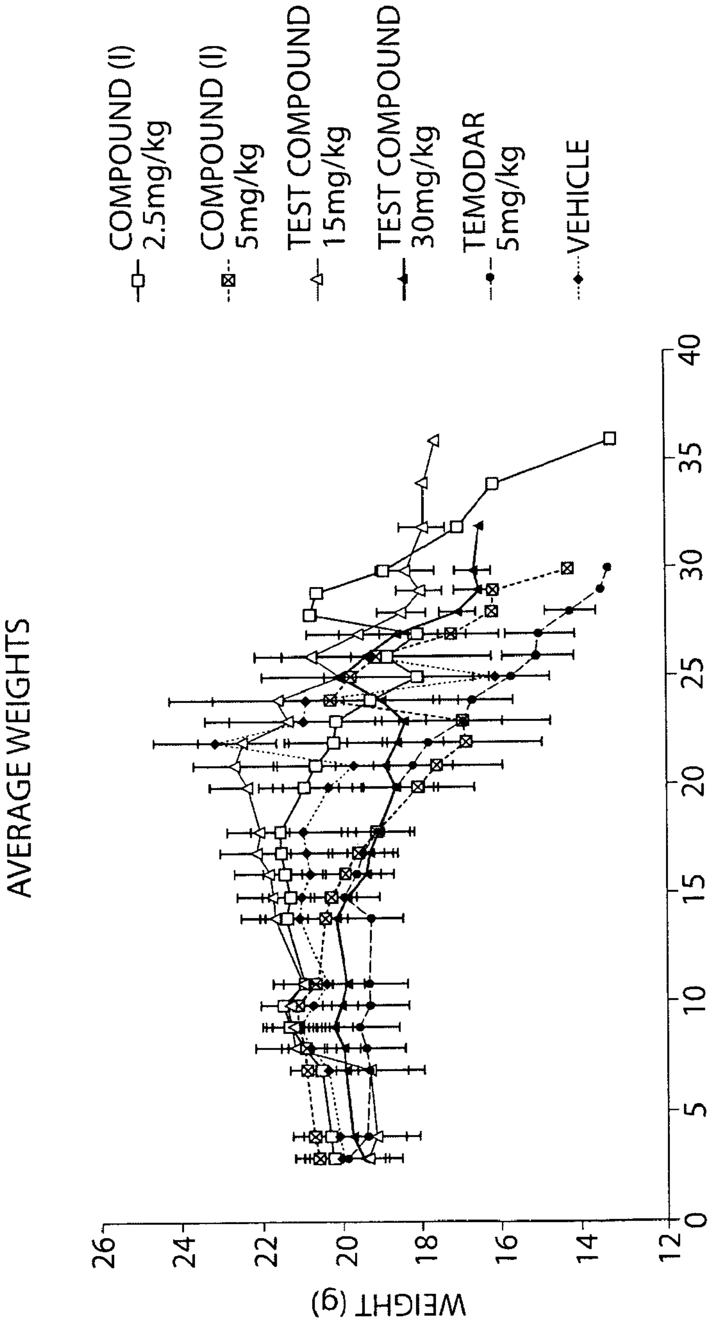
AVERAGE WEIGHTS
WEIGHT (g)
26
24
22
20
18
16
14
12
0
5
10
15
20
25
30
35
40
COMPOUND (I) 2.5mg/kg
COMPOUND (I) 5mg/kg
TEST COMPOUND 15mg/kg
TEST COMPOUND 30mg/kg
TEMODAR 5mg/kg
VEHICLE

Fig. 26

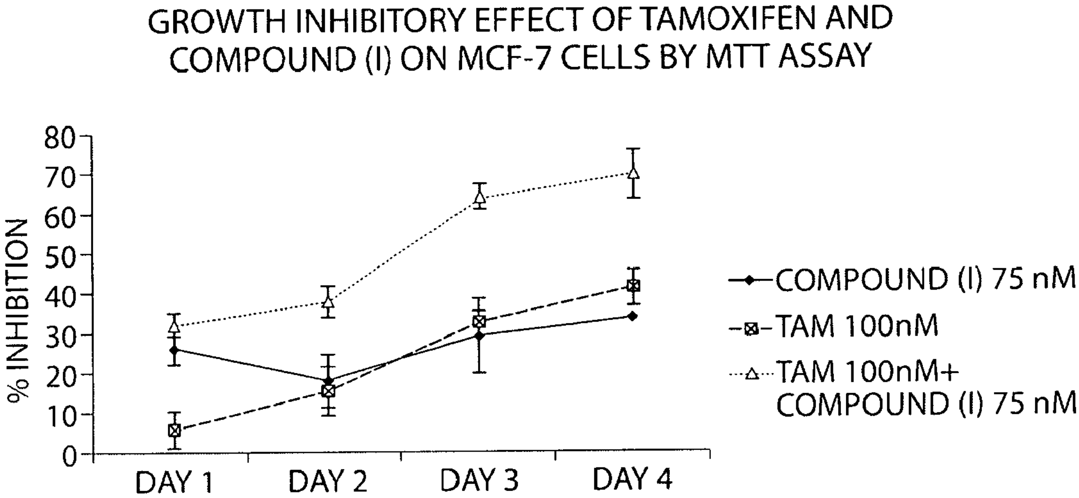
GROWTH INHIBITORY EFFECT OF TAMOXIFEN AND
COMPOUND (I) ON MCF-7 CELLS BY MTT ASSAY
% INHIBITION
80
70
60
50
40
30
20
10
0
DAY 1
DAY 2
DAY 3
DAY 4
COMPOUND (I) 75 nM
TAM 100nM
TAM 100nM+
COMPOUND (I) 75 nM

Fig. 27

Europäisches Patentamt
European Patent Office
Office européen des brevets

# EUROPEAN SEARCH REPORT

Application Number
EP 20 19 6756

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2007/015752 A1 (HANGAUER DAVID G JR [US]) 18 January 2007 (2007-01-18)<br>* the whole document *<br>* figures 13-15; example 6 *<br>----- | 1-12 | INV. A61K9/00 |
| A,D | US 2006/160800 A1 (HANGAUER DAVID G JR [US]) 20 July 2006 (2006-07-20)<br>* claims *<br>----- | 1-12 | |
| A | US 7 070 936 B1 (HANGAUER JR DAVID G [US] ET AL) 4 July 2006 (2006-07-04)<br>* figure 15a; example 5 *<br>----- | 1-12 | |

TECHNICAL FIELDS SEARCHED (IPC)

A61K
A61P
C07D

The present search report has been drawn up for all claims

1

| Place of search | Date of completion of the search | Examiner |
|---|---|---|
| Munich | 4 December 2020 | Giese, Hans-Hermann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................
& : member of the same patent family, corresponding document

EPO FORM 1503 03.82 (P04C01)

# ANNEX TO THE EUROPEAN SEARCH REPORT ON EUROPEAN PATENT APPLICATION NO. EP 20 19 6756

This annex lists the patent family members relating to the patent documents cited in the above-mentioned European search report.
The members are as contained in the European Patent Office EDP file on
The European Patent Office is in no way liable for these particulars which are merely given for the purpose of information.

04-12-2020

| Patent document cited in search report | Publication date | Patent family member(s) | Publication date |
|---|---|---|---|
| US 2007015752 A1 | 18-01-2007 | US 2007015752 A1 | 18-01-2007 |
| | | US 2011237586 A1 | 29-09-2011 |
| | | US 2012270874 A1 | 25-10-2012 |
| | | US 2014213587 A1 | 31-07-2014 |
| | | US 2017196874 A1 | 13-07-2017 |
| US 2006160800 A1 | 20-07-2006 | AT 544748 T | 15-02-2012 |
| | | AU 2005321966 A1 | 06-07-2006 |
| | | BR PI0519424 A2 | 20-01-2009 |
| | | CA 2594345 A1 | 06-07-2006 |
| | | CN 101184734 A | 21-05-2008 |
| | | CN 103274961 A | 04-09-2013 |
| | | DK 1836169 T3 | 26-03-2012 |
| | | EP 1836169 A2 | 26-09-2007 |
| | | ES 2382068 T3 | 05-06-2012 |
| | | HK 1108695 A1 | 16-05-2008 |
| | | HK 1188985 A1 | 23-05-2014 |
| | | JP 5124285 B2 | 23-01-2013 |
| | | JP 2008525530 A | 17-07-2008 |
| | | JP 2012082222 A | 26-04-2012 |
| | | KR 20070099622 A | 09-10-2007 |
| | | KR 20130079661 A | 10-07-2013 |
| | | PL 1836169 T3 | 31-07-2012 |
| | | US 2006160800 A1 | 20-07-2006 |
| | | US 2007197783 A1 | 23-08-2007 |
| | | US 2011065705 A1 | 17-03-2011 |
| | | US 2015182532 A1 | 02-07-2015 |
| | | WO 2006071960 A2 | 06-07-2006 |
| US 7070936 B1 | 04-07-2006 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 99994307
- US 7300931 B
- US 20070015752 A
- US 2008004847 W
- WO 2008002676 A
- US 6645972 B
- EP 337713 A
- US 20040214836 A
- US 20030130209 A
- US 20080221102 A
- US 2008006419 W
- US 5578322 A
- US 5607697 A
- US 4642903 A
- US 5631023 A
- US 4855326 A
- US 5380473 A
- US 5518730 A
- US 6471992 B
- US 5587172 A
- US 5616344 A
- US 6277406 B
- US 5622719 A
- US 20060160800 A1


### Non-patent literature cited in the description


- **HIDAKA ; KOBAYASHI.** *Annu. Rev. Pharmacol. Toxicol,* 1992, vol. 32, 377-397
- **DAVIES et al.** *Biochem. J.,* 2000, vol. 351, 95-105
- **PARANG ; SUN.** *Expert Opin. Ther. Patents,* 2005, vol. 15, 1183-1207
- **FRAME.** *Biochim. Biophys. Acta,* 2002, vol. 1602, 114-130
- **KAMI et al.** *Oncogene,* 1999, vol. 18 (33), 4654-4662
- **HISANO et al.** *Proc. Annu. Meet. Am. Assoc. Cancer Res.,* 1997, vol. 38, A1925
- **ELLIS et al.** *Journal of Biological Chemistry,* 1998, vol. 273 (2), 1052-1057
- **SCHWARTZBERG et al.** *Genes & Development,* 1997, vol. 11, 2835-2844
- **LEVITZKI.** *Current Opinion in Cell Biology,* 1996, vol. 8, 239-244
- **LEVITZKI.** *Anti-Cancer Drug Design,* 1996, vol. 11, 175-182
- **TREMBLAY et al.** *Int. J. Cancer,* 1996, vol. 68, 164-171
- **HOELZINGER et al.** *Neoplasia,* vol. 7 (1), 7-16
- **TWARD et al.** *PNAS,* vol. 104 (37), 14771-14776
- **SENGBUSCH et al.** *American Journal of Pathology,* vol. 166 (2), 585-595
- **L. GROVE et al.** *Cancer Res.,* 1995, vol. 55 (14), 3008-11
- **K. L. GROVE et al.** *Cancer Res.,* 1996, vol. 56 (18), 4187-4191
- **K. L. GROVE et al.** *Nucleosides Nucleotides,* 1997, vol. 16, 1229-33
- **S. A KADHIM et al.** *Can. Cancer Res.,* 1997, vol. 57 (21), 4803-10
- **GILES et al.** *J. Clin. Oncology,* 2001, vol. 19 (3
- **HU et al.** *Acta. Otolaryngol.,* 2000, vol. 120, 19-24
- **HIGHT et al.** *Hear. Res.,* 2003, vol. 179, 21-32
- **HU et al.** *Hear. Res.,* vol. 113, 198-206
- **KOPKE et al.** *Hear. Res.,* 2000, vol. 149, 138-146
- **HARRIS et al.** *Hear. Res.,* 2005, vol. 208, 14-25
- **GIANCOTTI ; RUOSLAHTI.** *Science,* 1999, vol. 285, 1028-1032
- **SORIANO et al.** *Cell,* 1991, vol. 64, 693-702
- **HONGMEI et al.** *J. Exp. Med.,* 2005, vol. 201, 1169-1177
- **DUONG et al.** *J. Clin. Invest.,* 1998, vol. 102, 881-892
- **DUONG et al.** *J. Bio. Chem.,* 2001, vol. 276, 7484-7492
- **HOME et al.** *Cell,* 1991, vol. 119, 1003-1013
- **MISSBACH et al.** *Bone,* 1999, vol. 24, 437-449
- **KLAMAN et al.** *Mol. Cell. Biol.,* 2000, vol. 20, 5479-5489
- **AHMAD et al.** *Metabolism,* 1997, vol. 46, 1140-1145
- **SALTIEL ; KAHN.** *Nature,* 2001, vol. 414, 799-806

- **AHMAD et al.** *J. Biol. Chem.,* 1997, vol. 270, 20503-20508
- **ELCHEBLY et al.** *Science,* 1999, vol. 283, 1544-1548
- **PATTI et al.** *J. Basic Clin. Physiol. Pharmacol.,* 1998, vol. 9, 89-109
- **KIDO et al.** *J. Clin. Endocrinol. Metab.,* 2001, vol. 86, 972-979
- **SALTIEL et al.** *Nature,* 2001, vol. 414, 799-806
- **QUON et al.** *Mol. Cell. Biol.,* 1995, vol. 15, 5403-5411
- **GOBERDHAN et al.** *Hum. Mol. Genet.,* 2003, vol. 12, R239-R248
- **LESLIE et al.** *J. Biochem.,* 2004, vol. 382, 1-11
- **TANG et al.** *J. Biol. Chem.,* 2005, vol. 280, 22523-22529
- **LAZAR ; SALTIEL.** *Nature Reviews,* 2006, vol. 5, 333-342
- **PESESSE et al.** *Biochem Biophys. Res. Commun.,* 1997, vol. 239, 697-700
- **BACKERS et al.** *Adv. Enzyme Regul.,* 2003, vol. 43, 15-28
- **CHI et al.** *J. Biol. Chem.,* 2004, vol. 279, 44987-44995
- **SLEEMAN et al.** *Nature Med.,* 2005, vol. 11, 199-205
- **ISHIHARA et al.** *Biochem. Biophys. Res. Commun.,* 1999, vol. 260, 265-272
- **KVANTA et al.** *Invest. Ophthal. Vis. Sci.,* 1996, vol. 37, 1929-1934
- **AIELLO et al.** *N. Engl. J. Med.,* 1994, vol. 331, 1480-1487
- **AIELLO et al.** *Proc. Natl. Acad. Sci.,* 1995, vol. 92, 10457-10461
- **KRZYSTOLIK et al.** *Arch. Ophthal.,* 2002, vol. 120, 338-346
- **QAUM et al.** *Invest. Ophthal. Vis. Sci.,* 2001, vol. 42, 2408-2413
- **PAUL et al.** *Nature Medicine,* 2001, vol. 7 (2), 222-227
- **PARANG ; SUN.** *Expert Opin. Ther. Patents,* 2005, vol. 15 (9), 1183-1206
- **YU et al.** *Proc. Natl. Acad. Sci.,* 1999, vol. 96, 7697-7704
- **GUO et al.** *J. Neuro.,* 2002, vol. 22, 6208-6217
- **KLEIN et al.** *EMBO J.,* 1999, vol. 18, 5019-5027
- **KLEIN et al.** *Mol. Cell. Biol.,* 1997, vol. 17, 6427-6436
- **ZAMOYSKA et al.** *Immunol. Rev.,* 2003, vol. 191, 107-118
- **LEVITZKI.** *Top. Curr. Chem.,* 2001, vol. 211, 1-15
- **LONGATI et al.** *Curr. Drug Targets,* 2001, vol. 2, 41-55
- **QIAN ; WEISS.** *Curr. Opin. Cell Biol.,* 1997, vol. 9, 205-211
- **PALACIOS ; WEISS.** *Oncogene,* 2004, vol. 23, 7990-8000
- **PARANG ; SUN.** *Expert Opin. The. Patents,* 2005, vol. 15, 1183-1207
- **KAMENS et al.** *Curr. Opin. Investig. Drugs,* 2001, vol. 2, 1213-1219
- **STRAUS ; WEISS.** *Cell,* 1992, vol. 70, 585-593
- **YAMASAKI et al.** *Mol. Cell. Biol.,* 1996, vol. 16, 7151-7160
- **HANKE ; POLLOK.** *Inflammation Res.,* 1995, vol. 44, 357-371
- Remington: the Science and Practice of Pharmacy. Mack Publishing Co, 1995
- **CHOU ; TALALAY.** *Adv. Enzyme Regul.,* 1984, vol. 22, 27-55
- Remington's Pharmaceutical Sciences. Mack Publishing Company, 1990
- **THERASSE, P.** New Guidelines to Evaluate the Response to Treatment in Solid Tumors. *J Nat Can Inst.,* 2000, vol. 92 (3), 205-216
- **PLÉ et al.** *J. Med. Chem,* 2004, vol. 47, 871-887
- **QUINN et al.** *Endocrinology,* 1998, vol. 139, 4424-4427
- **JIMI et al.** *J. Immunol.,* 1999, vol. 163, 434-442
- **MINET-RINGUET et al.** *Psychopharmacology,* 2006
- **NAKASHIMA et al.** *J. Biol. Chem.,* 2000, vol. 275, 12889-12895
- **PIERCE et al.** *Proc. Natl. Acad. Sci.,* 1995, vol. 92, 905-909
- **SMITH et al.** *Invest. Ophthal. Vis. Sci,* 1994, vol. 35, 101-111
- **SMITH et al.** *Invest. Ophthal. Vis. Sci.,* 1994, vol. 35, 101-111
- **ANDALUZ et al.** *Neurosurg. Clin. North Am.,* 2002, vol. 13, 385-393
- **ASHWAL, S. ; W. J. PEARCE.** *Curr. Opin. Pediatr.,* 2001, vol. 13, 506-516
- **DE LECINANA et al.** *Cerebrovasc. Dis.,* 2001, vol. 11 (1), 20-30
- **GINSBERG ; BUSTO.** *Stroke,* 1989, vol. 20, 1627-1642
- **LIN et al.** *J. Neurosci. Methods,* 2003, vol. 123, 89-97
- **MACRAE, I. M.** *Br. J. Clin. Pharmacol.,* 1992, vol. 34, 302-308
- **MCAULEY, M. A.** *Cerebrovasc. Brain Metab. Rev.,* 1995, vol. 7, 153-180
- **MEGYESI et al.** *Neurosurgery,* 2000, vol. 46, 448-460
- Stroke: animal models. Pergamon Press, 1983

- **TRAYSTMAN, R. J.** *ILAR J.,* 2003, vol. 44, 85-95
- **TRAYSTMAN.** *ILAR J.,* 2003, vol. 44 (2), 85-95
- **CARMICHAEL.** *NeuroRx®: The Journal of the American Society for Experimental NeuroTherapeutics,* 2005, vol. 2, 396-409
- **NARAYANASWAMY et al.** *JVIR,* 2000, vol. 11 (1), 5-17
- **BENNETT ; XIE.** *Pain,* 1988, vol. 33, 87-107
- **SELTZER et al.** *Pain,* 1990, vol. 43, 205-18
- **KIM ; CHUNG.** *Pain,* 1992, vol. 50, 355-63
- **MALMBERG ; BASBAUM.** *Pain,* 1998, vol. 76, 215-22
- **SUNG et al.** *Neurosci Lett.,* 1998, vol. 246, 117-9
- **LEE et al.** *Neuroreport,* 2000, vol. 11, 657-61
- **DECOSTERD ; WOOLF.** *Pain,* 2000, vol. 87, 149-58
- **VADAKKAN et al.** *J Pain,* 2005, vol. 6, 747-56
- **EATON.** *J. Rehabilitation Research and Development,* 2003, vol. 40, 41-54
- **GUHA et al.** *Lab Animal,* 2004, vol. 33 (7), 37-46
- **WALTER et al.** *Hepatology,* 1996, vol. 24 (1), 1-5
- **TENNANT ; GERIN.** *ILAR Journal,* 2001, vol. 42 (2), 89-102
- **GOLDBERG et al.** *J. Med. Chem.,* 2003, vol. 46, 1337-1349
- **JOHNSON et al.** *Clin Cancer Res 2005,* 01 October 2005, vol. 11 (19), 6924-6932
- **PUPUTTI et al.** *Mol Cancer Ther 2006,* December 2006, vol. 5 (12), 927-934
- **PALACIOS et al.** *Nature,* 1984, vol. 309, 126-131
- **PALACIOS et al.** *Cell,* 1985, vol. 41, 727-734
- **TREVINO et al.** *Am J Pathol.,* March 2006, vol. 168 (3), 962-72
- **PETTAWAY et al.** *Clin Cancer Res,* 1996, vol. 2, 1627-1636
- **KORBA et al.** *Antiviral Res.,* 1991, vol. 15, 217-228
- *Antiviral Res.,* 1992, vol. 19, 55-70
- **KORBA et al.** *Antiviral Res.,* 1992, vol. 19, 55-70
- **PIETSCHMANN, T. et al.** *J. Virol.,* vol. 76, 4008-4021